# EUROPEAN PATENT SPECIFICATION

(11) **EP 1 537 206 B1**
(45) Date of publication and mention of the grant of the patent: **06.05.2009**
(21) Application number: 03795150.6
(22) Date of filing: 12.09.2003
(51) Int. Cl.: C12N 7/04, C07K 14/18, C12Q 1/70, C12N 15/86

(54) **INFECTIOUS HEPACIVIRUS PSEUDO-PARTICLES CONTAINING FUNCTIONAL E1, E2 ENVELOPE PROTEINS**
INFEKTIÖSE HEPACIVIRUS-PSEUDOPARTIKEL MIT FUNKTIONELLEN E1-, E2-HÜLLPROTEINEN
PSEUDO-PARTICULES D'HEPACIVIRUS INFECTIEUSES RENFERMANT DES PROTEINES FONCTIONNELLES D'ENVELOPPE E1, E2

(30) Priority: 13.09.2002 EP 02292254; 03.03.2003 EP 03290505
(43) Date of publication of application: 08.06.2005
(73) Proprietor: INSTITUT NATIONAL DE LA SANTE ET DE LA RECHERCHE MEDICALE (INSERM), 75013 Paris (FR); ECOLE NORMALE SUPERIEURE DE LYON, 69364 Lyon Cedex 07 (FR)
(72) Inventor: BARTOSCH, Birke, F-38200 Chuzelles (FR); COSSET, François-Loic, F-69007 Lyon (FR)
(74) Representative: Colombet, Alain André
(86) International application number: PCT/IB2003/003882
(87) International publication number: WO 2004/024904

(56) References cited:
- WO-A-01/21807
- WO-A-02/070651
- WO-A-02/074941
- MATSUURA YOSHIHARU ET AL: "Characterization of pseudotype VSV possessing HCV envelope proteins" VIROLOGY, RAVEN PRESS, NEW YORK, NY, US, vol. 286, no. 2, 1 August 2001 (2001-08-01), pages 263-275, XP002206965 ISSN: 0042-6822
- COCQUEREL LAURENCE ET AL: "Topological changes in the transmembrane domains of hepatitis C virus envelope glycoproteins." THE EMBO JOURNAL. ENGLAND 17 JUN 2002, vol. 21, no. 12, 17 June 2002 (2002-06-17), pages 2893-2902, XP002271186 ISSN: 0261-4189
- BAUMERT THOMAS F ET AL: "Hepatitis C virus structural proteins assemble into viruslike particles in insect cells" JOURNAL OF VIROLOGY, THE AMERICAN SOCIETY FOR MICROBIOLOGY, US, vol. 72, no. 5, May 1998 (1998-05), pages 3827-3836, XP002167716 ISSN: 0022-538X
- BARTOSCH B ET AL: "Infectious hepatitis C virus pseudo-particles containing functional E1-E2 envelope protein complexes" JOURNAL OF EXPERIMENTAL MEDICINE, TOKYO, JP, vol. 197, no. 5, 3 March 2003 (2003-03-03), pages 633-642, XP002267934 ISSN: 0022-1007 cited in the application
- HSU MAYLA ET AL: "Hepatitis C virus glycoproteins mediate pH-dependent cell entry of pseudotyped retroviral particles." PROCEEDINGS OF THE NATIONAL ACADEMY OF SCIENCES OF THE UNITED STATES, vol. 100, no. 12, 10 June 2003 (2003-06-10), pages 7271-7276, XP002271187 June 10, 2003 ISSN: 0027-8424 (ISSN print) cited in the application
- BUONOCORE L ET AL.: "Characterization of Vesicular Stomatitis virus recombinants that express and incorporate high levels of Hepatitis C virus glycoproteins" J. VIROL., vol. 76, no. 14, July 2002 (2002-07), pages 6865-6872,
- DRUMMER H ET AL.: "Cell surface expression of functional hepatitis C virus E1 and E2 glycoproteins" FEBS LETTERS, vol. 546, 2003, pages 385-390,
- ROBERTS ML ET AL.: "Stable micro-dystrophin gene transfer using an integrating adeno-retroviral hybrid vector ameliorates the dystrophic pathology in mdx mouse muscle" HUMAN MOLECULAR GENETICS, vol. 11, no. 15, 2002, pages 1719-1730,

### SEQUENCE LISTING

<110> INSERM
<120> Infectious HCV pseudo-particles containing functionnal E1, E2 envelope proteins
<130> BET 03/P0879
<160> 16
<170> PatentIn version 3.2
<210> 1
   <211> 21
   <212> PRT
   <213> Hepatitis C virus : delta C
<400> 1
   Gly Cys Ser Phe Ser Ile Phe Leu Leu Ala Leu Leu Ser Cys Leu Thr
   1 5 10 15
   Val Pro Ala Ser Ala
   20
<210> 2
   <211> 20
   <212> DNA
   <213> Artificial
<220>
   <223> primer
<400> 2
   actggacgac gcaaagctgc
   20
<210> 3
   <211> 29
   <212> DNA
   <213> Artificial
<220>
   <223> primer
<400> 3
   cgcggatcct acgcgtcgac gccggcaaa
   29
<210> 4
   <211> 35
   <212> DNA
   <213> Artificial
<220>
   <223> primer
<400> 4
   tgcccgcttc agccgaaacc cacgtcaccg gggga
   35
<210> 5
   <211> 22
   <212> DNA
   <213> Artificial
<220>
   <223> primer
<400> 5
   gccagaagtc agatgctcaa gg
   22
<210> 6
   <211> 18
   <212> DNA
   <213> Artificial
<220>
   <223> primer
<400> 6
   tactctgagt ccaaaccg
   18
<210> 7
   <211> 35
   <212> DNA
   <213> Artificial
<220>
   <223> primer
<400> 7
   gtgacgtggg tttcggctga agcgggcaca gtcag
   35
<210> 8
   <211> 6274
   <212> DNA
   <213> Artificial
<220>
   <223> phCMV-deltaCE1-1a plasmid
<220>
   <221> promoter
   <222> (1)..(768)
   <223> human cytomegalovirus (hCMV) immediate-early promoter
<220>
   <221> misc_feature
   <222> (769)..(1429)
   <223> rabbit beta-globin intron II
<220>
   <221> misc_feature
   <222> (1425)..(1430)
   <223> BamHI 5'junction after ligation of deltaCE1 fragment in BamHI site of hCMV-G
<220>
   <221> misc_feature
   <222> (1430)..(2115)
   <223> IRES sequence of EMCV
<220>
   <221> CDS
   <222> (2116)..(2880)
<220>
   <221> misc_feature
   <222> (2125)..(2304)
   <223> deltaC part of the polyprotein
<220>
   <221> misc_feature
   <222> (2305)..(2880)
   <223> Mature E1 protein
<220>
   <221> misc_feature
   <222> (2883)..(2888)
   <223> BamHI 3' junction after ligation of deltaCE1 fragment in BamHI site of hCMV-G
<400> 8
   gcggccgctc tagagagctt ggcccattgc atacgttgta tccatatcat aatatgtaca      60
   tttatattgg ctcatgtcca acattaccgc catgttgaca ttgattattg actagttatt     120
   aatagtaatc aattacgggg tcattagttc atagcccata tatggagttc cgcgttacat     180
   aacttacggt aaatggcccg cctggctgac cgcccaacga cccccgccca ttgacgtcaa     240
   taatgacgta tgttcccata gtaacgccaa tagggacttt ccattgacgt caatgggtgg     300
   agtatttacg gtaaactgcc cacttggcag tacatcaagt gtatcatatg ccaagtacgc     360
   cccctattga cgtcaatgac ggtaaatggc ccgcctggca ttatgcccag tacatgacct     420
   tatgggactt tcctacttgg cagtacatct acgtattagt catcgctatt accatggtga     480
   tgcggttttg gcagtacatc aatgggcgtg gatagcggtt tgactcacgg ggatttccaa     540
   gtctccaccc cattgacgtc aatgggagtt tgttttggca ccaaaatcaa cgggactttc     600
   caaaatgtcg taacaactcc gccccattga cgcaaatggg cggtaggcgt gtacggtggg     660
   aggtctatat aagcagagct cgtttagtga accgtcagat cgcctggaga cgccatccac     720
   gctgttttga cctccataga agacaccggg accgatccag cctccggtcg accgatcctg     780
   agaacttcag ggtgagtttg gggacccttg attgttcttt ctttttcgct attgtaaaat    840
   tcatgttata tggagggggc aaagttttca gggtgttgtt tagaatggga agatgtccct    900
   tgtatcacca tggaccctca tgataatttt gtttctttca ctttctactc tgttgacaac    960
   cattgtctcc tcttattttc ttttcatttt ctgtaacttt ttcgttaaac tttagcttgc   1020
   atttgtaacg aatttttaaa ttcacttttg tttatttgtc agattgtaag tactttctct   1080
   aatcactttt ttttcaaggc aatcagggta tattatattg tacttcagca cagttttaga   1140
   gaacaattgt tataattaaa tgataaggta gaatatttct gcatataaat tctggctggc   1200
   gtggaaatat tcttattggt agaaacaact acaccctggt catcatcctg cctttctctt   1260
   tatggttaca atgatataca ctgtttgaga tgaggataaa atactctgag tccaaaccgg   1320
   gcccctctgc taaccatgtt catgccttct tctctttcct acagctcctg ggcaacgtgc   1380
   tggttgttgt gctgtctcat cattttggca aagaattcct cgacggatcc gatgaaggac   1440
   agttctttcc agacattgtt gaattgatct cgatcccgcg aaattaatac gactcactat   1500
   agggagacca caacggtttc cctctagcgg gatcaattcc gcccctctcc ctcccccccc   1560
   cctaacgtta ctggccgaag ccgcttggaa taaggccggt gtgcgtttgt ctatatgtta   1620
   ttttccacca tattgccgtc ttttggcaat gtgagggccc ggaaacctgg ccctgtcttc   1680
   ttgacgagca ttcctagggg tctttcccct ctcgccaaag gaatgcaagg tctgttgaat   1740
   gtcgtgaagg aagcagttcc tctggaagct tcttgaagac aaacaacgtc tgtagcgacc   1800
   ctttgcaggc agcggaaccc cccacctggc gacaggtgcc tctgcggcca aaagccacgt   1860
   gtataagata cacctgcaaa ggcggcacaa ccccagtgcc acgttgtgag ttggatagtt   1920
   gtggaaagag tcaaatggct ctcctcaagc gtattcaaca aggggctgaa ggatgcccag   1980
   aaggtacccc attgtatggg atctgatctg gggcctcggt gcacatgctt tacatgtgtt   2040
   tagtcgaggt taaaaaacgt ctaggccccc cgaaccacgg ggacgtggtt ttcctttgaa   2100
   aaacacgata atacc atg aat tcc gac ctc atg ggg tac ata ccg ctc gtc    2151
                    Met Asn Ser Asp Leu Met Gly Tyr Ile Pro Leu Val
                    1               5                   10
   ggc gcc cct ctt gga ggc gct gcc agg gcc ctg gcg cat ggc gtc cgg     2199
   Gly Ala Pro Leu Gly Gly Ala Ala Arg Ala Leu Ala His Gly Val Arg
       15                  20                  25
   gtt ctg gaa gac ggc gtg aac tat gca aca ggg aac ctt cct ggt tgc     2247
   Val Leu Glu Asp Gly Val Asn Tyr Ala Thr Gly Asn Leu Pro Gly Cys
       30                  35                  40
   ttt ttc tct atc ttc ctt ctg gcc ctg ctc tct tgc ctg act gtg ccc     2295
   Phe Phe Ser Ile Phe Leu Leu Ala Leu Leu Ser Cys Leu Thr Val Pro
   45                  50                  55                  60
   gct tca gcc tac caa gtg cgc aat tcc tcg ggg ctt tac cat gtc acc     2343
   Ala Ser Ala Tyr Gln Val Arg Asn Ser Ser Gly Leu Tyr His Val Thr
                   65                  70                  75
   aat gat tgc cct aat tcg agt att gtg tac gag gcg gcc gat gcc atc     2391
   Asn Asp Cys Pro Asn Ser Ser Ile Val Tyr Glu Ala Ala Asp Ala Ile
               80                  85                  90
   ctg cac act ccg ggg tgt gtc cct tgc gtt cgc gag ggt aac gcc tcg     2439
   Leu His Thr Pro Gly Cys Val Pro Cys Val Arg Glu Gly Asn Ala Ser
           95                  100                 105
   agg tgt tgg gtg gcg gtg acc ccc acg gtg gcc acc agg gac ggc aaa     2487
   Arg Cys Trp Val Ala Val Thr Pro Thr Val Ala Thr Arg Asp Gly Lys
       110                 115                 120
   ctc ccc aca acg cag ctt cga cgt cat atc gat ctg ctt gtc ggg agc     2535
   Leu Pro Thr Thr Gln Leu Arg Arg His Ile Asp Leu Leu Val Gly Ser
   125                 130                 135                 140
   gcc acc ctc tgc tca gcc ctc tac gtg ggg gac ctg tgc ggg tct gtt     2583
   Ala Thr Leu Cys Ser Ala Leu Tyr Val Gly Asp Leu Cys Gly Ser Val
                   145                 150                 155
   ttt ctt gtt ggt caa ctg ttt acc ttc tct ccc agg cgc cac tgg acg     2631
   Phe Leu Val Gly Gln Leu Phe Thr Phe Ser Pro Arg Arg His Trp Thr
               160                 165                 170
   acg caa agc tgc aat tgt tct atc tat ccc ggc cat ata acg ggt cat     2679
   Thr Gln Ser Cys Asn Cys Ser Ile Tyr Pro Gly His Ile Thr Gly His
           175                 180                 185
   cgc atg gca tgg gat atg atg atg aac tgg tcc cct acg gca gcg ttg     2727
   Arg Met Ala Trp Asp Met Met Met Asn Trp Ser Pro Thr Ala Ala Leu
       190                 195                 200
   gtg gta gct cag ctg ctc cgg atc cca caa gcc atc atg gac atg atc     2775
   Val Val Ala Gln Leu Leu Arg Ile Pro Gln Ala Ile Met Asp Met Ile
   205                 210                 215                 220
   gct ggt gct cac tgg gga gtc ctg gcg ggc ata gcg tat ttc tcc atg     2823
   Ala Gly Ala His Trp Gly Val Leu Ala Gly Ile Ala Tyr Phe Ser Met
                   225                 230                 235
   gtg ggg aac tgg gcg aag gtc ctg gta gtg ctg ctg tta ttt gcc ggc     2871
   Val Gly Asn Trp Ala Lys Val Leu Val Val Leu Leu Leu Phe Ala Gly
               240                 245                 250
   gtc gac gcg taggatccgt cgaggaattc actcctcagg tgcaggctgc             2920
   Val Asp Ala
           255
   ctatcagaag gtggtggctg gtgtggccaa tgccctggct cacaaatacc actgagatct   2980
   ttttccctct gccaaaaatt atggggacat catgaagccc cttgagcatc tgacttctgg   3040
   ctaataaagg aaatttattt tcattgcaat agtgtgttgg aattttttgt gtctctcact   3100
   cggaaggaca tatgggaggg caaatcattt aaaacatcag aatgagtatt tggtttagag   3160
   tttggcaaca tatgcccata tgctggctgc catgaacaaa ggttggctat aaagaggtca   3220
   tcagtatatg aaacagcccc ctgctgtcca ttccttattc catagaaaag ccttgacttg 3280
   aggttagatt ttttttatat tttgttttgt gttatttttt tctttaacat ccctaaaatt 3340
   ttccttacat gttttactag ccagattttt cctcctctcc tgactactcc cagtcatagc 3400
   tgtccctctt ctcttatgga gatccctcga cggatcggcc gcaattcgta atcatgtcat 3460
   agctgtttcc tgtgtgaaat tgttatccgc tcacaattcc acacaacata cgagccggaa 3520
   gcataaagtg taaagcctgg ggtgcctaat gagtgagcta actcacatta attgcgttgc 3580
   gctcactgcc cgctttccag tcgggaaacc tgtcgtgcca gctgcattaa tgaatcggcc 3640
   aacgcgcggg gagaggcggt ttgcgtattg ggcgctcttc cgcttcctcg ctcactgact 3700
   cgctgcgctc ggtcgttcgg ctgcggcgag cggtatcagc tcactcaaag gcggtaatac 3760
   ggttatccac agaatcaggg gataacgcag gaaagaacat gtgagcaaaa ggccagcaaa 3820
   aggccaggaa ccgtaaaaag gccgcgttgc tggcgttttt ccataggctc cgcccccctg 3880
   acgagcatca caaaaatcga cgctcaagtc agaggtggcg aaacccgaca ggactataaa 3940
   gataccaggc gtttccccct ggaagctccc tcgtgcgctc tcctgttccg accctgccgc 4000
   ttaccggata cctgtccgcc tttctccctt cgggaagcgt ggcgctttct catagctcac 4060
   gctgtaggta tctcagttcg gtgtaggtcg ttcgctccaa gctgggctgt gtgcacgaac 4120
   cccccgttca gcccgaccgc tgcgccttat ccggtaacta tcgtcttgag tccaacccgg 4180
   taagacacga cttatcgcca ctggcagcag ccactggtaa caggattagc agagcgaggt 4240
   atgtaggcgg tgctacagag ttcttgaagt ggtggcctaa ctacggctac actagaagaa 4300
   cagtatttgg tatctgcgct ctgctgaagc cagttacctt cggaaaaaga gttggtagct 4360
   cttgatccgg caaacaaacc accgctggta gcggtggttt ttttgtttgc aagcagcaga 4420
   ttacgcgcag aaaaaaagga tctcaagaag atcctttgat cttttctacg gggtctgacg 4480
   ctcagtggaa cgaaaactca cgttaaggga ttttggtcat gagattatca aaaaggatct 4540
   tcacctagat ccttttaaat taaaaatgaa gttttaaatc aatctaaagt atatatgagt 4600
   aaacttggtc tgacagttac caatgcttaa tcagtgaggc acctatctca gcgatctgtc 4660
   tatttcgttc atccatagtt gcctgactcc ccgtcgtgta gataactacg atacgggagg 4720
   gcttaccatc tggccccagt gctgcaatga taccgcgaga cccacgctca ccggctccag 4780
   atttatcagc aataaaccag ccagccggaa gggccgagcg cagaagtggt cctgcaactt 4840
   tatccgcctc catccagtct attaattgtt gccgggaagc tagagtaagt agttcgccag 4900
   ttaatagttt gcgcaacgtt gttgccattg ctacaggcat cgtggtgtca cgctcgtcgt 4960
   ttggtatggc ttcattcagc tccggttccc aacgatcaag gcgagttaca tgatccccca 5020
   tgttgtgcaa aaaagcgggt tagctccttc ggtcctccga tcgttgtcag aagtaagttg 5080
   gccgcagtgt tatcactcat ggttatggca gcactgcata attctcttac tgtcatgcca 5140
   tccgtaagat gcttttctgt gactggtgag tactcaacca agtcattctg agaatagtgt 5200
   atgcggcgac cgagttgctc ttgcccggcg tcaatacggg ataataccgc gccacatagc 5260
   agaactttaa aagtgctcat cattggaaaa cgttcttcgg ggcgaaaact ctcaaggatc 5320
   ttaccgctgt tgagatccag ttcgatgtaa cccactcgtg cacccaactg atcttcagca 5380
   tcttttactt tcaccagcgt ttctgggtga gcaaaaacag gaaggcaaaa tgccgcaaaa 5440
   aagggaataa gggcgacacg gaaatgttga atactcatac tcttcctttt tcaatattat 5500
   tgaagcattt atcagggtta ttgtctcatg agcggataca tatttgaatg tatttagaaa 5560
   aataaacaaa taggggttcc gcgcacattt ccccgaaaag tgccacctaa attgtaagcg 5620
   ttaatatttt gttaaaattc gcgttaaatt tttgttaaat cagctcattt tttaaccaat 5680
   aggccgaaat cggcaaaatc ccttataaat caaaagaata gaccgagata gggttgagtg 5740
   ttgttccagt ttggaacaag agtccactat taaagaacgt ggactccaac gtcaaagggc 5800
   gaaaaaccgt ctatcagggc gatggcccac tacgtgaacc atcaccctaa tcaagttttt 5860
   tggggtcgag gtgccgtaaa gcactaaatc ggaaccctaa agggagcccc cgatttagag 5920
   cttgacgggg aaagccggcg aacgtggcga gaaaggaagg gaagaaagcg aaaggagcgg 5980
   gcgctagggc gctggcaagt gtagcggtca cgctgcgcgt aaccaccaca cccgccgcgc 6040
   ttaatgcgcc gctacagggc gcgtcccatt cgccattcag gctgcgcaac tgttgggaag 6100
   ggcgatcggt gcgggcctct tcgctattac gccagctggc gaaaggggga tgtgctgcaa 6160
   ggcgattaag ttgggtaacg ccagggtttt cccagtcacg acgttgtaaa acgacggcca 6220
   gtgagcgcgc gtaatacgac tcactatagg gcgaattgga gctccaccgc ggtg 6274
<210> 9
   <211> 255
   <212> PRT
   <213> Artificial
<220>
   <223> phCMV-deltaCE1-1a plasmid
<400> 9
   Met Asn Ser Asp Leu Met Gly Tyr Ile Pro Leu Val Gly Ala Pro Leu
   1               5                   10                  15
   Gly Gly Ala Ala Arg Ala Leu Ala His Gly Val Arg Val Leu Glu Asp
               20                  25                  30
   Gly Val Asn Tyr Ala Thr Gly Asn Leu Pro Gly Cys Phe Phe Ser Ile
   35 40 45
   Phe Leu Leu Ala Leu Leu Ser Cys Leu Thr Val Pro Ala Ser Ala Tyr
   50 55 60
   Gln Val Arg Asn Ser Ser Gly Leu Tyr His Val Thr Asn Asp Cys Pro
   65 70 75 80
   Asn Ser Ser Ile Val Tyr Glu Ala Ala Asp Ala Ile Leu His Thr Pro
   85 90 95
   Gly Cys Val Pro Cys Val Arg Glu Gly Asn Ala Ser Arg Cys Trp Val
   100 105 110
   Ala Val Thr Pro Thr Val Ala Thr Arg Asp Gly Lys Leu Pro Thr Thr
   115 120 125
   Gln Leu Arg Arg His Ile Asp Leu Leu Val Gly Ser Ala Thr Leu Cys
   130 135 140
   Ser Ala Leu Tyr Val Gly Asp Leu Cys Gly Ser Val Phe Leu Val Gly
   145 150 155 160
   Gln Leu Phe Thr Phe Ser Pro Arg Arg His Trp Thr Thr Gln Ser Cys
   165 170 175
   Asn Cys Ser Ile Tyr Pro Gly His Ile Thr Gly His Arg Met Ala Trp
   180 185 190
   Asp Met Met Met Asn Trp Ser Pro Thr Ala Ala Leu Val Val Ala Gln
   195 200 205
   Leu Leu Arg Ile Pro Gln Ala Ile Met Asp Met Ile Ala Gly Ala His
   210 215 220
   Trp Gly Val Leu Ala Gly Ile Ala Tyr Phe Ser Met Val Gly Asn Trp
   225 230 235 240
   Ala Lys Val Leu Val Val Leu Leu Leu Phe Ala Gly Val Asp Ala
   245 250 255
<210> 10
   <211> 7754
   <212> DNA
   <213> Artificial
<220>
   <223> phCMV-deltaCE1E2-1a plasmid
<220>
   <221> promoter
   <222> (1)..(768)
   <223> human cytomegalovirus (hCMV) immediate-early promoter
<220>
   <221> misc_feature
   <222> (769)..(1429)
   <223> rabbit beta-globin intron II
<220>
   <221> misc feature
   <222> (1425)..(1433)
   <223> BamHI/ClaI junction after ligation of pTM-E1E2-745bit (ClaI-blunted/StuI) in BamHI-blunted of hCMV-G
<220>
   <221> misc feature
   <222> (1430)..(2115)
   <223> IRES sequence of EMCV
<220>
   <221> CDS
   <222> (2116)..(3966)
<220>
   <221> misc_feature
   <222> (2125)..(2304)
   <223> deltaC part of the polyprotein
<220>
   <221> misc_feature
   <222> (2305)..(2880)
   <223> Mature E1 protein
<220>
   <221> misc_feature
   <222> (2881)..(3966)
   <223> Mature E2 protein
<220>
   <221> misc_feature
   <222> (3979)..(4354)
   <223> Sinbis Virus junk sequence
<220>
   <221> misc_feature
   <222> (4361)..(4368)
   <223> StuI/BamHI junction after ligation of pTM-E1E2-745bit (ClaI-blunted/StuI) in BamHI-blunted of hCMV-G
<400> 10
   gcggccgctc tagagagctt ggcccattgc atacgttgta tccatatcat aatatgtaca 60
   tttatattgg ctcatgtcca acattaccgc catgttgaca ttgattattg actagttatt 120
   aatagtaatc aattacgggg tcattagttc atagcccata tatggagttc cgcgttacat 180
   aacttacggt aaatggcccg cctggctgac cgcccaacga cccccgccca ttgacgtcaa 240
   taatgacgta tgttcccata gtaacgccaa tagggacttt ccattgacgt caatgggtgg 300
   agtatttacg gtaaactgcc cacttggcag tacatcaagt gtatcatatg ccaagtacgc 360
   cccctattga cgtcaatgac ggtaaatggc ccgcctggca ttatgcccag tacatgacct 420
   tatgggactt tcctacttgg cagtacatct acgtattagt catcgctatt accatggtga 480
   tgcggttttg gcagtacatc aatgggcgtg gatagcggtt tgactcacgg ggatttccaa 540
   gtctccaccc cattgacgtc aatgggagtt tgttttggca ccaaaatcaa cgggactttc 600
   caaaatgtcg taacaactcc gccccattga cgcaaatggg cggtaggcgt gtacggtggg 660
   aggtctatat aagcagagct cgtttagtga accgtcagat cgcctggaga cgccatccac 720
   gctgttttga cctccataga agacaccggg accgatccag cctccggtcg accgatcctg 780
   agaacttcag ggtgagtttg gggacccttg attgttcttt ctttttcgct attgtaaaat 840
   tcatgttata tggagggggc aaagttttca gggtgttgtt tagaatggga agatgtccct 900
   tgtatcacca tggaccctca tgataatttt gtttctttca ctttctactc tgttgacaac 960
   cattgtctcc tcttattttc ttttcatttt ctgtaacttt ttcgttaaac tttagcttgc 1020
   atttgtaacg aatttttaaa ttcacttttg tttatttgtc agattgtaag tactttctct 1080
   aatcactttt ttttcaaggc aatcagggta tattatattg tacttcagca cagttttaga 1140
   gaacaattgt tataattaaa tgataaggta gaatatttct gcatataaat tctggctggc 1200
   gtggaaatat tcttattggt agaaacaact acaccctggt catcatcctg cctttctctt 1260
   tatggttaca atgatataca ctgtttgaga tgaggataaa atactctgag tccaaaccgg 1320
   gcccctctgc taaccatgtt catgccttct tctctttcct acagctcctg ggcaacgtgc 1380
   tggttgttgt gctgtctcat cattttggca aagaattcct cgacggatcc gatgaaggac 1440
   agttctttcc agacattgtt gaattgatct cgatcccgcg aaattaatac gactcactat 1500
   agggagacca caacggtttc cctctagcgg gatcaattcc gcccctctcc ctcccccccc 1560
   cctaacgtta ctggccgaag ccgcttggaa taaggccggt gtgcgtttgt ctatatgtta 1620
   ttttccacca tattgccgtc ttttggcaat gtgagggccc ggaaacctgg ccctgtcttc 1680
   ttgacgagca ttcctagggg tctttcccct ctcgccaaag gaatgcaagg tctgttgaat 1740
   gtcgtgaagg aagcagttcc tctggaagct tcttgaagac aaacaacgtc tgtagcgacc 1800
   ctttgcaggc agcggaaccc cccacctggc gacaggtgcc tctgcggcca aaagccacgt 1860
   gtataagata cacctgcaaa ggcggcacaa ccccagtgcc acgttgtgag ttggatagtt 1920
   gtggaaagag tcaaatggct ctcctcaagc gtattcaaca aggggctgaa ggatgcccag 1980
   aaggtacccc attgtatggg atctgatctg gggcctcggt gcacatgctt tacatgtgtt 2040
   tagtcgaggt taaaaaacgt ctaggccccc cgaaccacgg ggacgtggtt ttcctttgaa   2100
   aaacacgata atacc atg aat tcc gac ctc atg ggg tac ata ccg ctc gtc    2151
                    Met Asn Ser Asp Leu Met Gly Tyr Ile Pro Leu Val
                    1               5                   10
   ggc gcc cct ctt gga ggc gct gcc agg gcc ctg gcg cat ggc gtc cgg     2199
   Gly Ala Pro Leu Gly Gly Ala Ala Arg Ala Leu Ala His Gly Val Arg
           15                  20                  25
   gtt ctg gaa gac ggc gtg aac tat gca aca ggg aac ctt cct ggt tgc     2247
   Val Leu Glu Asp Gly Val Asn Tyr Ala Thr Gly Asn Leu Pro Gly Cys
       30                  35                  40
   ttt ttc tct atc ttc ctt ctg gcc ctg ctc tct tgc ctg act gtg ccc     2295
   Phe Phe Ser Ile Phe Leu Leu Ala Leu Leu Ser Cys Leu Thr Val Pro
   45                  50                  55                  60
   gct tca gcc tac caa gtg cgc aat tcc tcg ggg ctt tac cat gtc acc     2343
   Ala Ser Ala Tyr Gln Val Arg Asn Ser Ser Gly Leu Tyr His Val Thr
                   65                  70                  75
   aat gat tgc cct aat tcg agt att gtg tac gag gcg gcc gat gcc atc
   2391
   Asn Asp Cys Pro Asn Ser Ser Ile Val Tyr Glu Ala Ala Asp Ala Ile
               80                  85                  90
   ctg cac act ccg ggg tgt gtc cct tgc gtt cgc gag ggt aac gcc tcg
   2439
   Leu His Thr Pro Gly Cys Val Pro Cys Val Arg Glu Gly Asn Ala Ser
           95                  100                 105
   agg tgt tgg gtg gcg gtg acc ccc acg gtg gcc acc agg gac ggc aaa
   2487
   Arg Cys Trp Val Ala Val Thr Pro Thr Val Ala Thr Arg Asp Gly Lys
       110                 115                 120
   ctc ccc aca acg cag ctt cga cgt cat atc gat ctg ctt gtc ggg agc
   2535
   Leu Pro Thr Thr Gln Leu Arg Arg His Ile Asp Leu Leu Val Gly Ser
   125                 130                 135                 140
   gcc acc ctc tgc tca gcc ctc tac gtg ggg gac ctg tgc ggg tct gtt
   2583
   Ala Thr Leu Cys Ser Ala Leu Tyr Val Gly Asp Leu Cys Gly Ser Val
                   145                 150                 155
   ttt ctt gtt ggt caa ctg ttt acc ttc tct ccc agg cgc cac tgg acg
   2631
   Phe Leu Val Gly Gln Leu Phe Thr Phe Ser Pro Arg Arg His Trp Thr
               160                 165                 170
   acg caa agc tgc aat tgt tct atc tat ccc ggc cat ata acg ggt cat
   2679
   Thr Gln Ser Cys Asn Cys Ser Ile Tyr Pro Gly His Ile Thr Gly His
           175                 180                 185
   cgc atg gca tgg gat atg atg atg aac tgg tcc cct acg gca gcg ttg
   2727
   Arg Met Ala Trp Asp Met Met Met Asn Trp Ser Pro Thr Ala Ala Leu
       190                 195                 200
   gtg gta gct cag ctg ctc cgg atc cca caa gcc atc atg gac atg atc
   2775
   Val Val Ala Gln Leu Leu Arg Ile Pro Gln Ala Ile Met Asp Met Ile
   205 210 215 220
   gct ggt gct cac tgg gga gtc ctg gcg ggc ata gcg tat ttc tcc atg
   2823
   Ala Gly Ala His Trp Gly Val Leu Ala Gly Ile Ala Tyr Phe Ser Met
   225 230 235
   gtg ggg aac tgg gcg aag gtc ctg gta gtg ctg ctg tta ttt gcc ggc
   2871
   Val Gly Asn Trp Ala Lys Val Leu Val Val Leu Leu Leu Phe Ala Gly
   240 245 250
   gtc gac gcg gaa acc cac gtc acc ggg gga agt gcc ggc cac acc acg
   2919
   Val Asp Ala Glu Thr His Val Thr Gly Gly Ser Ala Gly His Thr Thr
   255 260 265
   gct ggg ctt gtt ggt ctc ctt aca cca ggc gcc aag cag aac atc caa
   2967
   Ala Gly Leu Val Gly Leu Leu Thr Pro Gly Ala Lys Gln Asn Ile Gln
   270 275 280
   ctg atc aac acc aac ggc agt tgg cac atc aat agc acg gcc ttg aac
   3015
   Leu Ile Asn Thr Asn Gly Ser Trp His Ile Asn Ser Thr Ala Leu Asn
   285 290 295 300
   tgc aac gat agc ctt acc acc ggc tgg tta gca ggg ctc ttc tat cgc
   3063
   Cys Asn Asp Ser Leu Thr Thr Gly Trp Leu Ala Gly Leu Phe Tyr Arg
   305 310 315
   cac aaa ttc aac tct tca ggc tgt cct gag agg ttg gcc agc tgc cga
   3111
   His Lys Phe Asn Ser Ser Gly Cys Pro Glu Arg Leu Ala Ser Cys Arg
   320 325 330
   cgc ctt acc gat ttt gcc cag ggc tgg ggt ccc atc agt tat gcc aac
   3159
   Arg Leu Thr Asp Phe Ala Gln Gly Trp Gly Pro Ile Ser Tyr Ala Asn
   335 340 345
   gga agc ggc ctt gac gaa cgc ccc tac tgt tgg cac tac cct cca aga
   3207
   Gly Ser Gly Leu Asp Glu Arg Pro Tyr Cys Trp His Tyr Pro Pro Arg
   350 355 360
   cct tgt ggc att gtg ccc gca aag agc gtg tgt ggc ccg gta tat tgc
   3255
   Pro Cys Gly Ile Val Pro Ala Lys Ser Val Cys Gly Pro Val Tyr Cys
   365 370 375 380
   ttc act ccc agc ccc gtg gtg gtg gga acg acc gac agg tcg ggc gcg
   3303
   Phe Thr Pro Ser Pro Val Val Val Gly Thr Thr Asp Arg Ser Gly Ala
   385 390 395
   cct acc tac agc tgg ggt gca aat gat acg gat gtc ttc gtc ctt aac
   3351
   Pro Thr Tyr Ser Trp Gly Ala Asn Asp Thr Asp Val Phe Val Leu Asn
   400 405 410
   aac acc agg cca ccg ctg ggc aat tgg ttc ggt tgt acc tgg atg aac
   3399
   Asn Thr Arg Pro Pro Leu Gly Asn Trp Phe Gly Cys Thr Trp Met Asn
   415 420 425
   tca act gga ttc acc aaa gtg tgc gga gcg ccc cct tgt gtc atc gga
   3447
   Ser Thr Gly Phe Thr Lys Val Cys Gly Ala Pro Pro Cys Val Ile Gly
   430 435 440
   ggg gtg ggc aac aac acc ttg ctc tgc ccc act gat tgc ttc cgc aaa
   3495
   Gly Val Gly Asn Asn Thr Leu Leu Cys Pro Thr Asp Cys Phe Arg Lys
   445 450 455 460
   cat ccg gaa gcc aca tac tct cgg tgc ggc tcc ggt ccc tgg att aca
   3543
   His Pro Glu Ala Thr Tyr Ser Arg Cys Gly Ser Gly Pro Trp Ile Thr
   465 470 475
   ccc agg tgc atg gtc gac tac ccg tat agg ctt tgg cac tat cct tgt
   3591
   Pro Arg Cys Met Val Asp Tyr Pro Tyr Arg Leu Trp His Tyr Pro Cys
   480 485 490
   act atc aat tac acc ata ttc aaa gtc agg atg tac gtg gga ggg gtc
   3639
   Thr Ile Asn Tyr Thr Ile Phe Lys Val Arg Met Tyr Val Gly Gly Val
   495 500 505
   gag cac agg ctg gaa gcg gcc tgc aac tgg acg cgg ggc gaa cgc tgt
   3687
   Glu His Arg Leu Glu Ala Ala Cys Asn Trp Thr Arg Gly Glu Arg Cys
   510 515 520
   gat ctg gaa gac agg gac agg tcc gag ctc agc cca ttg ctg ctg tcc
   3735
   Asp Leu Glu Asp Arg Asp Arg Ser Glu Leu Ser Pro Leu Leu Leu Ser
   525 530 535 540
   acc aca cag tgg cag gtc ctt ccg tgt tct ttc acg acc ctg cca gcc
   3783
   Thr Thr Gln Trp Gln Val Leu Pro Cys Ser Phe Thr Thr Leu Pro Ala
   545 550 555
   ttg tcc acc ggc ctc atc cac ctc cac cag aac att gtg gac gtg cag
   3831
   Leu Ser Thr Gly Leu Ile His Leu His Gln Asn Ile Val Asp Val Gln
   560 565 570
   tac ttg tac ggg gtg ggg tca agc atc gcg tcc tgg gcc att aag tgg
   3879
   Tyr Leu Tyr Gly Val Gly Ser Ser Ile Ala Ser Trp Ala Ile Lys Trp
   575 580 585
   gag tac gtc gtt ctc ctg ttc ctt ctg ctt gca gac gcg cgc gtc tgc 3927
   Glu Tyr Val Val Leu Leu Phe Leu Leu Leu Ala Asp Ala Arg Val Cys
   590 595 600
   tcc tgc ttg tgg atg atg tta ctc ata tcc caa gcg gag taagctcctt 3976
   Ser Cys Leu Trp Met Met Leu Leu Ile Ser Gln Ala Glu
   605 610 615
   gggcccaatg atccgaccag caaaactcga tgtacttccg aggaactgat gtgcataatg 4036
   catcaggctg gtacattaga tccccgctta ccgcgggcaa tatagcaaca ctaaaaactc 4096
   gatgtacttc cgaggaagcg cagtgcataa tgctgcgcag tgttgccaca taaccactat 4156
   attaaccatt tatctagcgg acgccaaaaa ctcaatgtat ttctgaggaa gcgtggtgca 4216
   taatgccacg cagcgtctgc ataactttta ttatttcttt tattaatcaa caaaattttg 4276
   tttttaacat ttcaaaaaaa aaaaaaaaaa aaaaaaaaaa aaaaaaaaaa gggaattcct 4336
   cgattaatta agcggccgct cgagagggat ccgtcgagga attcactcct caggtgcagg 4396
   ctgcctatca gaaggtggtg gctggtgtgg ccaatgccct ggctcacaaa taccactgag 4456
   atctttttcc ctctgccaaa aattatgggg acatcatgaa gccccttgag catctgactt 4516
   ctggctaata aaggaaattt attttcattg caatagtgtg ttggaatttt ttgtgtctct 4576
   cactcggaag gacatatggg agggcaaatc atttaaaaca tcagaatgag tatttggttt 4636
   agagtttggc aacatatgcc catatgctgg ctgccatgaa caaaggttgg ctataaagag 4696
   gtcatcagta tatgaaacag ccccctgctg tccattcctt attccataga aaagccttga 4756
   cttgaggtta gatttttttt atattttgtt ttgtgttatt tttttcttta acatccctaa 4816
   aattttcctt acatgtttta ctagccagat ttttcctcct ctcctgacta ctcccagtca 4876
   tagctgtccc tcttctctta tggagatccc tcgacggatc ggccgcaatt cgtaatcatg 4936
   tcatagctgt ttcctgtgtg aaattgttat ccgctcacaa ttccacacaa catacgagcc 4996
   ggaagcataa agtgtaaagc ctggggtgcc taatgagtga gctaactcac attaattgcg 5056
   ttgcgctcac tgcccgcttt ccagtcggga aacctgtcgt gccagctgca ttaatgaatc 5116
   ggccaacgcg cggggagagg cggtttgcgt attgggcgct cttccgcttc ctcgctcact 5176
   gactcgctgc gctcggtcgt tcggctgcgg cgagcggtat cagctcactc aaaggcggta 5236
   atacggttat ccacagaatc aggggataac gcaggaaaga acatgtgagc aaaaggccag 5296
   caaaaggcca ggaaccgtaa aaaggccgcg ttgctggcgt ttttccatag gctccgcccc 5356
   cctgacgagc atcacaaaaa tcgacgctca agtcagaggt ggcgaaaccc gacaggacta 5416
   taaagatacc aggcgtttcc ccctggaagc tccctcgtgc gctctcctgt tccgaccctg 5476
   ccgcttaccg gatacctgtc cgcctttctc ccttcgggaa gcgtggcgct ttctcatagc 5536
   tcacgctgta ggtatctcag ttcggtgtag gtcgttcgct ccaagctggg ctgtgtgcac 5596
   gaaccccccg ttcagcccga ccgctgcgcc ttatccggta actatcgtct tgagtccaac 5656
   ccggtaagac acgacttatc gccactggca gcagccactg gtaacaggat tagcagagcg 5716
   aggtatgtag gcggtgctac agagttcttg aagtggtggc ctaactacgg ctacactaga 5776
   agaacagtat ttggtatctg cgctctgctg aagccagtta ccttcggaaa aagagttggt 5836
   agctcttgat ccggcaaaca aaccaccgct ggtagcggtg gtttttttgt ttgcaagcag 5896
   cagattacgc gcagaaaaaa aggatctcaa gaagatcctt tgatcttttc tacggggtct 5956
   gacgctcagt ggaacgaaaa ctcacgttaa gggattttgg tcatgagatt atcaaaaagg 6016
   atcttcacct agatcctttt aaattaaaaa tgaagtttta aatcaatcta aagtatatat 6076
   gagtaaactt ggtctgacag ttaccaatgc ttaatcagtg aggcacctat ctcagcgatc 6136
   tgtctatttc gttcatccat agttgcctga ctccccgtcg tgtagataac tacgatacgg 6196
   gagggcttac catctggccc cagtgctgca atgataccgc gagacccacg ctcaccggct 6256
   ccagatttat cagcaataaa ccagccagcc ggaagggccg agcgcagaag tggtcctgca 6316
   actttatccg cctccatcca gtctattaat tgttgccggg aagctagagt aagtagttcg 6376
   ccagttaata gtttgcgcaa cgttgttgcc attgctacag gcatcgtggt gtcacgctcg 6436
   tcgtttggta tggcttcatt cagctccggt tcccaacgat caaggcgagt tacatgatcc 6496
   cccatgttgt gcaaaaaagc gggttagctc cttcggtcct ccgatcgttg tcagaagtaa 6556
   gttggccgca gtgttatcac tcatggttat ggcagcactg cataattctc ttactgtcat 6616
   gccatccgta agatgctttt ctgtgactgg tgagtactca accaagtcat tctgagaata 6676
   gtgtatgcgg cgaccgagtt gctcttgccc ggcgtcaata cgggataata ccgcgccaca 6736
   tagcagaact ttaaaagtgc tcatcattgg aaaacgttct tcggggcgaa aactctcaag 6796
   gatcttaccg ctgttgagat ccagttcgat gtaacccact cgtgcaccca actgatcttc 6856
   agcatctttt actttcacca gcgtttctgg gtgagcaaaa acaggaaggc aaaatgccgc 6916
   aaaaaaggga ataagggcga cacggaaatg ttgaatactc atactcttcc tttttcaata 6976
   ttattgaagc atttatcagg gttattgtct catgagcgga tacatatttg aatgtattta 7036
   gaaaaataaa caaatagggg ttccgcgcac atttccccga aaagtgccac ctaaattgta 7096
   agcgttaata ttttgttaaa attcgcgtta aatttttgtt aaatcagctc attttttaac 7156
   caataggccg aaatcggcaa aatcccttat aaatcaaaag aatagaccga gatagggttg 7216
   agtgttgttc cagtttggaa caagagtcca ctattaaaga acgtggactc caacgtcaaa 7276
   gggcgaaaaa ccgtctatca gggcgatggc ccactacgtg aaccatcacc ctaatcaagt 7336
   tttttggggt cgaggtgccg taaagcacta aatcggaacc ctaaagggag cccccgattt 7396
   agagcttgac ggggaaagcc ggcgaacgtg gcgagaaagg aagggaagaa agcgaaagga 7456
   gcgggcgcta gggcgctggc aagtgtagcg gtcacgctgc gcgtaaccac cacacccgcc 7516
   gcgcttaatg cgccgctaca gggcgcgtcc cattcgccat tcaggctgcg caactgttgg 7576
   gaagggcgat cggtgcgggc ctcttcgcta ttacgccagc tggcgaaagg gggatgtgct 7636
   gcaaggcgat taagttgggt aacgccaggg ttttcccagt cacgacgttg taaaacgacg 7696
   gccagtgagc gcgcgtaata cgactcacta tagggcgaat tggagctcca ccgcggtg 7754
<210> 11
   <211> 617
   <212> PRT
   <213> Artificial
<220>
   <223> phCMV-deltaCE1E2-1a plasmid
<400> 11
   Met Asn Ser Asp Leu Met Gly Tyr Ile Pro Leu Val Gly Ala Pro Leu
   1               5                   10                  15
   Gly Gly Ala Ala Arg Ala Leu Ala His Gly Val Arg Val Leu Glu Asp
               20                  25                  30
   Gly Val Asn Tyr Ala Thr Gly Asn Leu Pro Gly Cys Phe Phe Ser Ile
           35                  40                  45
   Phe Leu Leu Ala Leu Leu Ser Cys Leu Thr Val Pro Ala Ser Ala Tyr
       50                  55                  60
   Gln Val Arg Asn Ser Ser Gly Leu Tyr His Val Thr Asn Asp Cys Pro
   65                  70                  75                  80
   Asn Ser Ser Ile Val Tyr Glu Ala Ala Asp Ala Ile Leu His Thr Pro
                   85                  90                  95
   Gly Cys Val Pro Cys Val Arg Glu Gly Asn Ala Ser Arg Cys Trp Val
               100                 105                 110
   Ala Val Thr Pro Thr Val Ala Thr Arg Asp Gly Lys Leu Pro Thr Thr
           115                 120                 125
   Gln Leu Arg Arg His Ile Asp Leu Leu Val Gly Ser Ala Thr Leu Cys
       130                 135                 140
   Ser Ala Leu Tyr Val Gly Asp Leu Cys Gly Ser Val Phe Leu Val Gly
   145 150 155 160
   Gln Leu Phe Thr Phe Ser Pro Arg Arg His Trp Thr Thr Gln Ser Cys
   165 170 175
   Asn Cys Ser Ile Tyr Pro Gly His Ile Thr Gly His Arg Met Ala Trp
   180 185 190
   Asp Met Met Met Asn Trp Ser Pro Thr Ala Ala Leu Val Val Ala Gln
   195 200 205
   Leu Leu Arg Ile Pro Gln Ala Ile Met Asp Met Ile Ala Gly Ala His
   210 215 220
   Trp Gly Val Leu Ala Gly Ile Ala Tyr Phe Ser Met Val Gly Asn Trp
   225 230 235 240
   Ala Lys Val Leu Val Val Leu Leu Leu Phe Ala Gly Val Asp Ala Glu
   245 250 255
   Thr His Val Thr Gly Gly Ser Ala Gly His Thr Thr Ala Gly Leu Val
   260 265 270
   Gly Leu Leu Thr Pro Gly Ala Lys Gln Asn Ile Gln Leu Ile Asn Thr
   275 280 285
   Asn Gly Ser Trp His Ile Asn Ser Thr Ala Leu Asn Cys Asn Asp Ser
   290 295 300
   Leu Thr Thr Gly Trp Leu Ala Gly Leu Phe Tyr Arg His Lys Phe Asn
   305 310 315 320
   Ser Ser Gly Cys Pro Glu Arg Leu Ala Ser Cys Arg Arg Leu Thr Asp
   325 330 335
   Phe Ala Gln Gly Trp Gly Pro Ile Ser Tyr Ala Asn Gly Ser Gly Leu
   340 345 350
   Asp Glu Arg Pro Tyr Cys Trp His Tyr Pro Pro Arg Pro Cys Gly Ile
   355 360 365
   Val Pro Ala Lys Ser Val Cys Gly Pro Val Tyr Cys Phe Thr Pro Ser
   370 375 380
   Pro Val Val Val Gly Thr Thr Asp Arg Ser Gly Ala Pro Thr Tyr Ser
   385                 390                 395                 400
   Trp Gly Ala Asn Asp Thr Asp Val Phe Val Leu Asn Asn Thr Arg Pro
                   405                 410                 415
   Pro Leu Gly Asn Trp Phe Gly Cys Thr Trp Met Asn Ser Thr Gly Phe
               420                 425                 430
   Thr Lys Val Cys Gly Ala Pro Pro Cys Val Ile Gly Gly Val Gly Asn
           435                 440                 445
   Asn Thr Leu Leu Cys Pro Thr Asp Cys Phe Arg Lys His Pro Glu Ala
       450                 455                 460
   Thr Tyr Ser Arg Cys Gly Ser Gly Pro Trp Ile Thr Pro Arg Cys Met
   465                 470                 475                 480
   Val Asp Tyr Pro Tyr Arg Leu Trp His Tyr Pro Cys Thr Ile Asn Tyr
                   485                 490                 495
   Thr Ile Phe Lys Val Arg Met Tyr Val Gly Gly Val Glu His Arg Leu
               500                 505                 510
   Glu Ala Ala Cys Asn Trp Thr Arg Gly Glu Arg Cys Asp Leu Glu Asp
           515                 520                 525
   Arg Asp Arg Ser Glu Leu Ser Pro Leu Leu Leu Ser Thr Thr Gln Trp
       530                 535                 540
   Gln Val Leu Pro Cys Ser Phe Thr Thr Leu Pro Ala Leu Ser Thr Gly
   545                 550                 555                 560
   Leu Ile His Leu His Gln Asn Ile Val Asp Val Gln Tyr Leu Tyr Gly
                   565                 570                 575
   Val Gly Ser Ser Ile Ala Ser Trp Ala Ile Lys Trp Glu Tyr Val Val
               580                 585                 590
   Leu Leu Phe Leu Leu Leu Ala Asp Ala Arg Val Cys Ser Cys Leu Trp
           595                 600                 605
   Met Met Leu Leu Ile Ser Gln Ala Glu
       610                 615
<210> 12
   <211> 7178
   <212> DNA
   <213> Artificial
<220>
   <223> phCKV-deltaCE2-1a plasmid
<220>
   <221> promoter
   <222> (1)..(768)
   <223> human cytomegalovirus (hCMV) immediate-early promoter
<220>
   <221> misc_feature
   <222> (769)..(1429)
   <223> rabbit beta-globin intron II
<220>
   <221> misc_feature
   <222> (1425)..(1430)
   <223> BamHI 5'junction after ligation of deltaCE2 fragment in BamHI site of hCMV-G
<220>
   <221> misc_feature
   <222> (1430)..(2115)
   <223> IRES sequence of EMCV
<220>
   <221> CDS
   <222> (2116)..(3390)
<220>
   <221> misc_feature
   <222> (2125)..(2304)
   <223> deltaC part of the polyprotein
<220>
   <221> misc_feature
   <222> (2305)..(3390)
   <223> Mature E2 protein
<220>
   <221> misc_feature
   <222> (3403)..(3778)
   <223> Sinbis Virus junk sequence
<220>
   <221> misc_feature
   <222> (3787)..(3792)
   <223> BamHI 3' junction after ligation of deltaCE1 fragment in BamHI site of hCMV-G
<400> 12
   gcggccgctc tagagagctt ggcccattgc atacgttgta tccatatcat aatatgtaca      60
   tttatattgg ctcatgtcca acattaccgc catgttgaca ttgattattg actagttatt     120
   aatagtaatc aattacgggg tcattagttc atagcccata tatggagttc cgcgttacat     180
   aacttacggt aaatggcccg cctggctgac cgcccaacga cccccgccca ttgacgtcaa     240
   taatgacgta tgttcccata gtaacgccaa tagggacttt ccattgacgt caatgggtgg 300
   agtatttacg gtaaactgcc cacttggcag tacatcaagt gtatcatatg ccaagtacgc 360
   cccctattga cgtcaatgac ggtaaatggc ccgcctggca ttatgcccag tacatgacct 420
   tatgggactt tcctacttgg cagtacatct acgtattagt catcgctatt accatggtga 480
   tgcggttttg gcagtacatc aatgggcgtg gatagcggtt tgactcacgg ggatttccaa 540
   gtctccaccc cattgacgtc aatgggagtt tgttttggca ccaaaatcaa cgggactttc 600
   caaaatgtcg taacaactcc gccccattga cgcaaatggg cggtaggcgt gtacggtggg 660
   aggtctatat aagcagagct cgtttagtga accgtcagat cgcctggaga cgccatccac 720
   gctgttttga cctccataga agacaccggg accgatccag cctccggtcg accgatcctg 780
   agaacttcag ggtgagtttg gggacccttg attgttcttt ctttttcgct attgtaaaat 840
   tcatgttata tggagggggc aaagttttca gggtgttgtt tagaatggga agatgtccct 900
   tgtatcacca tggaccctca tgataatttt gtttctttca ctttctactc tgttgacaac 960
   cattgtctcc tcttattttc ttttcatttt ctgtaacttt ttcgttaaac tttagcttgc 1020
   atttgtaacg aatttttaaa ttcacttttg tttatttgtc agattgtaag tactttctct 1080
   aatcactttt ttttcaaggc aatcagggta tattatattg tacttcagca cagttttaga 1140
   gaacaattgt tataattaaa tgataaggta gaatatttct gcatataaat tctggctggc 1200
   gtggaaatat tcttattggt agaaacaact acaccctggt catcatcctg cctttctctt 1260
   tatggttaca atgatataca ctgtttgaga tgaggataaa atactctgag tccaaaccgg 1320
   gcccctctgc taaccatgtt catgccttct tctctttcct acagctcctg ggcaacgtgc 1380
   tggttgttgt gctgtctcat cattttggca aagaattcct cgacggatcc gatgaaggac 1440
   agttctttcc agacattgtt gaattgatct cgatcccgcg aaattaatac gactcactat 1500
   agggagacca caacggtttc cctctagcgg gatcaattcc gcccctctcc ctcccccccc 1560
   cctaacgtta ctggccgaag ccgcttggaa taaggccggt gtgcgtttgt ctatatgtta 1620
   ttttccacca tattgccgtc ttttggcaat gtgagggccc ggaaacctgg ccctgtcttc 1680
   ttgacgagca ttcctagggg tctttcccct ctcgccaaag gaatgcaagg tctgttgaat 1740
   gtcgtgaagg aagcagttcc tctggaagct tcttgaagac aaacaacgtc tgtagcgacc 1800
   ctttgcaggc agcggaaccc cccacctggc gacaggtgcc tctgcggcca aaagccacgt 1860
   gtataagata cacctgcaaa ggcggcacaa ccccagtgcc acgttgtgag ttggatagtt 1920
   gtggaaagag tcaaatggct ctcctcaagc gtattcaaca aggggctgaa ggatgcccag 1980
   aaggtacccc attgtatggg atctgatctg gggcctcggt gcacatgctt tacatgtgtt 2040
   tagtcgaggt taaaaaacgt ctaggccccc cgaaccacgg ggacgtggtt ttcctttgaa   2100
   aaacacgata atacc atg aat tcc gac ctc atg ggg tac ata ccg ctc gtc    2151
                    Met Asn Ser Asp Leu Met Gly Tyr Ile Pro Leu Val
                    1               5                   10
   ggc gcc cct ctt gga ggc gct gcc agg gcc ctg gcg cat ggc gtc cgg     2199
   Gly Ala Pro Leu Gly Gly Ala Ala Arg Ala Leu Ala His Gly Val Arg
           15                  20                  25
   gtt ctg gaa gac ggc gtg aac tat gca aca ggg aac ctt cct ggt tgc     2247
   Val Leu Glu Asp Gly Val Asn Tyr Ala Thr Gly Asn Leu Pro Gly Cys
       30                  35                  40
   ttt ttc tct atc ttc ctt ctg gcc ctg ctc tct tgc ctg act gtg ccc     2295
   Phe Phe Ser Ile Phe Leu Leu Ala Leu Leu Ser Cys Leu Thr Val Pro
   45                  50                  55                  60
   gct tca gcc gaa acc cac gtc acc ggg gga agt gcc ggc cac acc acg     2343
   Ala Ser Ala Glu Thr His Val Thr Gly Gly Ser Ala Gly His Thr Thr
                   65                  70                  75
   gct ggg ctt gtt ggt ctc ctt aca cca ggc gcc aag cag aac atc caa     2391
   Ala Gly Leu Val Gly Leu Leu Thr Pro Gly Ala Lys Gln Asn Ile Gln
               80                  85                  90
   ctg atc aac acc aac ggc agt tgg cac atc aat agc acg gcc ttg aac     2439
   Leu Ile Asn Thr Asn Gly Ser Trp His Ile Asn Ser Thr Ala Leu Asn
           95                  100                 105
   tgc aac gat agc ctt acc acc ggc tgg tta gca ggg ctc ttc tat cgc     2487
   Cys Asn Asp Ser Leu Thr Thr Gly Trp Leu Ala Gly Leu Phe Tyr Arg
       110                 115                 120
   cac aaa ttc aac tct tca ggc tgt cct gag agg ttg gcc agc tgc cga     2535
   His Lys Phe Asn Ser Ser Gly Cys Pro Glu Arg Leu Ala Ser Cys Arg
   125                 130                 135                 140
   cgc ctt acc gat ttt gcc cag ggc tgg ggt ccc atc agt tat gcc aac     2583
   Arg Leu Thr Asp Phe Ala Gln Gly Trp Gly Pro Ile Ser Tyr Ala Asn
                   145                 150                 155
   gga agc ggc ctt gac gaa cgc ccc tac tgt tgg cac tac cct cca aga     2631
   Gly Ser Gly Leu Asp Glu Arg Pro Tyr Cys Trp His Tyr Pro Pro Arg
               160                 165                 170
   cct tgt ggc att gtg ccc gca aag agc gtg tgt ggc ccg gta tat tgc     2679
   Pro Cys Gly Ile Val Pro Ala Lys Ser Val Cys Gly Pro Val Tyr Cys
           175                 180                 185
   ttc act ccc agc ccc gtg gtg gtg gga acg acc gac agg tcg ggc gcg     2727
   Phe Thr Pro Ser Pro Val Val Val Gly Thr Thr Asp Arg Ser Gly Ala
       190                 195                 200
   cct acc tac agc tgg ggt gca aat gat acg gat gtc ttc gtc ctt aac     2775
   Pro Thr Tyr Ser Trp Gly Ala Asn Asp Thr Asp Val Phe Val Leu Asn
   205                 210                 215                 220
   aac acc agg cca ccg ctg ggc aat tgg ttc ggt tgt acc tgg atg aac     2823
   Asn Thr Arg Pro Pro Leu Gly Asn Trp Phe Gly Cys Thr Trp Met Asn
                   225                 230                 235
   tca act gga ttc acc aaa gtg tgc gga gcg ccc cct tgt gtc atc gga        2871
   Ser Thr Gly Phe Thr Lys Val Cys Gly Ala Pro Pro Cys Val Ile Gly
               240                 245                 250
   ggg gtg ggc aac aac acc ttg ctc tgc ccc act gat tgc ttc cgc aaa        2919
   Gly Val Gly Asn Asn Thr Leu Leu Cys Pro Thr Asp Cys Phe Arg Lys
           255                 260                 265
   cat ccg gaa gcc aca tac tct cgg tgc ggc tcc ggt ccc tgg att aca        2967
   His Pro Glu Ala Thr Tyr Ser Arg Cys Gly Ser Gly Pro Trp Ile Thr
       270                 275                 280
   ccc agg tgc atg gtc gac tac ccg tat agg ctt tgg cac tat cct tgt        3015
   Pro Arg Cys Met Val Asp Tyr Pro Tyr Arg Leu Trp His Tyr Pro Cys
   285                 290                 295                 300
   act atc aat tac acc ata ttc aaa gtc agg atg tac gtg gga ggg gtc        3063
   Thr Ile Asn Tyr Thr Ile Phe Lys Val Arg Met Tyr Val Gly Gly Val
                   305                 310                 315
   gag cac agg ctg gaa gcg gcc tgc aac tgg acg cgg ggc gaa cgc tgt        3111
   Glu His Arg Leu Glu Ala Ala Cys Asn Trp Thr Arg Gly Glu Arg Cys
               320                 325                 330
   gat ctg gaa gac agg gac agg tcc gag ctc agc cca ttg ctg ctg tcc        3159
   Asp Leu Glu Asp Arg Asp Arg Ser Glu Leu Ser Pro Leu Leu Leu Ser
           335                 340                 345
   acc aca cag tgg cag gtc ctt ccg tgt tct ttc acg acc ctg cca gcc        3207
   Thr Thr Gln Trp Gln Val Leu Pro Cys Ser Phe Thr Thr Leu Pro Ala
       350                 355                 360
   ttg tcc acc ggc ctc atc cac ctc cac cag aac att gtg gac gtg cag        3255
   Leu Ser Thr Gly Leu Ile His Leu His Gln Asn Ile Val Asp Val Gln
   365                 370                 375                 380
   tac ttg tac ggg gtg ggg tca agc atc gcg tcc tgg gcc att aag tgg        3303
   Tyr Leu Tyr Gly Val Gly Ser Ser Ile Ala Ser Trp Ala Ile Lys Trp
                   385                 390                 395
   gag tac gtc gtt ctc ctg ttc ctt ctg ctt gca gac gcg cgc gtc tgc        3351
   Glu Tyr Val Val Leu Leu Phe Leu Leu Leu Ala Asp Ala Arg Val Cys
               400                 405                 410
   tcc tgc ttg tgg atg atg tta ctc ata tcc caa gcg gag taagctcctt         3400
   Ser Cys Leu Trp Met Met Leu Leu Ile Ser Gln Ala Glu
           415                 420                 425
   gggcccaatg atccgaccag caaaactcga tgtacttccg aggaactgat gtgcataatg     3460
   catcaggctg gtacattaga tccccgctta ccgcgggcaa tatagcaaca ctaaaaactc     3520
   gatgtacttc cgaggaagcg cagtgcataa tgctgcgcag tgttgccaca taaccactat     3580
   attaaccatt tatctagcgg acgccaaaaa ctcaatgtat ttctgaggaa gcgtggtgca     3640
   taatgccacg cagcgtctgc ataactttta ttatttcttt tattaatcaa caaaattttg     3700
   tttttaacat ttcaaaaaaa aaaaaaaaaa aaaaaaaaaa aaaaaaaaaa gggaattcct     3760
   cgattaatta agcggccgct cgagagggat ccgtcgagga attcactcct caggtgcagg 3820
   ctgcctatca gaaggtggtg gctggtgtgg ccaatgccct ggctcacaaa taccactgag 3880
   atctttttcc ctctgccaaa aattatgggg acatcatgaa gccccttgag catctgactt 3940
   ctggctaata aaggaaattt attttcattg caatagtgtg ttggaatttt ttgtgtctct 4000
   cactcggaag gacatatggg agggcaaatc atttaaaaca tcagaatgag tatttggttt 4060
   agagtttggc aacatatgcc catatgctgg ctgccatgaa caaaggttgg ctataaagag 4120
   gtcatcagta tatgaaacag ccccctgctg tccattcctt attccataga aaagccttga 4180
   cttgaggtta gatttttttt atattttgtt ttgtgttatt tttttcttta acatccctaa 4240
   aattttcctt acatgtttta ctagccagat ttttcctcct ctcctgacta ctcccagtca 4300
   tagctgtccc tcttctctta tggagatccc tcgacggatc ggccgcaatt cgtaatcatg 4360
   tcatagctgt ttcctgtgtg aaattgttat ccgctcacaa ttccacacaa catacgagcc 4420
   ggaagcataa agtgtaaagc ctggggtgcc taatgagtga gctaactcac attaattgcg 4480
   ttgcgctcac tgcccgcttt ccagtcggga aacctgtcgt gccagctgca ttaatgaatc 4540
   ggccaacgcg cggggagagg cggtttgcgt attgggcgct cttccgcttc ctcgctcact 4600
   gactcgctgc gctcggtcgt tcggctgcgg cgagcggtat cagctcactc aaaggcggta 4660
   atacggttat ccacagaatc aggggataac gcaggaaaga acatgtgagc aaaaggccag 4720
   caaaaggcca ggaaccgtaa aaaggccgcg ttgctggcgt ttttccatag gctccgcccc 4780
   cctgacgagc atcacaaaaa tcgacgctca agtcagaggt ggcgaaaccc gacaggacta 4840
   taaagatacc aggcgtttcc ccctggaagc tccctcgtgc gctctcctgt tccgaccctg 4900
   ccgcttaccg gatacctgtc cgcctttctc ccttcgggaa gcgtggcgct ttctcatagc 4960
   tcacgctgta ggtatctcag ttcggtgtag gtcgttcgct ccaagctggg ctgtgtgcac 5020
   gaaccccccg ttcagcccga ccgctgcgcc ttatccggta actatcgtct tgagtccaac 5080
   ccggtaagac acgacttatc gccactggca gcagccactg gtaacaggat tagcagagcg 5140
   aggtatgtag gcggtgctac agagttcttg aagtggtggc ctaactacgg ctacactaga 5200
   agaacagtat ttggtatctg cgctctgctg aagccagtta ccttcggaaa aagagttggt 5260
   agctcttgat ccggcaaaca aaccaccgct ggtagcggtg gtttttttgt ttgcaagcag 5320
   cagattacgc gcagaaaaaa aggatctcaa gaagatcctt tgatcttttc tacggggtct 5380
   gacgctcagt ggaacgaaaa ctcacgttaa gggattttgg tcatgagatt atcaaaaagg 5440
   atcttcacct agatcctttt aaattaaaaa tgaagtttta aatcaatcta aagtatatat 5500
   gagtaaactt ggtctgacag ttaccaatgc ttaatcagtg aggcacctat ctcagcgatc 5560
   tgtctatttc gttcatccat agttgcctga ctccccgtcg tgtagataac tacgatacgg 5620
   gagggcttac catctggccc cagtgctgca atgataccgc gagacccacg ctcaccggct 5680
   ccagatttat cagcaataaa ccagccagcc ggaagggccg agcgcagaag tggtcctgca 5740
   actttatccg cctccatcca gtctattaat tgttgccggg aagctagagt aagtagttcg 5800
   ccagttaata gtttgcgcaa cgttgttgcc attgctacag gcatcgtggt gtcacgctcg 5860
   tcgtttggta tggcttcatt cagctccggt tcccaacgat caaggcgagt tacatgatcc 5920
   cccatgttgt gcaaaaaagc gggttagctc cttcggtcct ccgatcgttg tcagaagtaa 5980
   gttggccgca gtgttatcac tcatggttat ggcagcactg cataattctc ttactgtcat 6040
   gccatccgta agatgctttt ctgtgactgg tgagtactca accaagtcat tctgagaata 6100
   gtgtatgcgg cgaccgagtt gctcttgccc ggcgtcaata cgggataata ccgcgccaca 6160
   tagcagaact ttaaaagtgc tcatcattgg aaaacgttct tcggggcgaa aactctcaag 6220
   gatcttaccg ctgttgagat ccagttcgat gtaacccact cgtgcaccca actgatcttc 6280
   agcatctttt actttcacca gcgtttctgg gtgagcaaaa acaggaaggc aaaatgccgc 6340
   aaaaaaggga ataagggcga cacggaaatg ttgaatactc atactcttcc tttttcaata 6400
   ttattgaagc atttatcagg gttattgtct catgagcgga tacatatttg aatgtattta 6460
   gaaaaataaa caaatagggg ttccgcgcac atttccccga aaagtgccac ctaaattgta 6520
   agcgttaata ttttgttaaa attcgcgtta aatttttgtt aaatcagctc attttttaac 6580
   caataggccg aaatcggcaa aatcccttat aaatcaaaag aatagaccga gatagggttg 6640
   agtgttgttc cagtttggaa caagagtcca ctattaaaga acgtggactc caacgtcaaa 6700
   gggcgaaaaa ccgtctatca gggcgatggc ccactacgtg aaccatcacc ctaatcaagt 6760
   tttttggggt cgaggtgccg taaagcacta aatcggaacc ctaaagggag cccccgattt 6820
   agagcttgac ggggaaagcc ggcgaacgtg gcgagaaagg aagggaagaa agcgaaagga 6880
   gcgggcgcta gggcgctggc aagtgtagcg gtcacgctgc gcgtaaccac cacacccgcc 6940
   gcgcttaatg cgccgctaca gggcgcgtcc cattcgccat tcaggctgcg caactgttgg 7000
   gaagggcgat cggtgcgggc ctcttcgcta ttacgccagc tggcgaaagg gggatgtgct 7060
   gcaaggcgat taagttgggt aacgccaggg ttttcccagt cacgacgttg taaaacgacg 7120
   gccagtgagc gcgcgtaata cgactcacta tagggcgaat tggagctcca ccgcggtg 7178
<210> 13
   <211> 425
   <212> PRT
   <213> Artificial
<220>
   <223> phCMV-deltaCE2-1a plasmid
<400> 13
   Met Asn Ser Asp Leu Met Gly Tyr Ile Pro Leu Val Gly Ala Pro Leu
   1               5                   10                  15
   Gly Gly Ala Ala Arg Ala Leu Ala His Gly Val Arg Val Leu Glu Asp
               20                  25                  30
   Gly Val Asn Tyr Ala Thr Gly Asn Leu Pro Gly Cys Phe Phe Ser Ile
           35                  40                  45
   Phe Leu Leu Ala Leu Leu Ser Cys Leu Thr Val Pro Ala Ser Ala Glu
       50                  55                  60
   Thr His Val Thr Gly Gly Ser Ala Gly His Thr Thr Ala Gly Leu Val
   65                  70                  75                  80
   Gly Leu Leu Thr Pro Gly Ala Lys Gln Asn Ile Gln Leu Ile Asn Thr
                   85                  90                  95
   Asn Gly Ser Trp His Ile Asn Ser Thr Ala Leu Asn Cys Asn Asp Ser
               100                 105                 110
   Leu Thr Thr Gly Trp Leu Ala Gly Leu Phe Tyr Arg His Lys Phe Asn
           115                 120                 125
   Ser Ser Gly Cys Pro Glu Arg Leu Ala Ser Cys Arg Arg Leu Thr Asp
       130                 135                 140
   Phe Ala Gln Gly Trp Gly Pro Ile Ser Tyr Ala Asn Gly Ser Gly Leu
   145                 150                 155                 160
   Asp Glu Arg Pro Tyr Cys Trp His Tyr Pro Pro Arg Pro Cys Gly Ile
                   165                 170                 175
   Val Pro Ala Lys Ser Val Cys Gly Pro Val Tyr Cys Phe Thr Pro Ser
               180                 185                 190
   Pro Val Val Val Gly Thr Thr Asp Arg Ser Gly Ala Pro Thr Tyr Ser
           195                 200                 205
   Trp Gly Ala Asn Asp Thr Asp Val Phe Val Leu Asn Asn Thr Arg Pro
       210                 215                 220
   Pro Leu Gly Asn Trp Phe Gly Cys Thr Trp Met Asn Ser Thr Gly Phe
   225                 230                 235                 240
   Thr Lys Val Cys Gly Ala Pro Pro Cys Val Ile Gly Gly Val Gly Asn
                   245                 250                 255
   Asn Thr Leu Leu Cys Pro Thr Asp Cys Phe Arg Lys His Pro Glu Ala
               260                 265                 270
   Thr Tyr Ser Arg Cys Gly Ser Gly Pro Trp Ile Thr Pro Arg Cys Met
           275                 280                 285
   Val Asp Tyr Pro Tyr Arg Leu Trp His Tyr Pro Cys Thr Ile Asn Tyr
       290                 295                 300
   Thr Ile Phe Lys Val Arg Met Tyr Val Gly Gly Val Glu His Arg Leu
   305                 310                 315                 320
   Glu Ala Ala Cys Asn Trp Thr Arg Gly Glu Arg Cys Asp Leu Glu Asp
                   325                 330                 335
   Arg Asp Arg Ser Glu Leu Ser Pro Leu Leu Leu Ser Thr Thr Gln Trp
               340                 345                 350
   Gln Val Leu Pro Cys Ser Phe Thr Thr Leu Pro Ala Leu Ser Thr Gly
           355                 360                 365
   Leu Ile His Leu His Gln Asn Ile Val Asp Val Gln Tyr Leu Tyr Gly
       370                 375                 380
   Val Gly Ser Ser Ile Ala Ser Trp Ala Ile Lys Trp Glu Tyr Val Val
   385                 390                 395                 400
   Leu Leu Phe Leu Leu Leu Ala Asp Ala Arg Val Cys Ser Cys Leu Trp
                   405                 410                 415
   Met Met Leu Leu Ile Ser Gln Ala Glu
               420                 425
<210> 14
   <211> 7551
   <212> DNA
   <213> Artificial
<220>
   <223> phCMV-DCE1E2p7 plasmid
<400> 14
   gcggccgctc tagagagctt ggcccattgc atacgttgta tccatatcat aatatgtaca 60
   tttatattgg ctcatgtcca acattaccgc catgttgaca ttgattattg actagttatt 120
   aatagtaatc aattacgggg tcattagttc atagcccata tatggagttc cgcgttacat 180
   aacttacggt aaatggcccg cctggctgac cgcccaacga cccccgccca ttgacgtcaa 240
   taatgacgta tgttcccata gtaacgccaa tagggacttt ccattgacgt caatgggtgg 300
   agtatttacg gtaaactgcc cacttggcag tacatcaagt gtatcatatg ccaagtacgc 360
   cccctattga cgtcaatgac ggtaaatggc ccgcctggca ttatgcccag tacatgacct 420
   tatgggactt tcctacttgg cagtacatct acgtattagt catcgctatt accatggtga 480
   tgcggttttg gcagtacatc aatgggcgtg gatagcggtt tgactcacgg ggatttccaa 540
   gtctccaccc cattgacgtc aatgggagtt tgttttggca ccaaaatcaa cgggactttc 600
   caaaatgtcg taacaactcc gccccattga cgcaaatggg cggtaggcgt gtacggtggg 660
   aggtctatat aagcagagct cgtttagtga accgtcagat cgcctggaga cgccatccac 720
   gctgttttga cctccataga agacaccggg accgatccag cctccggtcg accgatcctg 780
   agaacttcag ggtgagtttg gggacccttg attgttcttt ctttttcgct attgtaaaat 840
   tcatgttata tggagggggc aaagttttca gggtgttgtt tagaatggga agatgtccct 900
   tgtatcacca tggaccctca tgataatttt gtttctttca ctttctactc tgttgacaac 960
   cattgtctcc tcttattttc ttttcatttt ctgtaacttt ttcgttaaac tttagcttgc 1020
   atttgtaacg aatttttaaa ttcacttttg tttatttgtc agattgtaag tactttctct 1080
   aatcactttt ttttcaaggc aatcagggta tattatattg tacttcagca cagttttaga 1140
   gaacaattgt tataattaaa tgataaggta gaatatttct gcatataaat tctggctggc 1200
   gtggaaatat tcttattggt agaaacaact acaccctggt catcatcctg cctttctctt 1260
   tatggttaca atgatataca ctgtttgaga tgaggataaa atactctgag tccaaaccgg 1320
   gcccctctgc taaccatgtt catgccttct tctctttcct acagctcctg ggcaacgtgc 1380
   tggttgttgt gctgtctcat cattttggca aagaattcct cgacggatcc gatgaaggac 1440
   agttctttcc agacattgtt gaattgatct cgatcccgcg aaattaatac gactcactat 1500
   agggagacca caacggtttc cctctagcgg gatcaattcc gcccctctcc ctcccccccc 1560
   cctaacgtta ctggccgaag ccgcttggaa taaggccggt gtgcgtttgt ctatatgtta 1620
   ttttccacca tattgccgtc ttttggcaat gtgagggccc ggaaacctgg ccctgtcttc 1680
   ttgacgagca ttcctagggg tctttcccct ctcgccaaag gaatgcaagg tctgttgaat 1740
   gtcgtgaagg aagcagttcc tctggaagct tcttgaagac aaacaacgtc tgtagcgacc 1800
   ctttgcaggc agcggaaccc cccacctggc gacaggtgcc tctgcggcca aaagccacgt 1860
   gtataagata cacctgcaaa ggcggcacaa ccccagtgcc acgttgtgag ttggatagtt 1920
   gtggaaagag tcaaatggct ctcctcaagc gtattcaaca aggggctgaa ggatgcccag 1980
   aaggtacccc attgtatggg atctgatctg gggcctcggt gcacatgctt tacatgtgtt 2040
   tagtcgaggt taaaaaacgt ctaggccccc cgaaccacgg ggacgtggtt ttcctttgaa 2100
   aaacacgata ataccatgaa ttccgacctc atggggtaca taccgctcgt cggcgcccct 2160
   cttggaggcg ctgccagggc cctggcgcat ggcgtccggg ttctggaaga cggcgtgaac 2220
   tatgcaacag ggaaccttcc tggttgcttt ttctctatct tccttctggc cctgctctct 2280
   tgcctgactg tgcccgcttc agcctaccaa gtgcgcaatt cctcggggct ttaccatgtc 2340
   accaatgatt gccctaattc gagtattgtg tacgaggcgg ccgatgccat cctgcacact 2400
   ccggggtgtg tcccttgcgt tcgcgagggt aacgcctcga ggtgttgggt ggcggtgacc 2460
   cccacggtgg ccaccaggga cggcaaactc cccacaacgc agcttcgacg tcatatcgat 2520
   ctgcttgtcg ggagcgccac cctctgctca gccctctacg tgggggacct gtgcgggtct 2580
   gtttttcttg ttggtcaact gtttaccttc tctcccaggc gccactggac gacgcaaagc 2640
   tgcaattgtt ctatctatcc cggccatata acgggtcatc gcatggcatg ggatatgatg 2700
   atgaactggt cccctacggc agcgttggtg gtagctcagc tgctccggat cccacaagcc 2760
   atcatggaca tgatcgctgg tgctcactgg ggagtcctgg cgggcatagc gtatttctcc 2820
   atggtgggga actgggcgaa ggtcctggta gtgctgctgt tatttgccgg cgtcgacgcg 2880
   gaaacccacg tcaccggggg aagtgccggc cacaccacgg ctgggcttgt tggtctcctt 2940
   acaccaggcg ccaagcagaa catccaactg atcaacacca acggcagttg gcacatcaat 3000
   agcacggcct tgaactgcaa cgatagcctt accaccggct ggttagcagg gctcttctat 3060
   cgccacaaat tcaactcttc aggctgtcct gagaggttgg ccagctgccg acgccttacc 3120
   gattttgccc agggctgggg tcccatcagt tatgccaacg gaagcggcct tgacgaacgc 3180
   ccctactgtt ggcactaccc tccaagacct tgtggcattg tgcccgcaaa gagcgtgtgt 3240
   ggcccggtat attgcttcac tcccagcccc gtggtggtgg gaacgaccga caggtcgggc 3300
   gcgcctacct acagctgggg tgcaaatgat acggatgtct tcgtccttaa caacaccagg 3360
   ccaccgctgg gcaattggtt cggttgtacc tggatgaact caactggatt caccaaagtg 3420
   tgcggagcgc ccccttgtgt catcggaggg gtgggcaaca acaccttgct ctgccccact 3480
   gattgcttcc gcaaacatcc ggaagccaca tactctcggt gcggctccgg tccctggatt 3540
   acacccaggt gcatggtcga ctacccgtat aggctttggc actatccttg tactatcaat 3600
   tacaccatat tcaaagtcag gatgtacgtg ggaggggtcg agcacaggct ggaagcggcc 3660
   tgcaactgga cgcggggcga acgctgtgat ctggaagaca gggacaggtc cgagctcagc 3720
   ccattgctgc tgtccaccac acagtggcag gtccttccgt gttctttcac gaccctgcca 3780
   gccttgtcca ccggcctcat ccacctccac cagaacattg tggacgtgca gtacttgtac 3840
   ggggtggggt caagcatcgc gtcctgggcc attaagtggg agtacgtcgt tctcctgttc 3900
   cttctgcttg cagacgcgcg cgtctgctcc tgcttgtgga tgatgttact catatcccaa 3960
   gcggaggcgg ctttggagaa cctcgtaata ctcaatgcag catccctggc cgggacgcac 4020
   ggtcttgtgt ccttcctcgt gttcttctgc tttgcgtggt atctgaaggg taggtgggtg 4080
   cccggagcgg tctacgcctt ctacgggatg tggcctctcc tcctgctcct gctggcgttg 4140
   cctcagcggg catacgcatg aagatccctg cagctcgaga ggtcaggtgc aggctgccta 4200
   tcagaaggtg gtggctggtg tggccaatgc cctggctcac aaataccact gagatctttt 4260
   tccctctgcc aaaaattatg gggacatcat gaagcccctt gagcatctga cttctggcta 4320
   ataaaggaaa tttattttca ttgcaatagt gtgttggaat tttttgtgtc tctcactcgg 4380
   aaggacatat gggagggcaa atcatttaaa acatcagaat gagtatttgg tttagagttt 4440
   ggcaacatat gcccatatgc tggctgccat gaacaaaggt tggctataaa gaggtcatca 4500
   gtatatgaaa cagccccctg ctgtccattc cttattccat agaaaagcct tgacttgagg 4560
   ttagattttt tttatatttt gttttgtgtt atttttttct ttaacatccc taaaattttc 4620
   cttacatgtt ttactagcca gatttttcct cctctcctga ctactcccag tcatagctgt 4680
   ccctcttctc ttatggagat ccctcgacgg atcggccgca attcgtaatc atgtcatagc 4740
   tgtttcctgt gtgaaattgt tatccgctca caattccaca caacatacga gccggaagca 4800
   taaagtgtaa agcctggggt gcctaatgag tgagctaact cacattaatt gcgttgcgct 4860
   cactgcccgc tttccagtcg ggaaacctgt cgtgccagct gcattaatga atcggccaac 4920
   gcgcggggag aggcggtttg cgtattgggc gctcttccgc ttcctcgctc actgactcgc 4980
   tgcgctcggt cgttcggctg cggcgagcgg tatcagctca ctcaaaggcg gtaatacggt 5040
   tatccacaga atcaggggat aacgcaggaa agaacatgtg agcaaaaggc cagcaaaagg 5100
   ccaggaaccg taaaaaggcc gcgttgctgg cgtttttcca taggctccgc ccccctgacg 5160
   agcatcacaa aaatcgacgc tcaagtcaga ggtggcgaaa cccgacagga ctataaagat 5220
   accaggcgtt tccccctgga agctccctcg tgcgctctcc tgttccgacc ctgccgctta 5280
   ccggatacct gtccgccttt ctcccttcgg gaagcgtggc gctttctcat agctcacgct 5340
   gtaggtatct cagttcggtg taggtcgttc gctccaagct gggctgtgtg cacgaacccc 5400
   ccgttcagcc cgaccgctgc gccttatccg gtaactatcg tcttgagtcc aacccggtaa 5460
   gacacgactt atcgccactg gcagcagcca ctggtaacag gattagcaga gcgaggtatg 5520
   taggcggtgc tacagagttc ttgaagtggt ggcctaacta cggctacact agaagaacag 5580
   tatttggtat ctgcgctctg ctgaagccag ttaccttcgg aaaaagagtt ggtagctctt 5640
   gatccggcaa acaaaccacc gctggtagcg gtggtttttt tgtttgcaag cagcagatta 5700
   cgcgcagaaa aaaaggatct caagaagatc ctttgatctt ttctacgggg tctgacgctc 5760
   agtggaacga aaactcacgt taagggattt tggtcatgag attatcaaaa aggatcttca 5820
   cctagatcct tttaaattaa aaatgaagtt ttaaatcaat ctaaagtata tatgagtaaa 5880
   cttggtctga cagttaccaa tgcttaatca gtgaggcacc tatctcagcg atctgtctat 5940
   ttcgttcatc catagttgcc tgactccccg tcgtgtagat aactacgata cgggagggct 6000
   taccatctgg ccccagtgct gcaatgatac cgcgagaccc acgctcaccg gctccagatt 6060
   tatcagcaat aaaccagcca gccggaaggg ccgagcgcag aagtggtcct gcaactttat 6120
   ccgcctccat ccagtctatt aattgttgcc gggaagctag agtaagtagt tcgccagtta 6180
   atagtttgcg caacgttgtt gccattgcta caggcatcgt ggtgtcacgc tcgtcgtttg 6240
   gtatggcttc attcagctcc ggttcccaac gatcaaggcg agttacatga tcccccatgt 6300
   tgtgcaaaaa agcgggttag ctccttcggt cctccgatcg ttgtcagaag taagttggcc 6360
   gcagtgttat cactcatggt tatggcagca ctgcataatt ctcttactgt catgccatcc 6420
   gtaagatgct tttctgtgac tggtgagtac tcaaccaagt cattctgaga atagtgtatg 6480
   cggcgaccga gttgctcttg cccggcgtca atacgggata ataccgcgcc acatagcaga 6540
   actttaaaag tgctcatcat tggaaaacgt tcttcggggc gaaaactctc aaggatctta 6600
   ccgctgttga gatccagttc gatgtaaccc actcgtgcac ccaactgatc ttcagcatct 6660
   tttactttca ccagcgtttc tgggtgagca aaaacaggaa ggcaaaatgc cgcaaaaaag 6720
   ggaataaggg cgacacggaa atgttgaata ctcatactct tcctttttca atattattga 6780
   agcatttatc agggttattg tctcatgagc ggatacatat ttgaatgtat ttagaaaaat 6840
   aaacaaatag gggttccgcg cacatttccc cgaaaagtgc cacctaaatt gtaagcgtta 6900
   atattttgtt aaaattcgcg ttaaattttt gttaaatcag ctcatttttt aaccaatagg 6960
   ccgaaatcgg caaaatccct tataaatcaa aagaatagac cgagataggg ttgagtgttg 7020
   ttccagtttg gaacaagagt ccactattaa agaacgtgga ctccaacgtc aaagggcgaa 7080
   aaaccgtcta tcagggcgat ggcccactac gtgaaccatc accctaatca agttttttgg 7140
   ggtcgaggtg ccgtaaagca ctaaatcgga accctaaagg gagcccccga tttagagctt 7200
   gacggggaaa gccggcgaac gtggcgagaa aggaagggaa gaaagcgaaa ggagcgggcg 7260
   ctagggcgct ggcaagtgta gcggtcacgc tgcgcgtaac caccacaccc gccgcgctta 7320
   atgcgccgct acagggcgcg tcccattcgc cattcaggct gcgcaactgt tgggaagggc 7380
   gatcggtgcg ggcctcttcg ctattacgcc agctggcgaa agggggatgt gctgcaaggc 7440
   gattaagttg ggtaacgcca gggttttccc agtcacgacg ttgtaaaacg acggccagtg 7500
   agcgcgcgta atacgactca ctatagggcg aattggagct ccaccgcggt g 7551
<210> 15
   <211> 682
   <212> PRT
   <213> Artificial
<220>
   <223> delta CE E2p7 polyprotein
<400> 15
   Met Asn Ser Asp Leu Met Gly Tyr Ile Pro Leu Val Gly Ala Pro Leu
   1               5                   10                  15
   Gly Gly Ala Ala Arg Ala Leu Ala His Gly Val Arg Val Leu Glu Asp
               20                  25                  30
   Gly Val Asn Tyr Ala Thr Gly Asn Leu Pro Gly Cys Phe Phe Ser Ile
           35                  40                  45
   Phe Leu Leu Ala Leu Leu Ser Cys Leu Thr Val Pro Ala Ser Ala Tyr
       50                  55                  60
   Gln Val Arg Asn Ser Ser Gly Leu Tyr His Val Thr Asn Asp Cys Pro
   65                  70                  75                  80
   Asn Ser Ser Ile Val Tyr Glu Ala Ala Asp Ala Ile Leu His Thr Pro
                   85                  90                  95
   Gly Cys Val Pro Cys Val Arg Glu Gly Asn Ala Ser Arg Cys Trp Val
               100                 105                 110
   Ala Val Thr Pro Thr Val Ala Thr Arg Asp Gly Lys Leu Pro Thr Thr
           115                 120                 125
   Gln Leu Arg Arg His Ile Asp Leu Leu Val Gly Ser Ala Thr Leu Cys
       130                 135                 140
   Ser Ala Leu Tyr Val Gly Asp Leu Cys Gly Ser Val Phe Leu Val Gly
   145                 150                 155                 160
   Gln Leu Phe Thr Phe Ser Pro Arg Arg His Trp Thr Thr Gln Ser Cys
                   165                 170                 175
   Asn Cys Ser Ile Tyr Pro Gly His Ile Thr Gly His Arg Met Ala Trp
   180 185 190
   Asp Met Met Met Asn Trp Ser Pro Thr Ala Ala Leu Val Val Ala Gln
   195 200 205
   Leu Leu Arg Ile Pro Gln Ala Ile Met Asp Met Ile Ala Gly Ala His
   210 215 220
   Trp Gly Val Leu Ala Gly Ile Ala Tyr Phe Ser Met Val Gly Asn Trp
   225 230 235 240
   Ala Lys Val Leu Val Val Leu Leu Leu Phe Ala Gly Val Asp Ala Glu
   245 250 255
   Thr His Val Thr Gly Gly Ser Ala Gly His Thr Thr Ala Gly Leu Val
   260 265 270
   Gly Leu Leu Thr Pro Gly Ala Lys Gln Asn Ile Gln Leu Ile Asn Thr
   275 280 285
   Asn Gly Ser Trp His Ile Asn Ser Thr Ala Leu Asn Cys Asn Asp Ser
   290 295 300
   Leu Thr Thr Gly Trp Leu Ala Gly Leu Phe Tyr Arg His Lys Phe Asn
   305 310 315 320
   Ser Ser Gly Cys Pro Glu Arg Leu Ala Ser Cys Arg Arg Leu Thr Asp
   325 330 335
   Phe Ala Gln Gly Trp Gly Pro Ile Ser Tyr Ala Asn Gly Ser Gly Leu
   340 345 350
   Asp Glu Arg Pro Tyr Cys Trp His Tyr Pro Pro Arg Pro Cys Gly Ile
   355 360 365
   Val Pro Ala Lys Ser Val Cys Gly Pro Val Tyr Cys Phe Thr Pro Ser
   370 375 380
   Pro Val Val Val Gly Thr Thr Asp Arg Ser Gly Ala Pro Thr Tyr Ser
   385 390 395 400
   Trp Gly Ala Asn Asp Thr Asp Val Phe Val Leu Asn Asn Thr Arg Pro
   405 410 415
   Pro Leu Gly Asn Trp Phe Gly Cys Thr Trp Met Asn Ser Thr Gly Phe
   420 425 430
   Thr Lys Val Cys Gly Ala Pro Pro Cys Val Ile Gly Gly Val Gly Asn
   435 440 445
   Asn Thr Leu Leu Cys Pro Thr Asp Cys Phe Arg Lys His Pro Glu Ala
   450 455 460
   Thr Tyr Ser Arg Cys Gly Ser Gly Pro Trp Ile Thr Pro Arg Cys Met
   465 470 475 480
   Val Asp Tyr Pro Tyr Arg Leu Trp His Tyr Pro Cys Thr Ile Asn Tyr
   485 490 495
   Thr Ile Phe Lys Val Arg Met Tyr Val Gly Gly Val Glu His Arg Leu
   500 505 510
   Glu Ala Ala Cys Asn Trp Thr Arg Gly Glu Arg Cys Asp Leu Glu Asp
   515 520 525
   Arg Asp Arg Ser Glu Leu Ser Pro Leu Leu Leu Ser Thr Thr Gln Trp
   530 535 540
   Gln Val Leu Pro Cys Ser Phe Thr Thr Leu Pro Ala Leu Ser Thr Gly
   545 550 555 560
   Leu Ile His Leu His Gln Asn Ile Val Asp Val Gln Tyr Leu Tyr Gly
   565 570 575
   Val Gly Ser Ser Ile Ala Ser Trp Ala Ile Lys Trp Glu Tyr Val Val
   580 585 590
   Leu Leu Phe Leu Leu Leu Ala Asp Ala Arg Val Cys Ser Cys Leu Trp
   595 600 605
   Met Met Leu Leu Ile Ser Gln Ala Glu Ala Ala Leu Glu Asn Leu Val
   610 615 620
   Ile Leu Asn Ala Ala Ser Leu Ala Gly Thr His Gly Leu Val Ser Phe
   625 630 635 640
   Leu Val Phe Phe Cys Phe Ala Trp Tyr Leu Lys Gly Arg Trp Val Pro
   645 650 655
   Gly Ala Val Tyr Ala Phe Tyr Gly Met Trp Pro Leu Leu Leu Leu Leu
   660 665 670
   Leu Ala Leu Pro Gln Arg Ala Tyr Ala Glx
   675 680
<210> 16
   <211> 3011
   <212> PRT
   <213> Hepatitis C virus
<220>
   <221> MISC_FEATURE
   <222> (132)..(191)
   <223> delta C
<220>
   <221> MISC_FEATURE
   <222> (171)..(191)
   <223> signal peptide
<220>
   <221> MISC_FEATURE
   <222> (192)..(383)
   <223> E1
<220>
   <221> MISC_FEATURE
   <222> (384)..(746)
   <223> E2
<220>
   <221> MISC_FEATURE
   <222> (384)..(410)
   <223> HRV1
<220>
   <221> MISC_FEATURE
   <222> (747)..(809)
   <223> p7
<400> 16
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Val Pro Ala Ser Ala Tyr
   180 185 190
   Gln Val Arg Asn Ser Ser Gly Leu Tyr His Val Thr Asn Asp Cys Pro
   195 200 205
   Asn Ser Ser Ile Val Tyr Glu Ala Ala Asp Ala Ile Leu His Thr Pro
   210 215 220
   Gly Cys Val Pro Cys Val Arg Glu Gly Asn Ala Ser Arg Cys Trp Val
   225 230 235 240
   Ala Val Thr Pro Thr Val Ala Thr Arg Asp Gly Lys Leu Pro Thr Thr
   245 250 255
   Gln Leu Arg Arg His Ile Asp Leu Leu Val Gly Ser Ala Thr Leu Cys
   260 265 270
   Ser Ala Leu Tyr Val Gly Asp Leu Cys Gly Ser Val Phe Leu Val Gly
   275 280 285
   Gln Leu Phe Thr Phe Ser Pro Arg Arg His Trp Thr Thr Gln Asp Cys
   290 295 300
   Asn Cys Ser Ile Tyr Pro Gly His Ile Thr Gly His Arg Met Ala Trp
   305 310 315 320
   Asp Met Met Met Asn Trp Ser Pro Thr Ala Ala Leu Val Val Ala Gln
   325 330 335
   Leu Leu Arg Ile Pro Gln Ala Ile Met Asp Met Ile Ala Gly Ala His
   340 345 350
   Trp Gly Val Leu Ala Gly Ile Ala Tyr Phe Ser Met Val Gly Asn Trp
   355 360 365
   Ala Lys Val Leu Val Val Leu Leu Leu Phe Ala Gly Val Asp Ala Glu
   370 375 380
   Thr His Val Thr Gly Gly Ser Ala Gly Arg Thr Thr Ala Gly Leu Val
   385 390 395 400
   Gly Leu Leu Thr Pro Gly Ala Lys Gln Asn Ile Gln Leu Ile Asn Thr
   405 410 415
   Asn Gly Ser Trp His Ile Asn Ser Thr Ala Leu Asn Cys Asn Glu Ser
   420 425 430
   Leu Asn Thr Gly Trp Leu Ala Gly Leu Phe Tyr Gln His Lys Phe Asn
   435 440 445
   Ser Ser Gly Cys Pro Glu Arg Leu Ala Ser Cys Arg Arg Leu Thr Asp
   450 455 460
   Phe Ala Gln Gly Trp Gly Pro Ile Ser Tyr Ala Asn Gly Ser Gly Leu
   465 470 475 480
   Asp Glu Arg Pro Tyr Cys Trp His Tyr Pro Pro Arg Pro Cys Gly Ile
   485 490 495
   Val Pro Ala Lys Ser Val Cys Gly Pro Val Tyr Cys Phe Thr Pro Ser
   500 505 510
   Pro Val Val Val Gly Thr Thr Asp Arg Ser Gly Ala Pro Thr Tyr Ser
   515 520 525
   Trp Gly Ala Asn Asp Thr Asp Val Phe Val Leu Asn Asn Thr Arg Pro
   530 535 540
   Pro Leu Gly Asn Trp Phe Gly Cys Thr Trp Met Asn Ser Thr Gly Phe
   545 550 555 560
   Thr Lys Val Cys Gly Ala Pro Pro Cys Val Ile Gly Gly Val Gly Asn
   565 570 575
   Asn Thr Leu Leu Cys Pro Thr Asp Cys Phe Arg Lys His Pro Glu Ala
   580 585 590
   Thr Tyr Ser Arg Cys Gly Ser Gly Pro Trp Ile Thr Pro Arg Cys Met
   595 600 605
   Val Asp Tyr Pro Tyr Arg Leu Trp His Tyr Pro Cys Thr Ile Asn Tyr
   610 615 620
   Thr Ile Phe Lys Val Arg Met Tyr Val Gly Gly Val Glu His Arg Leu
   625 630 635 640
   Glu Ala Ala Cys Asn Trp Thr Arg Gly Glu Arg Cys Asp Leu Glu Asp
   645 650 655
   Arg Asp Arg Ser Glu Leu Ser Pro Leu Leu Leu Ser Thr Thr Gln Trp
   660 665 670
   Gln Val Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Ser Ile Ala Ser Trp Ala Ile Lys Trp Glu Tyr Val Val
   705 710 715 720
   Leu Leu Phe Leu Leu Leu Ala Asp Ala Arg Val Cys Ser Cys Leu Trp
   725 730 735
   Met Met Leu Leu Ile Ser Gln Ala Glu Ala Ala Leu Glu Asn Leu Val
   740 745 750
   Ile Leu Asn Ala Ala Ser Leu Ala Gly Thr His Gly Leu Val Ser Phe
   755 760 765
   Leu Val Phe Phe Cys Phe Ala Trp Tyr Leu Lys Gly Arg Trp Val Pro
   770 775 780
   Gly Ala Val Tyr Ala Phe Tyr Gly Met Trp Pro Leu Leu Leu Leu Leu
   785 790 795 800
   Leu Ala Leu Pro Gln Arg Ala Tyr Ala Leu Asp Thr Glu Val Ala Ala
   805 810 815
   Ser Cys Gly Gly Val Val Leu Val Gly Leu Met Ala Leu Thr Leu Ser
   820 825 830
   Pro Tyr Tyr Lys Arg Tyr Ile Ser Trp Cys Met Trp Trp Leu Gln Tyr
   835 840 845
   Phe Leu Thr Arg Val Glu Ala Gln Leu His Val Trp Val Pro Pro Leu
   850 855 860
   Asn Val Arg Gly Gly Arg Asp Ala Val Ile Leu Leu Met Cys Val Val
   865 870 875 880
   His Pro Thr Leu Val Phe Asp Ile Thr Lys Leu Leu Leu Ala Ile Phe
   885 890 895
   Gly Pro Leu Trp Ile Leu Gln Ala Ser Leu Leu Lys Val Pro Tyr Phe
   900 905 910
   Val Arg Val Gln Gly Leu Leu Arg Ile Cys Ala Leu Ala Arg Lys Ile
   915 920 925
   Ala Gly Gly His Tyr Val Gln Met Ala Ile Ile Lys Leu Gly Ala Leu
   930 935 940
   Thr Gly Thr Tyr Val Tyr Asn His Leu Thr Pro Leu Arg Asp Trp Ala
   945 950 955 960
   His Asn Gly Leu Arg Asp Leu Ala Val Ala Val Glu Pro Val Val Phe
   965 970 975
   Ser Arg Met Glu Thr Lys Leu Ile Thr Trp Gly Ala Asp Thr Ala Ala
   980 985 990
   Cys Gly Asp Ile Ile Asn Gly Leu Pro Val Ser Ala Arg Arg Gly Gln
   995 1000 1005
   Glu Ile Leu Leu Gly Pro Ala Asp Gly Met Val Ser Lys Gly Trp
   1010 1015 1020
   Arg Leu Leu Ala Pro Ile Thr Ala Tyr Ala Gln Gln Thr Arg Gly
   1025 1030 1035
   Leu Leu Gly Cys Ile Ile Thr Ser Leu Thr Gly Arg Asp Lys Asn
   1040 1045 1050
   Gln Val Glu Gly Glu Val Gln Ile Val Ser Thr Ala Thr Gln Thr
   1055 1060 1065
   Phe Leu Ala Thr Cys Ile Asn Gly Val Cys Trp Thr Val Tyr His
   1070 1075 1080
   Gly Ala Gly Thr Arg Thr Ile Ala Ser Pro Lys Gly Pro Val Ile
   1085 1090 1095
   Gln Met Tyr Thr Asn Val Asp Gln Asp Leu Val Gly Trp Pro Ala
   1100 1105 1110
   Pro Gln Gly Ser Arg Ser Leu Thr Pro Cys Thr Cys Gly Ser Ser
   1115 1120 1125
   Asp Leu Tyr Leu Val Thr Arg His Ala Asp Val Ile Pro Val Arg
   1130 1135 1140
   Arg Arg Gly Asp Ser Arg Gly Ser Leu Leu Ser Pro Arg Pro Ile
   1145 1150 1155
   Ser Tyr Leu Lys Gly Ser Ser Gly Gly Pro Leu Leu Cys Pro Ala
   1160 1165 1170
   Gly His Ala Val Gly Leu Phe Arg Ala Ala Val Cys Thr Arg Gly
   1175 1180 1185
   Val Ala Lys Ala Val Asp Phe Ile Pro Val Glu Asn Leu Glu Thr
   1190 1195 1200
   Thr Met Arg Ser Pro Val Phe Thr Asp Asn Ser Ser Pro Pro Ala
   1205 1210 1215
   Val Pro Gln Ser Phe Gln Val Ala His Leu His Ala Pro Thr Gly
   1220 1225 1230
   Ser Gly Lys Ser Thr Lys Val Pro Ala Ala Tyr Ala Ala Gln Gly
   1235 1240 1245
   Tyr Lys Val Leu Val Leu Asn Pro Ser Val Ala Ala Thr Leu Gly
   1250 1255 1260
   Phe Gly Ala Tyr Met Ser Lys Ala His Gly Val Asp Pro Asn Ile
   1265 1270 1275
   Arg Thr Gly Val Arg Thr Ile Thr Thr Gly Ser Pro Ile Thr Tyr
   1280 1285 1290
   Ser Thr Tyr Gly Lys Phe Leu Ala Asp Gly Gly Cys Ser Gly Gly
   1295 1300 1305
   Ala Tyr Asp Ile Ile Ile Cys Asp Glu Cys His Ser Thr Asp Ala
   1310 1315 1320
   Thr Ser Ile Leu Gly Ile Gly Thr Val Leu Asp Gln Ala Glu Thr
   1325 1330 1335
   Ala Gly Ala Arg Leu Val Val Leu Ala Thr Ala Thr Pro Pro Gly
   1340 1345 1350
   Ser Val Thr Val Ser His Pro Asn Ile Glu Glu Val Ala Leu Ser
   1355 1360 1365
   Thr Thr Gly Glu Ile Pro Phe Tyr Gly Lys Ala Ile Pro Leu Glu
   1370 1375 1380
   Val Ile Lys Gly Gly Arg His Leu Ile Phe Cys His Ser Lys Lys
   1385 1390 1395
   Lys Cys Asp Glu Leu Ala Ala Lys Leu Val Ala Leu Gly Ile Asn
   1400 1405 1410
   Ala Val Ala Tyr Tyr Arg Gly Leu Asp Val Ser Val Ile Pro Thr
   1415 1420 1425
   Ser Gly Asp Val Val Val Val Ser Thr Asp Ala Leu Met Thr Gly
   1430 1435 1440
   Phe Thr Gly Asp Phe Asp Ser Val Ile Asp Cys Asn Thr Cys Val
   1445 1450 1455
   Thr Gln Thr Val Asp Phe Ser Leu Asp Pro Thr Phe Thr Ile Glu
   1460 1465 1470
   Thr Thr Thr Leu Pro Gln Asp Ala Val Ser Arg Thr Gln Arg Arg
   1475 1480 1485
   Gly Arg Thr Gly Arg Gly Lys Pro Gly Ile Tyr Arg Phe Val Ala
   1490 1495 1500
   Pro Gly Glu Arg Pro Ser Gly Met Phe Asp Ser Ser Val Leu Cys
   1505 1510 1515
   Glu Cys Tyr Asp Ala Gly Cys Ala Trp Tyr Glu Leu Thr Pro Ala
   1520 1525 1530
   Glu Thr Thr Val Arg Leu Arg Ala Tyr Met Asn Thr Pro Gly Leu
   1535 1540 1545
   Pro Val Cys Gln Asp His Leu Glu Phe Trp Glu Gly Val Phe Thr
   1550 1555 1560
   Gly Leu Thr His Ile Asp Ala His Phe Leu Ser Gln Thr Lys Gln
   1565 1570 1575
   Ser Gly Glu Asn Phe Pro Tyr Leu Val Ala Tyr Gln Ala Thr Val
   1580 1585 1590
   Cys Ala Arg Ala Gln Ala Pro Pro Pro Ser Trp Asp Gln Met Trp
   1595 1600 1605
   Lys Cys Leu Ile Arg Leu Lys Pro Thr Leu His Gly Pro Thr Pro
   1610 1615 1620
   Leu Leu Tyr Arg Leu Gly Ala Val Gln Asn Glu Val Thr Leu Thr
   1625 1630 1635
   His Pro Ile Thr Lys Tyr Ile Met Thr Cys Met Ser Ala Asp Leu
   1640 1645 1650
   Glu Val Val Thr Ser Thr Trp Val Leu Val Gly Gly Val Leu Ala
   1655 1660 1665
   Ala Leu Ala Ala Tyr Cys Leu Ser Thr Gly Cys Val Val Ile Val
   1670 1675 1680
   Gly Arg Ile Val Leu Ser Gly Lys Pro Ala Ile Ile Pro Asp Arg
   1685 1690 1695
   Glu Val Leu Tyr Gln Glu Phe Asp Glu Met Glu Glu Cys Ser Gln
   1700 1705 1710
   His Leu Pro Tyr Ile Glu Gln Gly Met Met Leu Ala Glu Gln Phe
   1715 1720 1725
   Lys Gln Lys Ala Leu Gly Leu Leu Gln Thr Ala Ser Arg Gln Ala
   1730 1735 1740
   Glu Val Ile Thr Pro Ala Val Gln Thr Asn Trp Gln Lys Leu Glu
   1745 1750 1755
   Val Phe Trp Ala Lys His Met Trp Asn Phe Ile Ser Gly Ile Gln
   1760 1765 1770
   Tyr Leu Ala Gly Leu Ser Thr Leu Pro Gly Asn Pro Ala Ile Ala
   1775 1780 1785
   Ser Leu Met Ala Phe Thr Ala Ala Val Thr Ser Pro Leu Thr Thr
   1790 1795 1800
   Gly Gln Thr Leu Leu Phe Asn Ile Leu Gly Gly Trp Val Ala Ala
   1805 1810 1815
   Gln Leu Ala Ala Pro Gly Ala Ala Thr Ala Phe Val Gly Ala Gly
   1820 1825 1830
   Leu Ala Gly Ala Ala Ile Gly Ser Val Gly Leu Gly Lys Val Leu
   1835 1840 1845
   Val Asp Ile Leu Ala Gly Tyr Gly Ala Gly Val Ala Gly Ala Leu
   1850 1855 1860
   Val Ala Phe Lys Ile Met Ser Gly Glu Val Pro Ser Thr Glu Asp
   1865 1870 1875
   Leu Val Asn Leu Leu Pro Ala Ile Leu Ser Pro Gly Ala Leu Val
   1880 1885 1890
   Val Gly Val Val Cys Ala Ala Ile Leu Arg Arg His Val Gly Pro
   1895 1900 1905
   Gly Glu Gly Ala Val Gln Trp Met Asn Arg Leu Ile Ala Phe Ala
   1910 1915 1920
   Ser Arg Gly Asn His Val Ser Pro Thr His Tyr Val Pro Glu Ser
   1925 1930 1935
   Asp Ala Ala Ala Arg Val Thr Ala Ile Leu Ser Ser Leu Thr Val
   1940 1945 1950
   Thr Gln Leu Leu Arg Arg Leu His Gln Trp Ile Ser Ser Glu Cys
   1955 1960 1965
   Thr Thr Pro Cys Ser Gly Ser Trp Leu Arg Asp Ile Trp Asp Trp
   1970 1975 1980
   Ile Cys Glu Val Leu Ser Asp Phe Lys Thr Trp Leu Lys Ala Lys
   1985 1990 1995
   Leu Met Pro Gln Leu Pro Gly Ile Pro Phe Val Ser Cys Gln Arg
   2000 2005 2010
   Gly Tyr Arg Gly Val Trp Arg Gly Asp Gly Ile Met His Thr Arg
   2015 2020 2025
   Cys His Cys Gly Ala Glu Ile Thr Gly His Val Lys Asn Gly Thr
   2030 2035 2040
   Met Arg Ile Val Gly Pro Arg Thr Cys Arg Asn Met Trp Ser Gly
   2045 2050 2055
   Thr Phe Pro Ile Asn Ala Tyr Thr Thr Gly Pro Cys Thr Pro Leu
   2060 2065 2070
   Pro Ala Pro Asn Tyr Lys Phe Ala Leu Trp Arg Val Ser Ala Glu
   2075 2080 2085
   Glu Tyr Val Glu Ile Arg Arg Val Gly Asp Phe His Tyr Val Ser
   2090 2095 2100
   Gly Met Thr Thr Asp Asn Leu Lys Cys Pro Cys Gln Ile Pro Ser
   2105 2110 2115
   Pro Glu Phe Phe Thr Glu Leu Asp Gly Val Arg Leu His Arg Phe
   2120 2125 2130
   Ala Pro Pro Cys Lys Pro Leu Leu Arg Glu Glu Val Ser Phe Arg
   2135 2140 2145
   Val Gly Leu His Glu Tyr Pro Val Gly Ser Gln Leu Pro Cys Glu
   2150 2155 2160
   Pro Glu Pro Asp Val Ala Val Leu Thr Ser Met Leu Thr Asp Pro
   2165 2170 2175
   Ser His Ile Thr Ala Glu Ala Ala Gly Arg Arg Leu Ala Arg Gly
   2180 2185 2190
   Ser Pro Pro Ser Met Ala Ser Ser Ser Ala Ser Gln Leu Ser Ala
   2195 2200 2205
   Pro Ser Leu Lys Ala Thr Cys Thr Ala Asn His Asp Ser Pro Asp
   2210 2215 2220
   Ala Glu Leu Ile Glu Ala Asn Leu Leu Trp Arg Gln Glu Met Gly
   2225 2230 2235
   Gly Asn Ile Thr Arg Val Glu Ser Glu Asn Lys Val Val Ile Leu
   2240 2245 2250
   Asp Ser Phe Asp Pro Leu Val Ala Glu Glu Asp Glu Arg Glu Val
   2255 2260 2265
   Ser Val Pro Ala Glu Ile Leu Arg Lys Ser Arg Arg Phe Ala Arg
   2270 2275 2280
   Ala Leu Pro Val Trp Ala Arg Pro Asp Tyr Asn Pro Pro Leu Val
   2285 2290 2295
   Glu Thr Trp Lys Lys Pro Asp Tyr Glu Pro Pro Val Val His Gly
   2300 2305 2310
   Cys Pro Leu Pro Pro Pro Arg Ser Pro Pro Val Pro Pro Pro Arg
   2315 2320 2325
   Lys Lys Arg Thr Val Val Leu Thr Glu Ser Thr Leu Ser Thr Ala
   2330 2335 2340
   Leu Ala Glu Leu Ala Thr Lys Ser Phe Gly Ser Ser Ser Thr Ser
   2345 2350 2355
   Gly Ile Thr Gly Asp Asn Thr Thr Thr Ser Ser Glu Pro Ala Pro
   2360 2365 2370
   Ser Gly Cys Pro Pro Asp Ser Asp Val Glu Ser Tyr Ser Ser Met
   2375 2380 2385
   Pro Pro Leu Glu Gly Glu Pro Gly Asp Pro Asp Leu Ser Asp Gly
   2390 2395 2400
   Ser Trp Ser Thr Val Ser Ser Gly Ala Asp Thr Glu Asp Val Val
   2405 2410 2415
   Cys Cys Ser Met Ser Tyr Ser Trp Thr Gly Ala Leu Val Thr Pro
   2420 2425 2430
   Cys Ala Ala Glu Glu Gln Lys Leu Pro Ile Asn Ala Leu Ser Asn
   2435 2440 2445
   Ser Leu Leu Arg His His Asn Leu Val Tyr Ser Thr Thr Ser Arg
   2450 2455 2460
   Ser Ala Cys Gln Arg Gln Lys Lys Val Thr Phe Asp Arg Leu Gln
   2465 2470 2475
   Val Leu Asp Ser His Tyr Gln Asp Val Leu Lys Glu Val Lys Ala
   2480 2485 2490
   Ala Ala Ser Lys Val Lys Ala Asn Leu Leu Ser Val Glu Glu Ala
   2495 2500 2505
   Cys Ser Leu Thr Pro Pro His Ser Ala Lys Ser Lys Phe Gly Tyr
   2510 2515 2520
   Gly Ala Lys Asp Val Arg Cys His Ala Arg Lys Ala Val Ala His
   2525 2530 2535
   Ile Asn Ser Val Trp Lys Asp Leu Leu Glu Asp Ser Val Thr Pro
   2540 2545 2550
   Ile Asp Thr Thr Ile Met Ala Lys Asn Glu Val Phe Cys Val Gln
   2555 2560 2565
   Pro Glu Lys Gly Gly Arg Lys Pro Ala Arg Leu Ile Val Phe Pro
   2570 2575 2580
   Asp Leu Gly Val Arg Val Cys Glu Lys Met Ala Leu Tyr Asp Val
   2585 2590 2595
   Val Ser Lys Leu Pro Leu Ala Val Met Gly Ser Ser Tyr Gly Phe
   2600 2605 2610
   Gln Tyr Ser Pro Gly Gln Arg Val Glu Phe Leu Val Gln Ala Trp
   2615 2620 2625
   Lys Ser Lys Lys Thr Pro Met Gly Phe Ser Tyr Asp Thr Arg Cys
   2630 2635 2640
   Phe Asp Ser Thr Val Thr Glu Ser Asp Ile Arg Thr Glu Glu Ala
   2645 2650 2655
   Ile Tyr Gln Cys Cys Asp Leu Asp Pro Gln Ala Arg Val Ala Ile
   2660 2665 2670
   Lys Ser Leu Thr Glu Arg Leu Tyr Val Gly Gly Pro Leu Thr Asn
   2675 2680 2685
   Ser Arg Gly Glu Asn Cys Gly Tyr Arg Arg Cys Arg Ala Ser Gly
   2690 2695 2700
   Val Leu Thr Thr Ser Cys Gly Asn Thr Leu Thr Cys Tyr Ile Lys
   2705 2710 2715
   Ala Arg Ala Ala Cys Arg Ala Ala Gly Leu Gln Asp Cys Thr Met
   2720 2725 2730
   Leu Val Cys Gly Asp Asp Leu Val Val Ile Cys Glu Ser Ala Gly
   2735 2740 2745
   Val Gln Glu Asp Ala Ala Ser Leu Arg Ala Phe Thr Glu Ala Met
   2750 2755 2760
   Thr Arg Tyr Ser Ala Pro Pro Gly Asp Pro Pro Gln Pro Glu Tyr
   2765 2770 2775
   Asp Leu Glu Leu Ile Thr Ser Cys Ser Ser Asn Val Ser Val Ala
   2780 2785 2790
   His Asp Gly Ala Gly Lys Arg Val Tyr Tyr Leu Thr Arg Asp Pro
   2795 2800 2805
   Thr Thr Pro Leu Ala Arg Ala Ala Trp Glu Thr Ala Arg His Thr
   2810 2815 2820
   Pro Val Asn Ser Trp Leu Gly Asn Ile Ile Met Phe Ala Pro Thr
   2825 2830 2835
   Leu Trp Ala Arg Met Ile Leu Met Thr His Phe Phe Ser Val Leu
   2840 2845 2850
   Ile Ala Arg Asp Gln Leu Glu Gln Ala Leu Asn Cys Glu Ile Tyr
   2855 2860 2865
   Gly Ala Cys Tyr Ser Ile Glu Pro Leu Asp Leu Pro Pro Ile Ile
   2870 2875 2880
   Gln Arg  Leu His Gly Leu Ser  Ala Phe Ser Leu His  Ser Tyr Ser
       2885                 2890                 2895
   Pro Gly  Glu Ile Asn Arg Val  Ala Ala Cys Leu Arg  Lys Leu Gly
       2900                 2905                 2910
   Val Pro  Pro Leu Arg Ala Trp  Arg His Arg Ala Arg  Ser Val Arg
       2915                 2920                 2925
   Ala Arg  Leu Leu Ser Arg Gly  Gly Arg Ala Ala Ile  Cys Gly Lys
       2930                 2935                 2940
   Tyr Leu  Phe Asn Trp Ala Val  Arg Thr Lys Leu Lys  Leu Thr Pro
       2945                 2950                 2955
   Ile Ala  Ala Ala Gly Arg Leu  Asp Leu Ser Gly Trp  Phe Thr Ala
       2960                 2965                 2970
   Gly Tyr  Ser Gly Gly Asp Ile  Tyr His Ser Val Ser  His Ala Arg
       2975                 2980                 2985
   Pro Arg  Trp Phe Trp Phe Cys  Leu Leu Leu Leu Ala  Ala Gly Val
       2990                 2995                 3000
   Gly Ile  Tyr Leu Leu Pro Asn  Arg
       3005                 3010

## Description

The invention relates to the generation and the use of hepacivirus pseudo-particles containing functional E1, E2 envelope glycoproteins assembled onto retroviral core particles. These particles are highly infectious and constitute a valid model of hepacivirus or GB virus virion.

World-wide several hundred millions of people are infected with hepatitis C virus (HCV) (Lavanchy et al., 1999). Progression to chronic disease occurs in the majority of HCV infected persons. Infection is associated with an increased risk for liver diseases and hepato-cellular carcinoma and has become the main indication for liver transplantation. HCV infection also increases the number of complications in HIV infected people (Dieterich, 2002). No vaccine is currently available to prevent new infections and the only treatment for chronic hepatitis C is interferon-α therapy, either alone or in combination with the guanosine analogue ribavirin. However, only ∼40% of patients respond to treatment. Clearly, novel therapeutic strategies are urgently required as the health costs for HCV infected people are predicted to spiral dramatically in the next few decades.

Based upon the structure of its genome and mechanisms of replication, HCV has been regarded as the prototype for a new class of viruses and was tentatively classified within the Hepacivirus genus, within the Flaviviridae virus family (Robertson et al., 1998).

Hepacivirus proteins structural and non-structural proteins are expressed from a single polyprotein precursor and individually released in their respective cell compartments upon cleavage by cellular and viral proteases (Lindenbach and Rice, 2001). By analogy with other members of the Flaviviridae, hepacivirus, and in particular HCV genomic organization, suggests a virus consisting of a nucleocapsid comprising a viral genome and core protein (C) coated by a lipid envelop containing the two envelope glycoproteins E1 and E2.

So far, the study of hepatitis C virus (HCV) has been hammered by the lack of an efficient and reliable culture system for amplifying the virus and by the lack of suitable animal model to study HCV replication in vivo (Lindenbach and Rice, 2001). Recently, a model for HCV replication, based on the self-replication of engineered minigenomes in cell culture, has been established (Blight *et al*., 2000; Lohmann *et al*., 1999). Although very useful to study HCV genomic replication, this system does not support production of HCV particles (Pietschmann *et al*., 2002).

Production of HCV virus-like particles (VLP) in insect cells has already been reported (WO 98/21338, Wellnitz *et al*., 2002; Baumert *et al*.; 1998, Owsianka *et al*., 2001). However these particles were not secreted and their extraction from intracellular compartments yielded VLP preparations that were not infectious.

Pseudotyped Vesicular Stomatitis Virus (VSV) viral particles have also been engineered with chimeric E1 and/or E2 glycoproteins whose transmembrane domains were modified to allow their transport to the cell surface (Buonocore *et al*., 2002; Matsuura et *al*., 2001). However, such modifications are likely to disturb conformation and functions of the E1E2 complexes (Matsuura *et al*., 2001) and although such pseudo-particles stand among candidate HCV vaccines (Rose *et al*., 2001), their use as a tool to investigate HCV assembly and cell entry remains controversial since there is now a consensus that these previous results are artefactual (Buonocore et *al*., 2002).

Similarly, the international patent application WO 02/074941 discloses that lentiviral-based pseudo-particles displaying modified E1 and E2 HCV glycoproteins, which harbour alteration of their transmembrane domain, can infect 293 human kidney cells and HepG2 hepato-carcinoma cells. This patent application further shows that pseudo-particles derived from human foamy virus displaying unmodified E1 E2 glycoproteins can also infect 293 and HepG2 cells. However, these finding are artefactual because, as stated above, transmembrane modifications of E1E2 loaded on HCV pseudo-particles (Hsu et al., 2003) abolishes their functional properties, and because wild-type HCV is not infectious for 293 as well as HepG2 cells (Bartosch et al., 2003a and b).

In WO02/070651 it is suggested to pseudotype lentiviral particles with HCV E1 and E2, however, this patent application does not disclose how to incorporate said glycoproteins in the viral particle.

Chimeric HCV-BVDV (Bovine Viral Diarrhea Virus) particles were shown to be infectious for the human hepatocyte line Huh-7 (WO 00/75352). However, infectivity could not be neutralised by an anti-serum against HCV which indicated that these particles did not constitute a valid model of HCV virion.

New approaches are therefore sorely needed to study HCV assembly and cell entry in order to design HCV cell entry inhibitors and to study the humoral immune response against HCV. Availability of infectious, amplifiable HCV particles would also provide useful material for the development of diagnostic application as well as therapeutical drugs.

The inventors have successfully generated infectious pseudo-particles that were assembled by displaying functional, in particular unmodified, HCV glycoproteins onto retroviral and lentiviral core particles. The presence of a green fluorescent protein marker gene packaged within these HCV pseudo-particles allowed reliable and fast determination of infectivity mediated by the HCV glycoproteins. Primary hepatocytes as well as hepato-carcinoma cells were found to be the major targets of infection in vitro. Albeit low residual infectivity may be observed with pseudo-particles harbouring either E1 or E2 glycoprotein, high infectivity of the pseudo-particles required both E1 and E2 HCV glycoproteins. Infectivity was further found to be neutralized by sera from HCV-infected patients and by some anti-E2 monoclonal antibodies. Altogether, these results indicate that the pseudo-particles described herein are the first pseudo-particle reported so far to mimic the early infection steps of wild-type HCV (Bartosch et al., 2003a; Castet, 2003).

The description thus proposes infectious hepacivirus pseudo-particles, and in particular HCV pseudo-particles harboring E1 and E2 glycoproteins, that constitute a valid model of hepacivirus or GB virus virions.

The object of the present invention is as defined in claims 1 to 45.

### Definitions

The terms "*vector*", *"cloning vector*" and *"expression vector*" mean the vehicle by which a DNA or RNA sequence (e.g. a foreign gene) can be introduced into a host cell, so as to transform the host and promote expression (e.g. transcription and translation) of the introduced sequence. Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a *"DNA construct*". A common type of vector is a "*plasmid*", which generally is a self-contained molecule of double-stranded DNA, usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence that initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts.

A *"coding sequence"* or a sequence *"encoding"* an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme.

The term *"transfection"* means the introduction of a foreign nucleic acid (DNA, cDNA or RNA) into a cell so that the host cell will express the introduced gene or sequence to produce a desired substance, typically a protein coded by the introduced gene or sequence. The introduced gene may include regulatory or control sequences, such as start, stop, promoter, signal, secretion, or other sequences used by a cell's genetic machinery. A host cell that receives and expresses introduced DNA or RNA has been "*transformed*".

The term "*host cell*" means any cell of any organism that is selected, modified, transformed, grown, or used or manipulated in any way, for the production of a substance by the cell, for example the expression by the cell of a gene, a DNA sequence, a protein, a virion. In the context of the invention, the host cell is a mammalian cell. Suitable host cells include Huh-7 human hepatocellular carcinoma (Nakabayashi et *al*., 1982), Hep3B human hepatocellular carcinoma (ATCC HB-8064), HepG2 human hepatocellular carcinoma (HB-8065), HT-1080 human fibrosarcoma (CCL-*12*1), 293T human embryo kidney cells (ATCC CRL-1573), TE671 human rhabdomyosarcoma (ATCC CRL-8805), Jurkat human T cell leukemia (TIB-152), CEM human lymphoblastic leukemia (CCL-119), COS-7 African green monkey fibroblasts kidney (CRL-1651), VERO African green monkey kidney (CCL-81), PG-4 feline astrocyte (CRL-2032), BHK-21 golden hamster kidney (CCL-10), CHO Chinese hamster ovary (ATCC CCL-61), and NIH3T3 mouse fibroblasts. In a specific embodiment said host cell is 293T.

As used herein, the term "*permissive cell*" is meant for a cell that is permissive for a hepacivirus or GB virus infection.

"*Hepacivirus or GB virus*" denotes hepatitis C virus, GB viruses denote GB virus A, GB virus B, GB virus C, GBV-A like agents, and hepatitis G virus. Preferably, said hepacivirus is hepatitis C virus (HCV). In the context of the invention, said hepacivirus or GB virus may be of any species, genotype and subtype, where appropriate, and variants thereof.

"*Hepatitis C Virus*" or "*HCV*" is a member of the *Flaviviridae* family. HCV is the type species of the genus hepacivirus. HCV genome, like other hepaciviruses genome, encodes a single polyprotein NH₂C-E1-E2-P7-NS2-NS3-NS4a-NS4b-NS5a-NS5b-COOH that is processed co and post-translationally into both structural (N-terminal nucleocapsid protein termed "Core" (C), and glycoproteins E1 and E2) and non-structural (NS) proteins. The amino-terminal part of the polyprotein is cleaved by host cell proteases and its products, core and envelope (E1 and E2) proteins, are believed to be the major constituents of HCV particles (virions).

Although most cleavages in the polyprotein precursor proceed to completion during or immediately after translation, processing between E2 and p7, a hydrophobic domain found at the carboxy terminus of E2, is incomplete and results in the production of fully processed E2 and uncleaved E2-p7. The p7 polypeptide of HCV is a small hydrophobic protein which has not been well characterized yet. Indeed, the structure of this 63-amino-acid-long polypeptide has not been determined, and its putative function(s) remains unknown.

In the context of the invention, HCV is of any genotype, *e.g*. 1, 2, 3, 4, 5, 6, and subtype, *e.g*. a, b, c, d, e, f, g, h, k, and variants thereof.

The polyprotein sequence of HCV strain H (GenBank accession number AF009606) is shown in SEQ ID N°16. Amino acid positions of HCV E1, E2, p7 and core proteins are indicated below by reference to this sequence SEQ ID N°16.

The term "*variant*" refers to the homologous polynucleotide sequences and corresponding amino acid sequences found in the different HCV strains owing to HCV hypervariability.

Preferably, HCV has either 1a or 1b genotype (Dubuisson *et al*., 1994) that stand among HCV genotypes that are the most prevalent and the most resistant to interferon-α therapy (Zein, 2000). Also preferably HCV has either genotype 2a, 2b, 3a, 3b, 4a or 4b.

The term "*hepacivirus-like particles*", "*hepacivirus-like pseudo-particles*", or "*hepacivirus pseudo-particles*"*,* or "*hepacivirus pseudotype particles*" as used herein, refer to non naturally occurring viral particles that comprise an envelope protein of an hepacivirus or GB virus. Preferably, the hepacivirus-like particles according to the invention display the same cellular tropism as the wild-type hepacivirus or GB virus virion.

The term "*HCV-like particles*" or *"HCV pseudo-particles"* or *"HCV pseudotype particles*" as used herein refers to non naturally occurring viral particles that comprise an envelope protein of HCV.

The particles of the invention more particularly comprise retroviral core proteins. Such particles may be readily produced by one skilled in genetic engineering techniques. One can for instance refer to EP 1 201 750 that describes production of synthetic retroviral particles expressing an antigen for modulating an immune response.

The hepacivirus, in particular HCV, pseudo-particles of the invention are infectious for a target cell. Preferably the HCV pseudo-particles display the same cellular tropism as wild-type HCV virion (also called "tropism of wild-type HCV virion"), i.e. a preferential tropism for hepatic cells. Preferably, an hepatic target cell may be selected from the group consisting of a primary human hepatocyte, and a cell from an hepatocarcinoma cell line HuH7, PLC/PRF/5, or Hep3B, and an HepG2 cell genetically engineered to express CD81 ("HepG2-CD81"). As stated above, wild-type HCV is not infectious for the HepG2 cell line, and as described herein, the HepG2 cell line shows a very weak level of infection with the HCV pseudo-particles of the invention. However, the inventors demonstrated that expression of CD81 is sufficient to restore HCV pseudo-particles entry in HepG2 cells (Bartosch et al., 2003b). HepG2-CD81 thus represents a further example of hepatic target cell for HCV virions and HCV pseudo-particles.

In the context of the invention, the term *"infectious"* is used to describe the capacity of the particles of the invention to complete the initial steps of viral cycle that lead to cell entry. However, upon interaction with the host cell, hepacivirus-like particles may or may not produce progeny viruses.

The term "*an envelope protein of a hepacivirus or GB virus*" denotes the native E1 or E2 glycoprotein of a hepacivirus or GB virus, or a mutant thereof.

By an "*E1 glycoprotein"* or "*E1 protein"* is meant an envelope 1 protein (E1) from any specie, genotype, subtype and variants of hepacivirus or GB virus strains.

By an *"E2 glycoprotein"* or *"E2 protein"* is meant an envelope 2 protein (E2) from any specie, genotype, subtype and variants of hepacivirus or GB virus strains.

Preferably, E1 and E2 glycoproteins are derived from a same hepacivirus or GB virus strain. Preferably, E1 and/or E2 glycoproteins are native.

By a "*p7 protein"* is meant a native p7 protein, or a mutant thereof, from any specie, genotype, subtype and variants of hepacivirus or GB virus strains. Preferably, p7 protein and E1 and/or E2 glycoproteins are derived from a same hepacivirus or GB virus strain. Preferably, p7 protein and E1 and/or E2 glycoproteins are native.

The term *"an envelope protein of HCV*" denotes the E1 or E2 glycoprotein of HCV, or a mutant thereof.

By a *"HCV E1 glycoprotein"* or *"HCV E1 protein"* is meant an envelope 1 protein (E1) from any genotype, subtype and variant of HCV strains. Full length E1 protein is defined by amino acids 192 to 383 of SEQ ID N°16.

By a *"HCV E2 glycoprotein"* or *"HCV E2 protein"* is meant an envelope 2 protein (E2) from any genotype subtype and variant of HCV strains. Full length E2 protein is defined by amino acids 384 to 746 of SEQ ID N°16.

Preferably, HCV E1 and E2 glycoproteins are derived from a same HCV strain. Preferably, HCV E1 and/or E2 glycoproteins are native.

By a *"HCV p7 protein"* is meant a native p7 protein (amino acids 747 to 809 as shown in SEQ ID N°16), or a mutant thereof, from any genotype, subtype and variant of HCV strains. Preferably, HCV p7 protein and E1 and/or E2 glycoproteins are derived from a same HCV strain. Preferably, HCV p7 protein and E1 and/or E2 glycoproteins are native.

The term "*mutant*" or "*mutation*" is meant for alteration of the DNA sequence that result in a modification of the amino acid sequence of native E1, E2, or p7 proteins. Such a modification can be for instance the substitution and/or deletion of one or more amino acids. Mutants notably include fragments of native E1, E2 and p7 proteins. Variants are particular examples of naturally occurring mutants. Mutants are more particularly contemplated as useful for identifying the structural elements of E1 and/or E2 proteins, and optionally p7 protein, necessary for maintaining cell infectivity or for increasing E1 and/or E2 antigenicity for vaccination purposes.

Preferably, i) a mutant E1, E2, or p7 protein retains the capacity to assemble on the retroviral-based pseudo-particles and makes it possible for the pseudo-particles so produced to be released by the host cell; and/or ii) the pseudo-particles harbouring a mutant E1, E2, or p7 protein retains the preferential tropism of HCV virion for hepatic cells.

In this regards, substantial modifications of the C-terminal transmembrane domain of E1 or E2 glycoprotein, which allows anchoring of the glycoprotein to the membrane and thus assembly on the viral particle, should preferably be avoided as they may result in an alteration of cellular tropism and/or prevent pseudo-particle assembly and release. More specifically, mutated E1 or E2 protein wherein the transmembrane domain and/or cytoplasmic tail has been substituted, at least partially, preferably substantially totally, with the transmembrane domain/cytoplasmic tail of a heterologous protein, such as the VSV-G protein, is excluded from the scope of the invention, in particular when the pseudo-particles so produced no longer display the tropism of wild-type HCV virion.

Advantageously, hepacivirus pseudo-particles with mutated E1, E2, or p7 protein are those that do not show decreased infectiosity compared with the corresponding pseudo-particle harbouring native E1, E2, or p7 protein. In this respect, preferred hepacivirus pseudo-particles do not contain mutations affecting a domain of E1 or E2 which is implicated in hepacivirus or GB virus attachment to a cell receptor. In particular, said pseudo-particle may not comprise a HCV E2 glycoprotein mutated in one of the binding domains to CD81 or Scavenger Receptor class B type 1 (SR-B1), which are putative HCV cell receptors (Pileri et al., 1998; Scarselli et al., 2002). E2 binding domain to CD81 has been defined as three discrete segments at both ends of E2 (amino-acids 474-494, 522-551 and 612-620 of SEQ ID N°16) that may join together in the head-to-tail model of E2 glycoprotein homodimer (Yagnik et al., 2000) whereas E2 bonding to SR-B1 is mediated by the hypervariable region 1 (HVR1, amino acids 384 to 410 of SEQ ID N°16) (Bartosch et al., 2003b; Scarselli et al., 2002).

Preferred mutations of E1, E2 or p7 protein are those that result in increased infectivity of the hepacivirus pseudo-particles harbouring them. In particular, said E2 mutant may be a E2 glycoprotein in which the last C-terminal residue is deleted (Ala 746 as shown in SEQ ID N°16). A 10 to 50 fold enhancement of infectiosity was observed with the HCV pseudo-particles harbouring this C-terminally truncated E2 protein (amino acids 384 to 745 of SEQ ID N°16) compared with HCV pseudo-particles harbouring the native, full length E2 glycoprotein.

However, a hepacivirus pseudo-particle according to the invention may contain mutated E1, E2, or p7 protein and show decreased infectiosity compared with the corresponding pseudo-particle harbouring native E1, E2, or p7 protein. Such pseudo-particles are nonetheless useful for vaccination purposes and represent an interesting tool for the comprehension of mechanisms for virus entry in cells. Accordingly, the mutants may encompass a E2 glycoprotein wherein hypervariable region 1 (HVR1) has been deleted, while the particles so produced remain infectious, albeit with a decreased infectiosity.

The term *"hepacivirus or GB virus core"* is meant for a native, full length, core protein of a hepacivirus or GB virus strains, a fragment thereof, or a variant thereof. According to an embodiment, the core protein is a N-terminally truncated form of hepacivirus or GB virus core (ΔC) that comprises the core signal peptide. Upon completion of its addressing function, the core protein is processed by a cellular protease and thereby cleaved from the polyprotein E1E2. Accordingly, the hepacivirus or GB virus core protein is not found in the pseudo-particles according to the invention. The one skilled in the art will readily understand that the hepacivirus or GB virus core protein can be replaced with a peptide sequence containing the signal sequence of any heterologous type I membrane protein (i.e. a protein anchored to the membrane by its C-terminus).

*"Signal peptide"* is intended for a peptide present on proteins that are destinated either to be secreted or to be membrane components. The signal peptide is usually located at the N-terminus and normally absent from the mature protein. It normally refers to the sequence (about 20 amino acids long) that interacts with signal recognition particle and directs the ribosome to the endoplasmic reticulum where co-translational insertion takes place. The signal sequence is normally removed from the growing peptide chain by signal peptidase, a specific protease located on the cisternal face of the endoplasmic reticulum.

In the context of the invention, *"heterologous"* is intended for a protein that is non-naturally occurring in the hepacivirus or GB virus virion, and in particular in the HCV virion.

Examples of signal sequence from type I membrane protein are well known by the one skilled in the art. Reference may be made for instance to the signal sequence of immunoglobulins, or to signal sequences of viral type I glycoproteins, in particular retroviral surface glycoproteins. Examples of type I glycoproteins are further described in Paetzel et al. (2002) and von Heijne (1990).

As used herein, the term *"HCV core"* denotes a native core protein of the various HCV strains, a fragment thereof, or a variant thereof. The HCV core provides a signal peptide for the E1 or optionally E2 linked thereto that allows protein translocation to the endoplasmic reticulum. HCV core signal peptide corresponds to the last 21 residues of the carboxy-terminus of HCV core (GCSFSIFLLALLSCLTVPASA, SEQ ID N°1; which corresponds to amino acids 171 to 191 of SEQ ID N°16). According to an embodiment, the core protein is thus a N-terminally truncated form of HCV core (ΔC). Preferably ΔC comprises the last 21 residues of the carboxy-terminus of HCV core. In particular, HCV core may consist in the last 60 residues of the carboxy-terminus of HCV core (amino acids 132 to 191 of SEQ ID N°16).

In the context of the invention, the terms *"native"* or "*unmodified*" are indifferently used to describe a wild-type, full-length protein.

The term "*polyprotein*" as used herein is used to describe a protein construct made up of individual proteins that are joined together in a sequence whereby they retain their original relevant biological activities.

The term *"a polyprotein comprising a hepacivirus or GB virus core protein linked to hepacivirus or GB virus E1 protein and*/*or hepacivirus or GB virus E2 protein",* or *"a polyprotein comprising successively a hepacivirus or GB virus core protein, and a hepacivirus or GB virus E1protein and*/*or hepacivirus or GB virus E2 protein"* includes the CE1E2, CE2E1, CE1, CE2, ΔCE1E2, ΔCE2E1, ΔCE1, and ΔCE2 polyproteins. Optionally, said polyproteins further contain the p7 protein. The polyprotein comprising a hepacivirus or GB virus core protein linked to hepacivirus or GB virus E1 protein and/or hepacivirus or GB virus E2 protein thus additionally includes the CE1 E2p7, CE2p7E1, CE1p7, CE2p7, ΔCE1 E2p7, ΔCE2p7E1, ΔCE1p7, and ΔCE2p7 polyproteins.

"*CE1E2*" denotes a polyprotein comprising successively a hepacivirus or GB virus core protein, a hepacivirus or GB virus E1 protein and a hepacivirus or GB virus E2 protein. "*CE2E1*" denotes a polyprotein comprising successively a hepacivirus or GB virus core protein, a hepacivirus or GB virus E2 protein and a hepacivirus or GB virus E1 protein. "*CE1*" denotes a polyprotein comprising a hepacivirus or GB virus core protein linked to a hepacivirus or GB virus E1 protein. "*CE2*" denotes a polyprotein comprising a hepacivirus or GB virus core protein linked to a hepacivirus or GB virus E2 protein. "Δ*CE1E2*" denotes a polyprotein comprising a carboxy terminus of hepacivirus or GB virus core protein, and hepacivirus or GB virus E1 and hepacivirus or GB virus E2 proteins. "Δ*CE2E1*" denotes a polyprotein comprising a carboxy terminus of hepacivirus or GB virus core protein, and hepacivirus or GB virus E2 and hepacivirus or GB virus E1 proteins. "*ΔCE1*" denotes a polyprotein comprising a carboxy terminus of hepacivirus or GB virus core protein, linked to hepacivirus or GB virus E1 protein. "Δ*CE2*" denotes a polyprotein comprising a carboxy terminus of hepacivirus or GB virus core protein, linked to hepacivirus or GB virus E2 protein. Δ*CE1E2*, as well as Δ*CE2*, have been built by inserting a stop codon at the end of E2, whereas Δ*CE2E1* and Δ*CE1* have been built by inserting a stop codon at the end of E1. "*CE1E2p7*" denotes a polyprotein comprising successively a hepacivirus or GB virus core protein, a hepacivirus or GB virus E1 protein, a hepacivirus or GB virus E2 protein, and a hepacivirus or GB virus p7 protein. "*CE2p7E1*" denotes a polyprotein comprising successively a hepacivirus or GB virus core protein, a hepacivirus or GB virus E2 protein, a hepacivirus or GB virus p7 protein, and a hepacivirus or GB virus E2 protein. "*CE1p7*" denotes a polyprotein comprising successively a hepacivirus or GB virus core protein, a hepacivirus or GB virus E1 protein, and a hepacivirus or GB virus p7 protein. "*CE2p7*" denotes a polyprotein comprising successively a hepacivirus or GB virus core protein, a hepacivirus or GB virus E2 protein, and a hepacivirus or GB virus p7 protein. "Δ*CE1E2p7*" denotes a polyprotein comprising a carboxy terminus of hepacivirus or GB virus core protein, a hepacivirus or GB virus E1 protein, a hepacivirus or GB virus E2 protein, and a hepacivirus or GB virus p7 protein. "Δ*CE2p7E1*" denotes a polyprotein comprising a carboxy terminus of hepacivirus or GB virus core protein, a hepacivirus or GB virus E2 protein, a hepacivirus or GB virus p7 protein and a hepacivirus or GB virus E1 protein. "Δ*CE1p7*" denotes a polyprotein comprising a carboxy terminus of hepacivirus or GB virus core protein, a hepacivirus or GB virus E1 protein, and a p7 protein. "Δ*CE2p7*" denotes a polyprotein comprising a carboxy terminus of hepacivirus or GB virus core protein, a hepacivirus or GB virus E2 protein, and a p7 protein. Δ*CE1E2p7*, Δ*CE1p7* and Δ*CE2p7*, have been built by inserting a stop codon at the end of p7 whereas ΔCE2p7E1 has been built by inserting a stop codon at the end of E1.

The term "*a polyprotein comprising a HCV core protein linked to HCV E1 protein and*/*or HCV E2 protein*", or "a *polyprotein comprising successively a HCV core protein and a HCV E1 protein and*/*or a HCV E2 protein",* includes the HCV CE1E2, CE2E1, CE1, CE2, ΔCE1E2, ΔCE2E1, ΔCE1, and ΔCE2 polyproteins. Optionally, said polyproteins further contain the p7 protein. The polyprotein comprising a HCV core protein linked to HCV E1 protein and/or HCV E2 protein thus additionally includes the HCV CE1 E2p7, CE2p7E1, CE1p7, CE2p7, ΔCE1E2p7, ΔCE2p7E1, ΔCE1p7, and ΔCE2p7 polyproteins. "*HCV CE1E2*" denotes a polyprotein comprising successively a HCV core protein, a HCV E1 protein and a HCV E2 protein. *"HCV CE2E1*" denotes a polyprotein comprising successively a HCV core protein, a HCV E2 protein and a HCV E1 protein. *"HCV CE1*" denotes a polyprotein comprising a HCV core protein linked to a HCV E1 protein. *"HCV CE2*" denotes a polyprotein comprising a HCV core protein linked to a HCV E2 protein. "HCV Δ*CE1E2*" denotes a polyprotein comprising a carboxy terminus of HCV core protein, and HCV E1 and HCV E2 proteins. "*HCV* Δ*CE2E1*" denotes a polyprotein comprising a carboxy terminus of HCV core protein, and HCV E2 and HCV E1 proteins. *"HCV* Δ*CE1*" denotes a poiyprotein comprising a carboxy terminus of HCV core protein, and a HCV E1 protein. *"HCV* Δ*CE2*" denotes a polyprotein comprising a carboxy terminus of HCV core protein, and a HCV E2 protein. *HCV* Δ*CE1E2*, as well as *HCV* Δ*CE2*, have been built by inserting a stop codon at the end of E2, whereas Δ*CE2E1* and Δ*CE1* have been built by inserting a stop codon at the end of E1. "*HCV CE1E2p7*" denotes a polyprotein comprising successively a HCV core protein, a HCV E1 protein, a HCV E2 protein, and a HCV p7 protein. *"HCV CE2p7E1*" denotes a polyprotein comprising successively a HCV core protein, a HCV E2 protein, a HCV p7 protein, and a HCV E2 protein. *"HCV CE1p7*" denotes a polyprotein comprising successively a HCV core protein, a HCV E1 protein, and a HCV p7 protein. "*HCV CE2p7*" denotes a polyprotein comprising successively a HCV core protein, a HCV E2 protein, and a HCV p7 protein. *"HCV* Δ*CE1E2p7*" denotes a polyprotein comprising a carboxy terminus of HCV core protein, a HCV E1 protein, a HCV E2 protein, and a HCV p7 protein. "*HCV* Δ*CE2p7E1*" denotes a polyprotein comprising a carboxy terminus of HCV core protein, a HCV E2 protein, a HCV p7 protein, and a HCV E1 protein. *"HCV* Δ*CE1p7*" denotes a polyprotein comprising a carboxy terminus of HCV core protein, a HCV E1 protein, and a p7 protein. *"HCV* Δ*CE2p7*" denotes a polyprotein comprising a carboxy terminus of HCV core protein, a HCV E2 protein, and a p7 protein. HCV ΔCE1E2p7, HCV ΔCE1p7, and HCV ΔCE2p7, have been built by inserting a stop codon at the end of p7 whereas ΔCE2p7E1 has been built by inserting a stop codon at the end of E1.

By "*retrovirus*" is meant a virus whose genome consists of a RNA molecule and that comprises a reverse-transcriptase, *i.e*. a member of the Retroviridae family. Retroviruses are divided into Oncovirus, Lentivirus and Spumavirus. Preferably said retrovirus is an oncovirus, *e.g*. MLV, ALV, RSV, or MPMV, a lentivirus, *e.g*. HIV-1, HIV-2, SIV, EIAV, or CAEV, or a spumavirus such as HFV. Genomes of these retroviruses are readily available in databanks.

In the context of the invention "*a nucleic sequence comprising a packaging competent retrovirus-derived genome*" is intended for a sequence that comprises the retroviral nucleic acid sequences known as "cis-acting" sequences. These include the Long Terminal Repeats (LTRs) for the control of transcription and integration, the psi sequence necessary for encapsidation, and the Primer Binding site (PBS) and polypurine track (PPT) sequences necessary for reverse transcription of the retroviral genome. Advantageously, said nucleic acid sequence comprising a packaging competent retrovirus-derived genome further comprises a transgene.

Said retroviral genome may be replication-defective or replication-competent, in the absence of any trans-complementing function. A replication-competent genome would further comprise the gag, pol, and env retroviral genes. In a replication-defective genome, the viral genes gag, pol, and env are deleted. However, assembly of viral pseudo-particles may be achieved by providing in trans another vector that comprises gag, pol and env but that is defective for the "cis" sequences. Their expression allows the encapsidation of the transgene, excluding the genes necessary for the multiplication of the viral genome and for the formation of complete viral particles.

As used herein, the term "*transgene*" designates the gene that is expressed in the target cell upon infection by the particles of the invention.

Examples of transgenes include a gene encoding a molecule of therapeutic interest, a marker gene, a gene coding for an immune modulator, an antigen, or a suicide gene.

A *"marker gene"* denotes a gene whose expression is detectable. For instance marker gene expression can generate a detectable signal, such as a fluorescence emission, a chromogenic reaction, or confer a growth advantage to the cells wherein it is expressed (antibiotic resistance genes).

An *"immune modulator*" refers to the product of a gene that modifies the activity of the immune system of a subject *in vivo.* Examples of immune modulators include cytokines, (*e.g*. interleukins, interferons, or haematopoietic colony stimulating factors), chemokines, and the like. Expression of an immune modulator by transformed cells may change the cellular environment and alter differentiation of immune cells and thus modify the type and the strength of immune response elicited against a given antigen.

An *"antigen"* refers to a molecule, such as a peptide, a polypeptide or a protein, against which an immune response is sought. Said antigen may be for instance a tumor, a bacterial, a pathogenic, a proteic, or a viral antigen.

A *"suicide gene"* is meant for a gene whose expression in cells induces programmed-cell death (apoptosis) such as the conditional Herpes Simplex virus type I thymidine kinase gene.

The "*core protein from a retrovirus*" refers to proteins encoded by the gag and pol genes. The gag gene encodes a polyprotein which is further processed by the retroviral protease into structural proteins that comprise the core. The pol gene encodes the retroviral protease, reverse-transcriptase, and integrase.

A "*pharmaceutically acceptable carrier*" refers to any vehicle wherein the vaccine composition according to the invention may be formulated. It includes a saline solution such as phosphate buffer saline. In general, a diluent or carrier is selected on the basis of the mode and route of administration, and standard pharmaceutical practice.

In the context of the present application, *"vaccination"* is intended for prophylactic or therapeutical vaccination. "*Therapeutical vaccination"* is meant for vaccination of a patient with HCV infection.

According to the invention, the term "*subject*" or "*patient*" is meant for any mammal likely to be infected with a hepacivirus or a GB virus, in particular with HCV. Human, chimpanzee, tamarin, and mice, especially human liver-xenogratfed mice are examples of hosts for hepaciviruses or GB viruses, and in particular HCV.

### Production of hepacivirus pseudo- particles

The inventors have generated infectious pseudo-particles that contain functional, and more particularly unmodified, hepacivirus or GB virus glycoproteins, in particular HCV glycoproteins, assembled onto retroviral core particles. Hepacivirus or GB virus (HCV) E1E2, and optionally p7, are expressed from a polyprotein containing the core (C) protein or a fragment thereof, in particular the carboxy-terminus of the C protein, which served as signal peptide for E1 or E2, and the E1 and/or E2 glycoproteins. More generally, hepacivirus or GB virus E1 E2 may be expressed from a polyprotein containing a signal peptide from a heterologous type I membrane protein.

The invention thus provides a method for producing hepacivirus-like particles *ex vivo* comprising the steps of:
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a signal peptide from a type I membrane protein, preferably a hepacivirus or GB virus core protein, and a hepacivirus or GB virus E1 protein and/or a hepacivirus or GB virus E2 protein;
- transfecting host cells with said nucleic acid sequences and maintaining the transfected cells in culture for sufficient time to allow expression of the cDNAs to produce structural proteins from hepacivirus or GB virus and retrovirus; and allowing the structural proteins to form virus-like particles.

The description further provides a method for producing hepacivirus-like particles *in vivo,* which method comprises the steps of :
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a signal peptide from a type I membrane protein, preferably a hepacivirus or GB virus core protein, and a hepacivirus or GB virus E1 protein and/or a hepacivirus or GB virus E2 protein;
- transfecting cells of a subject *in vivo* with said nucleic acid sequences, to allow expression of the cDNAs to produce structural proteins from hepacivirus or GB virus and retrovirus; and to allow the structural proteins to form virus-like particles.

Another aspect of the invention is the use of three nucleic acid sequences for the preparation of a medicament useful as a vaccine against a hepacivirus or GB virus infection, *i.e*. hepatitis, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a signal peptide from a type I membrane protein, preferably a hepacivirus or GB virus core protein, and a hepacivirus or GB virus E1 protein and/or a hepacivirus or GB virus E2 protein ;
and, when transferred into cells of a subject, the nucleic acid sequences allow the production of structural proteins from hepacivirus or GB virus and retrovirus, wherein the structural proteins form virus-like particles that are immunogenic.

Preferably, said third nucleic acid sequence comprises a cDNA encoding a polyprotein that further comprises a hepacivirus or GB virus p7 protein. Thus, preferably said polyprotein comprises successively a signal peptide from a type I membrane protein, preferably a hepacivirus or GB virus core protein, a hepacivirus or GB virus E1 protein and/or a hepacivirus or GB virus E2 protein, and optionally a hepacivirus or GB virus p7 protein.

According to a specific embodiment, said packaging competent retroviral genome and core proteins are derived from a retrovirus selected from the group consisting of MLV, ALV, RSV, MPMV, HIV-1, HIV-2, SIV, EIAV, CAEV, and HFV.

Advantageously, the packaging competent retroviral genome further comprises a marker gene or an immune modulator.

In the method described above, the polyprotein may comprise a hepacivirus or GB virus core protein linked to a hepacivirus or GB virus E1 protein, or a hepacivirus or GB virus core protein linked to a hepacivirus or GB virus E2 protein, or successively a hepacivirus or GB virus core protein, a hepacivirus or GB virus E1 protein and a hepacivirus or GB virus E2 protein, or successively a hepacivirus or GB virus core protein, a hepacivirus or GB virus E2 protein and a hepacivirus or GB virus E1 protein. Said polyprotein may further comprise successively a hepacivirus or GB virus core protein, a hepacivirus or GB virus E1 protein and a p7 protein, or successively a hepacivirus or GB virus core protein, a hepacivirus or GB virus E2 protein and a hepacivirus or GB virus p7 protein, or successively hepacivirus or GB virus core protein, a hepacivirus or GB virus E1 protein, a hepacivirus or GB virus E2 protein and a hepacivirus or GB virus p7 protein, or successively a hepacivirus or GB virus core protein, a hepacivirus or GB virus E2 protein, a hepacivirus or GB virus p7 protein, and a hepacivirus or GB virus E1 protein.

According to an embodiment, E1 and/or E2, and optionally p7 protein, are native proteins. According to another embodiment, E1 and/or E2 glycoproteins, and optionally p7 protein, are mutated to obtain particles that may be useful for characterizing the glycoprotein determinants for hepacivirus or GB virus infectivity.

Preferably, said E1 and E2 glycoproteins are both derived from a same hepacivirus or GB virus strain. Preferably, said E1 or E2 glycoprotein and p7 protein are both derived from a same hepacivirus or GB virus strain. Still preferably, said E1 and E2 glycoproteins, and p7 protein are derived from a same hepacivirus or GB virus strain.

According to another embodiment said hepacivirus or GB virus core protein is a carboxy terminus form (ΔC) of hepacivirus or GB virus core protein, comprising the core protein signal peptide.

Preferably said hepacivirus or GB virus is a hepatitis C virus (HCV).

The invention thus provides a method for producing hepatitis C virus (HCV)-like particles *ex vivo* comprising the steps of:
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a signal peptide from a type I membrane protein, preferably a HCV core protein, and a HCV E1 protein and/or a HCV E2 protein;
- transfecting host cells with said nucleic acid sequences and maintaining the transfected cells in culture for sufficient time to allow expression of the cDNAs to produce structural proteins from hepatitis C virus and retrovirus; and allowing the structural proteins to form virus-like particles.

A method for producing hepatitis C-virus (HCV)-like particles *in vivo,* is further described herein, which method comprises the steps of :
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a signal peptide from a type I membrane protein, preferably a HCV core protein, and a HCV E1 protein and/or a HCV E2 protein;
- transfecting cells of a subject *in vivo* with said nucleic acid sequences, to allow expression of the cDNAs to produce structural proteins from hepatitis C virus and retrovirus; and to allow the structural proteins to form virus-like particles.

Another aspect of the invention is the use of three nucleic acid sequences for the preparation of a medicament useful as a vaccine against hepatitis C, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a signal peptide from a type I membrane protein, preferably a HCV core protein, and a HCV E1 protein and/or a HCV E2 protein ;
and, when transferred into cells of a subject, the nucleic acid sequences allow the production of structural proteins from hepatitis C virus and retrovirus, wherein the structural proteins form virus-like particles that are immunogenic.

According to a specific embodiment, said packaging competent retroviral genome and core proteins are derived from a retrovirus selected from the group consisting of MLV, ALV, RSV, MPMV, HIV-1, HIV-2, SIV, EIAV, CAEV, and HFV.

Advantageously, the packaging competent retroviral genome further comprises a marker gene or an immune modulator

Preferably, said third nucleic acid sequence comprises a cDNA encoding a polyprotein that further comprises a HCV p7 protein. Thus, preferably said polyprotein comprises successively a signal peptide from a type I membrane protein, preferably a HCV core protein, a HCV E1 protein and/or a HCV E2 protein, and optionally a HCV p7 protein.

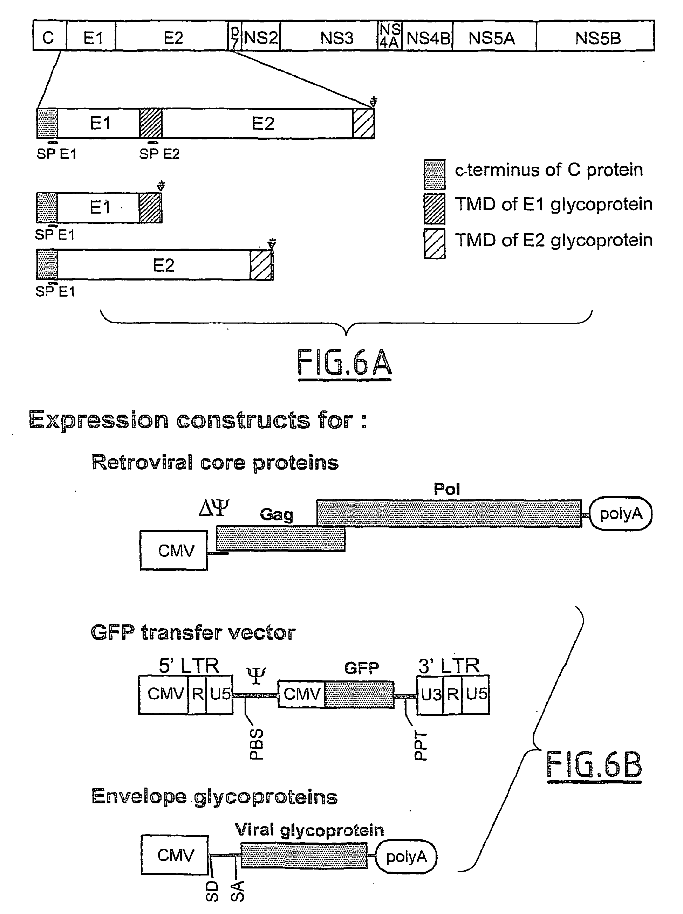

An example of HCV E1 E2 and retroviral expression constructs is shown in figures 6A and 6B.

In the method described above, the polyprotein may comprise a HCV core protein linked to a HCV E1 protein, a HCV core protein linked to a HCV E2 protein, or successively a HCV core protein, a HCV E1 protein and a HCV E2 protein, or successively a HCV core protein, a HCV E2 protein and a HCV E1 protein. Said polyprotein may further comprise successively a HCV core protein, a HCV E1 protein and a p7 protein, or successively a HCV core protein, a HCV E2 protein and a HCV p7 protein, or successively HCV core protein, a HCV E1 protein, a HCV E2 protein and a HCV p7 protein, or successively a HCV core protein, a HCV E2 protein, a HCV p7 protein, and a HCV E1 protein.

According to an embodiment, HCV E1 and/or E2, and optionally HCV p7 protein, are native proteins. According to another embodiment, HCV E1 and/or E2 glycoproteins, and optionally HCV p7 protein, are mutated to obtain particles that may be useful for characterizing the glycoprotein determinants for HCV infectivity. A preferred mutant of E2 protein is the C-terminally truncated form of E2 protein, wherein the C-terminal Ala residue (amino acid 746 in SEQ ID N°16) has been deleted. In another preferred mutant of E2 protein, the hypervariable region I (located in the N-terminus region, *i.e*. the first 27 amino acids of the E2 protein after the signal peptide, i.e. amino-acids 384-410 of SEQ ID N°16) is deleted.

Preferably, said E1 and E2 glycoproteins are both derived from a same HCV strain. Preferably, said E1 or E2 glycoprotein, and p7 protein are both derived from a same HCV strain. Still preferably, said E1 and E2 glycoproteins, and p7 protein are derived from a same HCV strain.

According to another embodiment said HCV core protein is a carboxy terminus form (ΔC) of HCV core protein. In particular, said HCV core protein may comprise the last 21 amino acids of the carboxy-terminus of HCV core.

For the purpose of transfection, said first, second and third nucleic acid sequences may be carried on a same vector, or on two or three separated vectors.

In particular, plasmoviruses, adenoretroviruses and replicating pseudo-viruses are examples of vectors suitable for carrying the above-mentioned sequences. A plasmovirus vaccine consists in such a plasmid DNA preparation, that allow expression of hepacivirus pseudo-particles after administration in an patient in order to elicit a immune response against said hepacivirus or GB virus. Administration of such a plasmovirus vaccine being achieved for preventive vaccination into people at risk for hepacivirus or GB virus-induced disease or for therapeutic vaccination into hepacivirus or GB virus-infected patients. Adenoretroviruses consist in an alternative way to provide the above-mentioned nucleic acid sequences encoding hepacivirus pseudo-particles. In this case, it is possible to design three independent adenoretroviruses, *i.e*. recombinant adenoviruses, that encode the three nucleic acid sequences mentioned above (retroviral core and genome and hepacivirus or GB virus glycoproteins), or, alternatively, it is also possible to design a single adenoretrovirus, derived from "guttless" recombinant adenoviruses, that contains the different nucleic acid sequences. Such adenoretroviruses can be administered to patient as for plasmoviruses, in order to elicit an anti-hepacivirus or GB virus immune response. Replicating pseudo-retroviruses are another alternative possibility to express all the above-mentioned nucleic acid sequences encoding the hepacivirus pseudo-particles. Such structures are in fact hepacivirus pseudo-particles whose genome is engineered to allow, following infection, its propagation into cells of an inoculated patient, thereby inducing the production of further replicating hepacivirus or GB virus-pseudo-particles. In this case the genome of a retrovirus is modified so as to express the hepacivirus or GB virus E1 E2 glycoproteins in place of the retroviral Env gene (encoding the retroviral glycoproteins). The genes encoding the retroviral core proteins are left unchanged. Futhermore an additional gene, encoding a marker gene or an immunomodulator, for example, can be expressed from this genome.

In accordance with the present invention there may be employed conventional molecular biology, microbiology, and recombinant DNA techniques within the skill of the art. Such techniques are explained fully in the literature. See, *e.g*., Sambrook et al., 1989 ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et *al*., 1994.

In particular, the vectors disclosed herein may be introduced into the target cell by means of any technique known for the delivery of nucleic acids to the nucleus of cells, either in culture, *ex vivo,* or *in vivo.*

Introduction of the nucleic acid sequences may be performed by any standard method well known by one skilled in the art, *e.g*. transfection, electroporation, microinjection, transduction, cell fusion, DEAE dextran, calcium phosphate precipitation, or use of a gene gun (see for instance Wu et al., 1992 ; Wu et al, 1988).

The donor nucleic acid targeting system can also be introduced by lipofection. In particular, the use of liposomes and/or nanoparticles may be contemplated for the introduction of the donor nucleic acid targeting system into host cells. Nanocapsules can generally entrap compounds in a stable and reproducible way. Ultrafine particles (sized around 0.1 µm) that can be designed using biodegradable polyalkyl-cyanoacrylate polymers are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 A, containing an aqueous solution in the core. The use of cationic lipids may promote encapsulation of negatively charged nucleic acids, and also promote fusion with negatively charged cell membranes (Felgner et al., 1989).

*In vivo* targeted gene delivery is described in international patent publication WO 95/28 494. Alternatively, the vector can be introduced *in vivo* by lipofection, using liposomes or nanoparticles as above described. It is also possible to introduce the vector *in vivo* using techniques that are similar to the techniques that are employed *in vitro* (*e.g.* transfection, electroporation...).

### Transformed cells

The invention further relates to a transformed host cell that contains :
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a signal peptide from a type I membrane protein, preferably a hepacivirus or GB virus core protein, and a hepacivirus or GB virus E1 protein and/or a hepacivirus or GB virus E2 protein.

Preferably, said third nucleic acid sequence comprises a cDNA encoding a polyprotein that further comprises a hepacivirus or GB virus p7 protein. Thus, preferably said polyprotein comprises successively a signal peptide from a type I membrane protein, preferably a hepacivirus or GB virus core protein, a hepacivirus or GB virus E1 protein and/or a hepacivirus or GB virus E2 protein, and optionally a hepacivirus or GB virus p7 protein.

Such a transformed host cell is obtainable as described in a method above.

In another aspect, the invention relates to the use of a transformed host cell as defined above, for the identification of molecules capable of interfering with hepacivirus or GB virus entry in cells. The invention provides in particular a method of *ex vivo* screening or identification of molecules capable of interfering with hepacivirus or GB virus entry in cells comprising comparison of the level of transformed host cell fusion to a target host cell, in the presence or the absence of a candidate molecule. Said method preferably comprises the steps consisting of:
- co-culturing a transformed host cell with a target host cell, in the absence or presence of a candidate molecule, under conditions that allow syncytia formation, *i.e.* cell-cell fusion, and hepacivirus-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with hepacivirus or GB virus entry the candidate molecule for which syncytia formation, as measured in the presence of said molecule, is decreased as compared to syncytia formation measured in the absence of any candidate molecule.

Preferably, said hepacivirus or GB virus is a hepatitis C virus. The invention thus also relates to a transformed host cell that contains :
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a signal peptide from a type I membrane protein, preferably a HCV core protein, and a HCV E1 protein and/or a HCV E2 protein.

According to another embodiment, said third nucleic acid sequence comprises a cDNA encoding a polyprotein that further comprises a HCV p7 protein. Preferably said polyprotein comprises successively a signal peptide from a type I membrane protein, preferably a HCV core protein, a HCV E1 protein and/or a HCV E2 protein, and optionally a HCV p7 protein.

Such a transformed host cell is obtainable as described in a method above.

In another aspect, the invention relates to the use of a transformed host cell as defined above, for the identification of molecules capable of interfering with HCV entry in cells. The invention provides in particular a method of *ex vivo* screening or identification of molecules capable of interfering with HCV entry in cells comprising comparison of the level of transformed host cell fusion to a target host cell, in the presence or the absence of a candidate molecule. Said method preferably comprises the steps consisting of:
- co-culturing a transformed host cell with a target host cell, in the absence or presence of a candidate molecule, under conditions that allow syncytia formation, *i.e.* cell-cell fusion, and HCV-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with HCV entry the candidate molecule for which syncytia formation, as measured in the presence of said molecule, is decreased as compared to syncytia formation measured in the absence of any candidate molecule.

Contacting a transformed host cell with a target host cell, and a candidate molecule can be carried out by contacting simultaneously said transformed host cell, target host cell and candidate molecule. Otherwise, two of these three elements can be contacted under conditions sufficient to allow their interaction before addition of the third missing element.

Preferably said target host cell is not transformed , i.e. said target host cell does not contains at least one of the first, second, and third nucleic acid sequence as defined above.

Syncytia formation can be readily assessed by one skilled in the art. Briefly, the coculture is submitted to a acidic pH drop by incubation for 5 min at pH-5 and incubated in a normal medium for an additional 12 hrs. Cultures are then stained by adding the May-Grunwald and Giemsa solutions (MERCK) according to the manufacturer recommendations. Cells containing two or more nuclei can be defined as syncytia. A fusion index is then defined as the percentage of (N-S)/T where N is the number of nuclei in the syncytia, S is the number of syncytia and T is the total number of nuclei counted.

### Hepacivirus-like particles

In the method described above no structural modifications of the E1E2 glycoproteins are required for their correct assembly on retroviral cores. The method of the invention thus makes it possible to generate high titre infectious hepacivirus pseudo-particles, ex vivo and in particular HCV pseudo-particles, with functional E1 E2 glycoproteins. As demonstrated herein, these particles constitute a valid model of hepacivirus or GB virus virions, and in particular of HCV virions, as regards to early steps of viral infection cycle. However, depending of the intended use of the pseudo-particle of the invention, mutant E1, E2 or p7 protein may be assembled on the hepacivirus-like particle, with the limitations recited in the definition section.

The invention further relates to an infectious hepacivirus-like particle, comprising the core proteins from a retrovirus, E1 and/or E2 hepacivirus or GB virus glycoprotein(s), and optionally hepacivirus or GB virus p7 protein. Such a particle is obtainable by a method as described above.

According to an embodiment, the infectious particle of the invention may comprise native hepacivirus or GB virus E1 protein, or native hepacivirus or GB virus E2 protein, or native hepacivirus or GB virus E1 protein and native hepacivirus or GB virus E2 protein. Preferably said E1 and E2 glycoproteins are both derived from a same hepacivirus or GB virus strain. According to another embodiment, E1 and/or E2 glycoproteins are mutated.

According to another embodiment the infectious particle of the invention may comprise native hepacivirus or GB virus E1 and native hepacivirus or GB virus p7 proteins, or native hepacivirus or GB virus E2 and native hepacivirus or GB virus p7 proteins, or native hepacivirus or GB virus E1 protein, native hepacivirus or GB virus E2 protein and native hepacivirus or GB virus p7 protein Preferably, said E1 or E2 glycoprotein and p7 protein are both derived from a same hepacivirus or GB virus strain. Still preferably, said E1 and E2 glycoproteins, and p7 protein are derived from a same hepacivirus or GB virus strain. According to another embodiment, E1 and/or E2 glycoproteins and/or p7 protein are mutated.

Preferably, said hepacivirus or GB virus is a hepatitis C virus. The invention thus also relates to an infectious HCV-like particle, comprising the core proteins from a retrovirus, E1 and/or E2 HCV glycoprotein(s), and optionally p7 protein. Such a particle is obtainable by a method as described above.

According to an embodiment, the infectious particle of the invention may comprise native HCV E1 protein, or native HCV E2 protein, or native HCV E1 protein and native HCV E2 protein. Preferably said E1 and E2 glycoproteins are both derived from a same HCV strain. According to another embodiment, E1 and/or E2 glycoproteins are mutated.

According to another embodiment the infectious particle of the invention may comprise native HCV E1 and native HCV p7 proteins, or native HCV E2 and native HCV p7 proteins, or native HCV E1 protein, native HCV E2 protein and native HCV p7 protein Preferably, said E1 or E2 glycoprotein and p7 protein are both derived from a same HCV strain. Still preferably, said E1 and E2 glycoproteins, and p7 protein are derived from a same HCV strain. According to another embodiment, E1 and/or E2 glycoproteins and/or p7 protein are mutated.

In the above embodiment, a mutated E2 protein is preferably the C-terminally truncated form of E2 protein, wherein the C-terminal Ala residue (amino acid 746 in SEQ ID N°16) has been deleted.

In another preferred embodiment, the hypervariable region I (located in the N-terminus region, *i.e*. the first 27 amino acids of the E2 protein after the signal peptide, i.e. amino-acids 384-410 of SEQ ID N°16) is deleted. The HCV-like particles (called ΔHVR1) produced with E2 protein deleted from this region are particularly advantageous as a diagnostic tool or in vaccination, to enhance induction or binding of neutralizing antibodies. The ΔHVR1 HCV-like particles are also of interest to identify the epitopes/mechanisms within the HCV glycoproteins that can be targeted/inhibited by neutralizing antibodies or other therapeutic agents.

Said retrovirus may be selected from the group consisting of MLV, ALV, RSV, MPMV, HIV-1, HIV-2, SIV, EIAV, CAEV, and HFV.

Advantageously, said infectious particles further carry a transgene. For instance said transgene may be a marker gene which make it possible to follow-up cell infection by the infectious particles of the invention and can find application for instance in the identification of a cell receptor involved in hepacivirus or GB virus entry, and in particular HCV entry. Said transgene can also be a gene encoding a molecule of therapeutic interest and/or a suicide gene. Accordingly, the particles of the invention that specifically target primary or cancerous hepatocytes comprise a useful vector for gene transfer and/or gene therapy.

### Use of the infectious hepacivirus-like particles of the invention

### Hepacivirus or GB virus cell receptor identification

High infectivity of these particles makes it possible for the investigation of the role of hepacivirus or GB virus (HCV) E1 and E2 glycoproteins and their potential receptors in cell entry, hepacivirus or GB virus host-range and neutralisation by antibodies from hepacivirus or GB virus patient sera. These particles make it possible for the investigation of the role of p7 protein in E1 E2 maturation and functions, viral assembly, budding and release.

The invention therefore concerns the use of a hepacivirus-like infectious particle as described above, for *ex vivo* identification of a cell receptor for hepacivirus or GB virus E1 and/or E2 glyccprotein.

According to an embodiment, the invention provides a method for *ex vivo* identification of a receptor for hepacivirus or GB virus E1 and/or E2 glycoprotein comprising detection of the binding of said particle to a cell receptor. More specifically, the method may comprise the steps consisting of:
- contacting a cell susceptible to hepacivirus or GB virus infection with an infectious hepacivirus-like particle of the invention, under conditions sufficient to allow specific binding of said particle to a receptor expressed at the surface of said cell;
- detecting binding of said particle to a receptor; and
- identifying said receptor.

Preferably, said hepacivirus or GB virus is a hepatitis C virus. The invention therefore concerns the use of an HCV-like infectious particle as described above, for *ex vivo* identification of a cell receptor for HCV E1 and/or E2 glycoprotein.

According to an embodiment, the invention provides a method for *ex vivo* identification of a receptor for HCV E1 and/or E2 glycoprotein comprising detection of the binding of said particle to a cell receptor. More specifically, the method may comprise the steps consisting of:
- contacting a cell susceptible to HCV infection with an infectious HCV-like particle of the invention, under conditions sufficient to allow specific binding of said particle to a receptor expressed at the surface of said cell;
- detecting binding of said particle to a receptor; and
- identifying said receptor.

A cell susceptible to a hepacivirus or GB virus infection, and in particular to a HCV infection, may preferably be selected from the group consisting of a hepatocyte cell line, such as Huh-7 human hepatocellular carcinoma (Nakabayashi et *al*., 1982), PLC/PRF/5 human hepatoma (CRL-8024), Hep3B human hepatocellular carcinoma (ATCC HB-8064), or HepG2 human hepatocellular carcinoma (HB-8065), and a primary human hepatocyte. Primary human hepatocytes may be isolated from human adult biopsy samples according to procedures well-known by one skilled in the art. One can for example refer to Guguen-Guillouzo and Guillouzo (1986). Otherwise such cells are commercially available, and can be purchased for instance from Biopredic International (Rennes, France).

Detection of particle binding to a receptor can be achieved according to classical procedures well known by one skilled in the art. For instance, this could involve radioactive, enzyme or fluorescent labelling of the particles of the invention, and subsequent detection with an appropriate method. A number of fluorescent materials are known and can be utilized as labels. These include, for example, fluorescein, rhodamine, auramine, Texas Red. Enzyme labels consist in conjugation of an enzyme to a molecule of interest, *e*.*g*. a polypeptide, and can be detected by any of colorimetric, spectrophotometric, or fluorospectrophotometric techniques. Flow cytometry analysis (FACS) together with labelled antibodies directed against E1 or E2 proteins harboured by the pseudo-particles of the invention is also appropriate.

According to another embodiment, the invention provides a method for ex *vivo* identifying a cell receptor for a hepacivirus or GB virus comprising the step consisting of:
- transfecting a cell which is not permissive for hepacivirus or GB virus infection with a nucleic acid sequence encoding a protein likely to be a receptor for hepacivirus or GB virus;
- contacting said transformed cell with a hepacivirus-like particle of the invention;
- determining whether said transformed cell has become permissive or not for hepacivirus or GB virus infection; and
- identifying as a cell receptor for a hepacivirus or GB virus said protein expressed by the transformed cell that has become permissive.

Preferably, the invention provides a method for ex *vivo* identifying a cell receptor for HCV comprising the step consisting of:
- transfecting a cell which is not permissive for HCV infection with a nucleic acid sequence encoding a protein likely to be a receptor for HCV;
- contacting said transformed cell with a HCV-like particle of the invention;
- determining whether said transformed cell has become permissive or not for HCV infection; and
- identifying as a cell receptor for HCV said protein expressed by the transformed cell that has become permissive.

Determination of whether the transformed cell has become permissive for hepacivirus or GB virus infection, and in particular HCV infection, can be readily achieved using the hepacivirus-like (HCV-like) particles of the invention. In particular, where said particles carry a marker gene, such as GFP, permissivity (*i*.*e*. the capacity of cells to be infected with a hepacivirus or GB virus, or with a hepacivirus-like particle, in particular with HCV or with HCV-like particles) can be assessed by FACS analysis of the transformed cells. Where the marker gene is an antibiotic resistance gene, identification of cells infected by the hepacivirus-like (HCV-like) particle is readily achieved through exposure to said antibiotic.

Where one does not suspect a given protein to be a receptor for hepacivirus or GB virus entry, in cells, the above method can advantageously be adapted for the screening and the identification of a cell receptor for a hepacivirus or GB virus, such as HCV. In particular, an expression cDNA library can be prepared, for instance from a cDNA library obtained by reverse-transcription of cellular mRNAs from a cell permissive for hepacivirus or GB virus infection, in particular for HCV infection. Expression of such a cDNA library would be driven by a constitutive promoter whose nucleic acid sequence has been fused to the cDNA library in suitable vectors. Such a library would contain a vector encoding a cell receptor for a hepacivirus or GB virus, for instance for HCV. Non permissive cells can then be transfected with this expression library and further screened for the identification of a cell receptor for a hepacivirus or GB virus.

To this end, the invention proposes a method for *ex vivo* identifying a cell receptor for hepacivirus or GB virus comprising the step consisting of:
- providing an expression cDNA library obtained from a cell permissive for hepacivirus or GB virus infection;
- transfecting cells that are not permissive for hepacivirus or GB virus infection with said expression cDNA library;
- contacting said transformed cells with hepacivirus-like particles of the invention;
- identifying and isolating those transformed cells that have become permissive for hepacivirus or GB virus infection;
- isolating the expression vector transfected in cells that have become permissive; and
- identifying as a receptor for hepacivirus or GB virus the proteins encoded by the cDNA sequence of said isolated expression vectors.

Preferably said hepacivirus or GB virus is a hepatitis C virus. The invention thus proposes a method for ex *vivo* identifying a cell receptor for HCV comprising the step consisting of:
- providing an expression cDNA library obtained from a cell permissive for HCV infection;
- transfecting cells that are not permissive for HCV infection with said expression cDNA library;
- contacting said transformed cells with HCV-like particles of the invention;
- identifying and isolating those transformed cells that have become permissive for HCV infection;
- isolating the expression vector transfected in cells that have become permissive; and
- identifying as a receptor for HCV the proteins encoded by the cDNA sequence of said isolated expression vectors.

Determination of whether the transformed cell has become permissive for hepacivirus or GB virus (HCV) infection can be readily achieved using the hepacivirus-like (HCV-like) particles of the invention. In particular, where said particles carry a marker gene, such as GFP, permissivity (*i*.*e*. the capacity of cells to be infected with hepacivirus or GB virus, and in particular with hepacivirus-like particles, for instance with HCV or with HCV-like particles) can be assessed by FACS analysis of the transformed cells. Where the marker gene is an antibiotic resistance gene, identification of cells infected by hepacivirus-like particles, in particular by HCV-like particles, is readily achieved through exposure to said antibiotic.

Advantageously, the expression cDNA library is expressed from retroviral vectors that comprise glycoproteins that allow infection of the hepacivirus or GB virus (HCV) non permissive cells. Such glycoproteins can be the VSV-G glycoprotein derived from vesicular stomatitis virus (VSV) whose receptor is expressed in most cell types ex *vivo.* Such viral particles can be assembled using a packaging competent retrovirus-derived genome that comprises the expression cDNA library, and optionally a marker gene. According to this embodiment the method for isolating the expression vector expressed in cells that have become permissive to infection by the hepacivirus-like (HCV-like) particles of the invention is greatly facilitated. Indeed this latter embodiment is particularly advantageous in that the process of cell infection with retroviral vectors has greater efficacy, as compared to cell transfection. Furthermore, cell infection leads to stable integration of viral genome in the cellular genome, which greatly facilitates recovery and isolation of the cDNA of interest. Accordingly, transgenes, *i*.*e*. cDNA and marker gene that are carried by the pseudo-particles of the invention, are found to be stably expressed by infected cells. This in contrast with classical vectors used for transfection that do not integrate into cellular genome and for which expression may be transient.

### Identification of agents interfering with hepacivirus or GB virus infection

In another aspect, the invention relates to the use of an infectious particle as defined above, for the identification of molecules capable of interfering with hepacivirus or GB virus, and in particular HCV, entry in cells.

In particular, herein is provided a method of ex *vivo* screening or identification of molecules capable of interfering with hepacivirus or GB virus entry in cells comprising comparison of the level of cell infection by the particles of the invention in the presence or the absence of a candidate molecule. Said method preferably comprises the steps consisting of:
- contacting a cell susceptible to hepacivirus or GB virus infection with an infectious hepacivirus-like particle, in the absence or presence of a candidate molecule, under conditions that allow cell infection with hepacivirus-like particle in the absence of any candidate molecule;
- assessing cell infectivity in the absence and in the presence of said candidate molecule;
- comparing cell infectivity measured in presence of said candidate molecule with cell infectivity measured in absence of any candidate molecule;
- identifying as a molecule capable of interfering with hepacivirus or GB virus entry the candidate molecule for which cell infectivity, as measured in the presence of said molecule, is decreased as compared to cell infectivity measured in the absence of any candidate molecule.

Contacting a cell susceptible to hepacivirus or GB virus infection with an infectious hepacivirus-like particle, and a candidate molecule can be carried out by contacting simultaneously said cell, hepacivirus-like particle and candidate molecule. Otherwise, two of these three elements can be contacted under conditions sufficient to allow their interaction before addition of the third missing element.

Cell infectivity can be readily assessed by one skilled in the art. One can take advantage of the embodiment wherein the infectious hepacivirus-like particle carries a detectable marker gene to detect cell infection. In a preferred embodiment, the marker gene is a fluorescent marker gene, such as GFP, and the infection is detected by means of fluorescence measurement, for instance by flow cytometry analysis of cells contacted with said infectious particles.

A cell suitable to be used in the method of identification of molecules interfering with hepacivirus or GB virus cell entry may be selected from the group consisting of a hepatocyte cell line and a primary human hepatocyte, as described above.

Preferably said hepacivirus or GB virus is a hepatitis C virus. The invention thus further provides a method of ex *vivo* screening or identification of molecules capable of interfering with HCV entry in cells comprising comparison of the level of cell infection by the particles of the invention in the presence or the absence of a candidate molecule. Said method preferably comprises the steps consisting of:
- contacting a cell susceptible to HCV infection with an infectious HCV-like particle, in the absence or presence of a candidate molecule, under conditions that allow cell infection with HCV-like particle in the absence of any candidate molecule;
- assessing cell infectivity in the absence and in the presence of said candidate molecule;
- comparing cell infectivity measured in presence of said candidate molecule with cell infectivity measured in absence of any candidate molecule;
- identifying as a molecule capable of interfering with HCV entry the candidate molecule for which cell infectivity, as measured in the presence of said molecule, is decreased as compared to cell infectivity measured in the absence of any candidate molecule.

Contacting a cell susceptible to HCV infection with an infectious HCV-like particle, and a candidate molecule can be carried out by contacting simultaneously said cell, HCV-like particle and candidate molecule. Otherwise, two of these three elements can be contacted under conditions sufficient to allow their interaction before addition of the third missing element.

Cell infectivity can be readily assessed by one skilled in the art. One can take advantage of the embodiment wherein the infectious HCV-like particle carries a detectable marker gene to detect cell infection. In a preferred embodiment, the marker gene is a fluorescent marker gene, such as GFP, and the infection is detected by means of fluorescence measurement, for instance by flow cytometry analysis of cells contacted with said infectious particles.

A cell suitable to be used in the method of identification of molecules interfering with HCV cell entry may be selected from the group consisting of a hepatocyte cell line and a primary human hepatocyte, as described above.

Such molecules capable of interfering with hepacivirus or GB virus , and in particular with HCV, entry in cells may constitute new antiviral drugs.

### Hepacivirus or GB virus infection diagnosis

The infectious particles of the invention are further useful for diagnosis of hepacivirus or GB virus infection and follow-up of hepacivirus or GB virus infection, for instance to assess efficacy of a therapy in a patient.

The invention thus concerns the use of an infectious hepacivirus-like particle for the *in vitro* detection of antibodies directed against hepacivirus or GB virus in a biological sample from a subject susceptible to be infected with hepacivirus or GB virus. Said biological sample may be a biological fluid, such as blood or serum, or a tissue biopsy. In a specific embodiment, said antibodies are directed against E1 and/or E2 hepacivirus or GB virus glycoproteins.

Accordingly, the invention provides a method of *in vitro* diagnosis of a hepacivirus or GB virus infection in a patient comprising detecting immune complexes formed by interaction of anti-hepacivirus or GB virus antibodies likely to be present in a biological sample of the patient, with hepacivirus-like particle of the invention. Said method may in particular comprise the steps consisting of:
- contacting a biological sample with an infectious hepacivirus-like particle of the invention under conditions sufficient to allow formation of complexes by binding of said infectious particle to antibodies directed against hepacivirus or GB virus present in the biological sample;
- detecting said complexes, which presence is indicative of a hepacivirus or GB virus infection.

The presence of antibodies reactive with hepacivirus-like particles can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the hepacivirus-like particle and the antibody or antibodies reacted therewith.

In another embodiment, said method of *in vitro* diagnosis of a hepacivirus or GB virus infection in a patient comprises detecting an inhibitory effect of anti-hepacivirus or GB virus antibodies likely to be present in a biological sample of the patient, on the infection of a permissive cell by a hepacivirus-like particle of the invention. Said method may in particular comprise the steps consisting of:
- contacting a cell permissive for hepacivirus or GB virus infection with a hepacivirus-like particle and a biological sample;
- comparing cell infectivity measured in presence of said biological sample with cell infectivity measured in absence of said biological sample;
- detecting the inhibition of hepacivirus-like particle infection of a permissive cell as a decrease in cell infectivity measured in presence of said biological sample compared with cell infectivity measured in absence of said biological sample, said inhibition being indicative of a hepacivirus or GB virus infection.

This embodiment is advantageous in that the method relies on the detection of the specific antibodies that are neutralizing for cell infection, i.e. those patient's antibodies that are effective against viraemia.

In a further embodiment of this invention, commercial diagnostic kits may be useful to carry out the above diagnosis methods, by detecting the presence or absence of immune complexes formed by hepacivirus or GB virus particles and antibodies directed against hepacivirus or GB virus in a biological sample from a subject susceptible to be infected with hepacivirus or GB virus, or by detecting an inhibition of hepacivirus-like particle infection of a permissive cell by anti-hepacivirus or GB virus neutralizing antibodies likely to be present in a biological sample of the patient. Such kits may comprise at least a hepacivirus-like particle of the present invention. Where the method involves detection of immune complexes, the kits may further comprise appropriate means of detection of said immune complexes. Preferably the kit of the invention further comprises directions, and protocols, depending upon the method selected, *e*.*g*., "competitive", "sandwich", and the like. The kits may also contain peripheral reagents such as buffers, stabilizers, etc..

Preferably said hepacivirus or GB virus is a hepatitis C virus. The infectious particles of the invention are further useful for diagnosis of HCV infection and follow-up of HCV infection, for instance to assess efficacy of a therapy in a patient.

The invention thus concerns the use of an infectious HCV-like particle for the *in vitro* detection of antibodies directed against HCV in a biological sample from a subject susceptible to be infected with HCV. Said biological sample may be a biological fluid, such as blood or serum, or a tissue biopsy. In a specific embodiment, said antibodies are directed against E1 and/or E2 HCV glycoproteins.

Accordingly, the invention provides a method of *in vitro* diagnosis of a HCV infection in a patient comprising detecting immune complexes formed by interaction of anti-HCV antibodies likely to be present in a biological sample of the patient, with HCV-like particle of the invention. Said method may in particular comprise the steps consisting of:
- contacting a biological sample with an infectious HCV-like particle of the invention under conditions sufficient to allow formation of complexes by binding of said infectious particle to antibodies directed against HCV present in the biological sample;
- detecting said complexes, which presence is indicative of a HCV infection.

The presence of antibodies reactive with HCV-like particles can be detected using standard electrophoretic and immunodiagnostic techniques, including immunoassays such as competition, direct reaction, or sandwich type assays, as described above. The reactions generally include revealing labels such as fluorescent, chemiluminescent, radioactive, enzymatic labels or dye molecules, or other methods for detecting the formation of a complex between the HCV-like particle and the antibody or antibodies reacted therewith.

In another embodiment, said method of *in vitro* diagnosis of a HCV infection in a patient comprises detecting an inhibitory effect of anti-HCV antibodies likely to be present in a biological sample of the patient, on the infection of a permissive cell by a HCV-like particle of the invention. Said method may in particular comprise the steps consisting of:
- contacting a cell permissive for HCV infection with a HCV-like particle and a biological sample;
- comparing cell infectivity measured in presence of said biological sample with cell infectivity measured in absence of said biological sample;
- detecting the inhibition of HCV-like particle infection of a permissive cell as a decrease in cell infectivity measured in presence of said biological sample compared with cell infectivity measured in absence of said biological sample, said inhibition being indicative of a HCV infection.

This embodiment is advantageous in that the method relies on the detection of the specific antibodies that are neutralizing for cell infection, i.e. those patient's antibodies that are effective against viraemia.

In a further embodiment of this invention, commercial diagnostic kits may be useful to carry out the above diagnosis methods, by detecting the presence or absence of immune complexes formed by HCV particles and antibodies directed against HCV in a biological sample from a subject susceptible to be infected with HCV, or by detecting an inhibition of HCV-like particle infection of a permissive cell by anti-HCV neutralizing antibodies likely to be present in a biological sample of the patient. Such kits may comprise at least a HCV-like particle of the present invention. Where the method involves detection of immune complexes, the kits may further comprise appropriate means of detection of said immune complexes. Preferably the kit of the invention further comprises directions, and protocols, depending upon the method selected, *e*.*g*., "competitive", "sandwich", and the like. The kits may also contain peripheral reagents such as buffers, stabilizers, etc..

### Vaccination against hepacivirus or GB virus infection

In another aspect of the invention, the infectious hepacivirus-like particles may be used for vaccination purposes.

The description thus proposes a method of vaccination, notably against hepacivirus or GB virus infection, that comprises administration of a hepacivirus-like particle to a subject in need thereof. The invention also relates to a vaccine composition comprising a hepacivirus-like particle and a pharmaceutically acceptable carrier. The invention further provides a immunogenic composition comprising in a pharmaceutical acceptable carrier, a hepacivirus-like particle disclosed herein.

The vaccine and immunogenic compositions of the invention may be drawn to confer immunity, or elicit an immune response against hepacivirus or GB virus.

However, where the hepacivirus-like particles of the invention further carry an additional gene encoding another antigen, different from hepacivirus or GB virus antigens, the invention provides a recombinant viral vaccine useful to raise an immune response against said antigen. Actually, the use of pseudo-particles described herein makes it possible to improve the elicited immune response through combining several presentations and processing pathways of an antigen. For instance, a vaccine composition of the invention, when administered, results in the hepacivirus-like particles infecting cells of the host. The transgene encoding the antigen is then integrated in the cellular genome, and subsequently expressed by the cell, such that there is both a cellular and a humoral immune response elicited by the vaccine composition.

Advantageously, the hepacivirus-like particles may further carry a transgene encoding an immune modulator, which allows for enhancement of the raised immune reaction.

Preferably said hepacivirus or GB virus is a hepatitis C virus. The description thus proposes a method of vaccination, notably against HCV infection, that comprises administration of a HCV-like particle to a subject in need thereof. The invention also relates to a vaccine composition comprising a HCV-like particle and a pharmaceutically acceptable carrier. The invention further provides a immunogenic composition comprising in a pharmaceutical acceptable carrier, a HCV-like particle disclosed herein.

The vaccine and immunogenic compositions of the invention may be drawn to confer immunity, or elicit an immune response against HCV.

However, where the HCV-like particles of the invention further carry an additional gene encoding another antigen, different from HCV antigens, the invention provides a recombinant viral vaccine useful to raise an immune response against said antigen. Actually, the use of pseudo-particles described herein makes it possible to improve the elicited immune response through combining several presentations and processing pathways of an antigen. For instance, a vaccine composition of the invention, when administered, results in the HCV-like particles infecting cells of the host. The transgene encoding the antigen is then integrated in the cellular genome, and subsequently expressed by the cell, such that there is both a cellular and a humoral immune response elicited by the vaccine composition.

Advantageously, the HCV-like particles may further carry a transgene encoding an immune modulator, which allows for enhancement of the raised immune reaction.

The vaccination or immunogenic composition of the present invention may additionally contain an adjuvant. A number of adjuvants are known to those skilled in the art. Examples of suitable adjuvants include, for example, include aluminum hydroxide; Saponin; detergents such as Tween 80; animal, mineral or vegetable oils, Corynebacterium or Propionibacterium -derived adjuvants; Mycobacterium bovis (Bacillus Calmette and Guerinn, or BCG); cytokines; acrylic acid polymers such as carbomer; EMA; or combinations thereof.

The route of administration is any conventional route used in the vaccine field. As general guidance, a vaccine composition of the invention is administered via a mucosal surface, *e*.*g*., an ocular, intranasal, pulmonary, oral, intestinal, rectal, vaginal, and urinary tract surface; or via a parenteral route, *e*.*g*., by an intravenous, subcutaneous, intraperitoneal, intradermal, intraepidermal, or intramuscular route. The choice of administration route depends on the formulation that is selected.

### Vectors for gene transfer and/or therapy

In still another embodiment the particles of the invention may be used as vectors for gene transfer and/or gene therapy. Gene therapy is defined as the introduction of genetic material into a cell in order to either change its phenotype or genotype. Owing to their tropism for hepatic cells, either primary or cancerous, the hepacivirus-like particles, and in particular the HCV-like particles, described herein comprise an efficient gene delivery system specific for hepatocytes. Furthermore, such a delivery system is amenable to scale up for reproducibly producing large titers of infectious, replication-defective hepacivirus-like particles, in particular HCV-like particles.

Accordingly, the description discloses a method for *in vivo* or *in vitro* transferring a transgene of interest in a cell, which method comprises infecting a cell with a hepacivirus-like particle of the invention, wherein the particle carries a transgene of interest.

The invention further relates to the use of a hepacivirus-like particle of the invention, that carries a transgene of interest, for the preparation of a medicament for the prevention or treatment of a disease in a patient, wherein the hepacivirus-like particle allows the transfer of the transgene of interest into a cell of the patient, and encodes a product that has a prophylactic or therapeutic effect against the disease.

Preferably said hepacivirus or GB virus is a hepatitis C virus. The description thus proposes a method for *in vivo* or *in vitro* transferring a transgene of interest in a cell, which method comprises infecting a cell with a HCV-like particle of the invention, wherein the particle carries a transgene of interest.

The invention further relates to the use of a HCV-like particle of the invention, that carries a transgene of interest, for the preparation of a medicament for the prevention or treatment of a disease in a patient, wherein the HCV-like particle allows the transfer of the transgene of interest into a cell of the patient, and encodes a product that has a prophylactic or therapeutic effect against the disease.

Preferably, the targeted cell is a hepatic cell.

The invention will be further understood in view of the following examples and the annexed figures.

### LEGEND TO THE FIGURES:

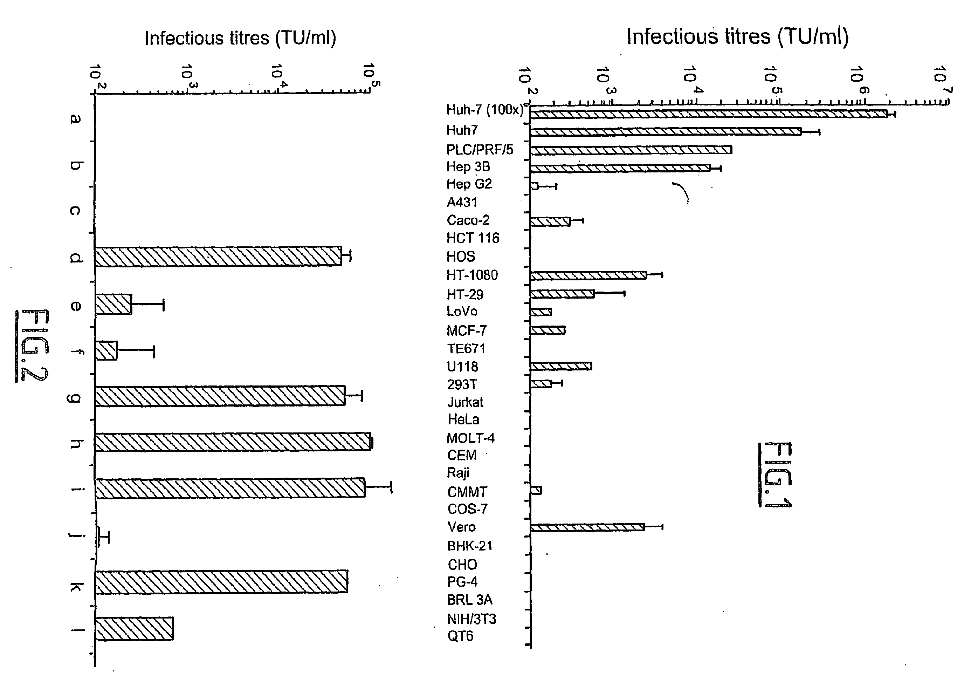

Figure 1 depicts the results of infectivity experiments performed with HCV pseudo-particles of 1 a genotype and different target cell types. Results are displayed as transducing units (TU) per ml of supernatant (mean ± standard deviations of up to six experiments) for HCVpp. The infectivity on Huh-7 cells of HCVpp concentrated 100 times by ultra-centrifugation is shown (100x).
Figure 2 represents the infectious titres determined on Huh-7 target cells with HCV pseudo-particles generated without E1E2 (lane a), without retroviral core proteins (lane b), with MLV-G2A assembly-defective core proteins (lane c), with HIV-1 core proteins (lane d), with E1 or E2 alone (lane e or f, respectively), with E1+E2 expressed in *trans* from two independent vectors (lane g), with HCV-1a E1E2 expressed from the same vector in *cis* (lane h), or with HCV-1 b E1 E2 (lane i). HCVpp were treated with 25 µM AZT (3'-azido-3'-deoxythymidine; Sigma-Aldrich, France) before and during infection of target cells (lane j). Infectivity of HCV pseudo-particles generated with E1 from 1 a HCV genotype and E2 form 1 b HCV or genotype (lane k) or with E1 from 1 b HCV genotype and E2 form 1 a HCV or genotype (lane I). Results are expressed as TU/ml and are displayed as mean ± standard deviations of up to four experiments.

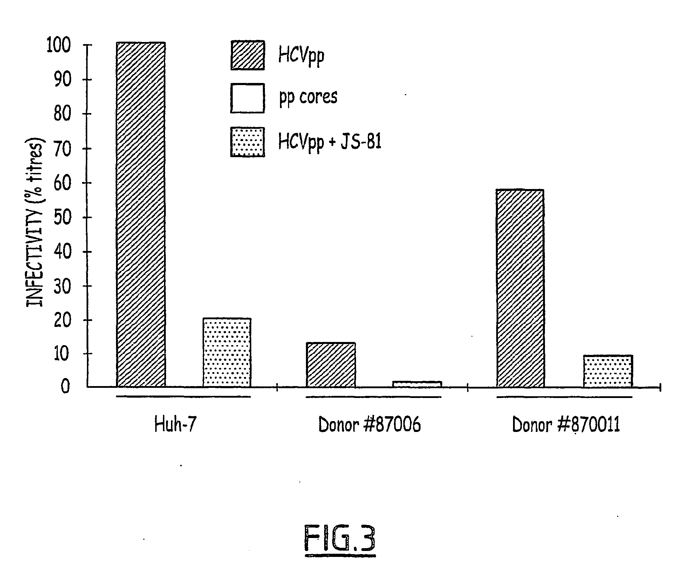

Figure 3 shows the results of infection on human primary hepatocytes derived from two donors with of HCVpp of genotype 1a. Infectivity is expressed as percentage of infectivity determined on Huh-7 cells.

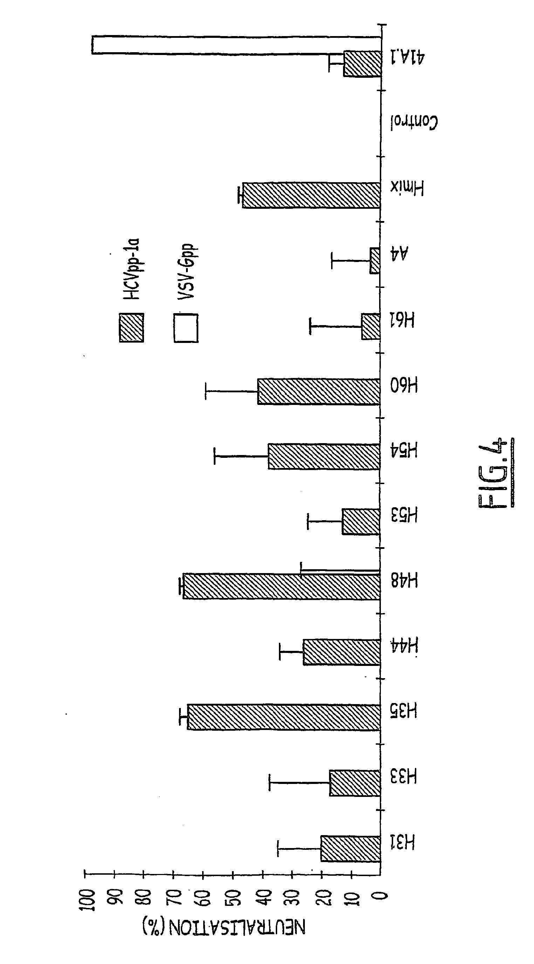

Figure 4 displays neutralisation of HCV pseudo-particles with monoclonal antibodies against E1 (A4) or E2 (H31, H33, H35, H44, H48, H53, H54, H60 and H61) glycoproteins of genotype 1a, with pooled antibodies (Hmix), with no antibodies or using pseudo-particles generated with VSV-G (control). Neutralization of the control was achieved with the VSV-G neutralising 41A.1 monoclonal antibody. Results are expressed as the percentages of inhibition of the average infectious titres ± standard deviations relative to incubation in the absence of antibodies.

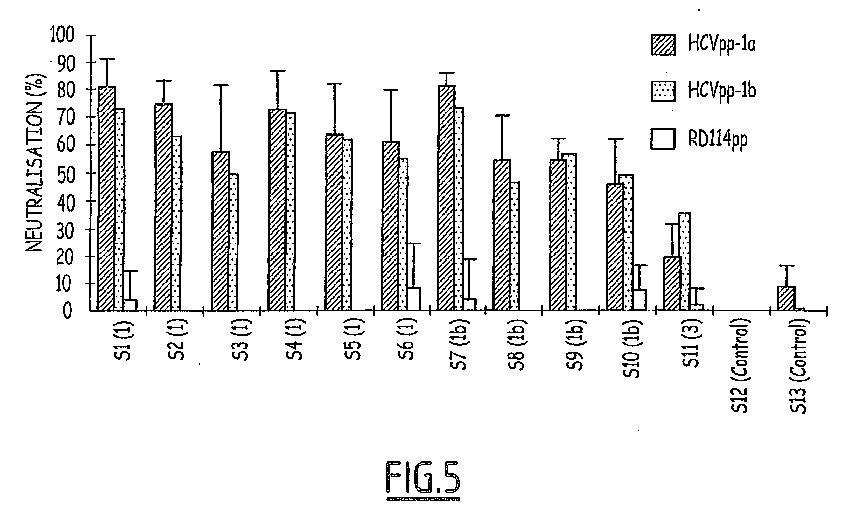

Figure 5 represents neutralisation of HCVpp with HCV patient sera. The genotype of HCV diagnosed in these patients is indicated in brackets. Results are expressed as percentages of inhibition of the average infectious titres ± standard deviations relative to incubation with control sera from healthy individuals.
Figures 6A and 6B show HCV E1E2 and retroviral expression constructs. (A) : A cDNA derived from the HCV polyprotein gene was used to express the E1 E2 glycoproteins and the carboxy-terminus of the C protein, which provides the signal peptide for E1 (SP E1). The position of stop codons (star) inserted in the expression constructs to terminate translation of the proteins is shown. The transmembrane domain (TMD) of E1 provides the signal peptide (SP E2) for the E2 glycoprotein. (B) : The expression constructs encoding the different components required to assemble infectious pseudo-particles are shown. The filled boxes represent the viral genes and the marker gene (GFP) transferred to the infected cells. The open boxes show the cis-acting sequences. LTR, long terminal repeat ; CMV, human cytomegalovirus immediate-early promoter ; PBS, primer binding site ; ψ, packaging sequence ; PPT, poly-purine track ; polyA, polyadenylation site ; SD, splice donor site ; SA, splice acceptor site. Vector particles were produced by cotransfection of plasmids harboring the packaging functions, the transfer vector and the viral glycoproteins into 293 T cells. The viral glycoproteins were the HCV E1 and E2 glycoproteins, expressed individually or as a CE1E2, the VSV-G or the RD114 glycoproteins. The supernatants of transfected cells were collected during transient expression, concentrated by ultracentrifugation, and used for target cell transduction.

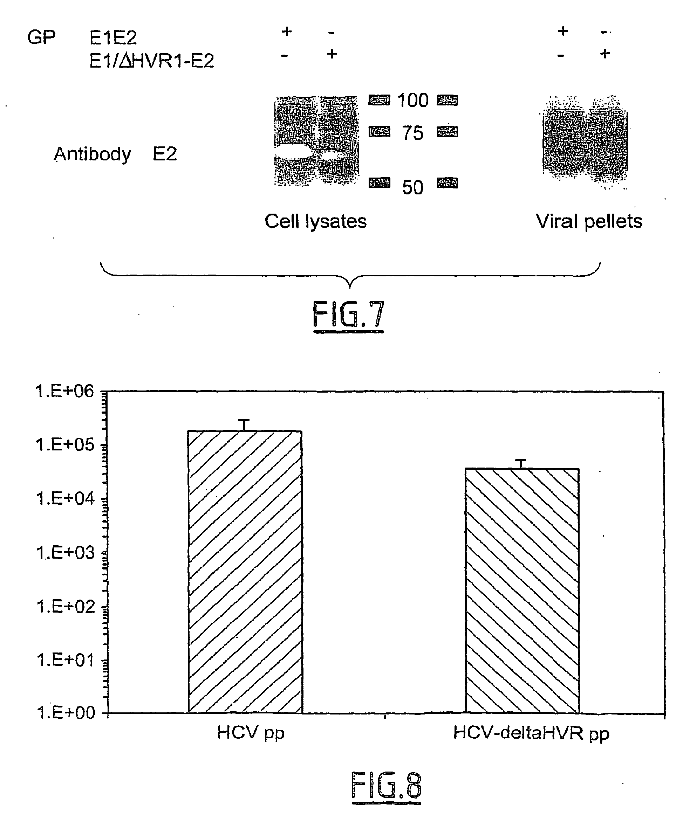

Figure 7 shows incorporation of ΔHVR1 HCV pseudo-particles. Immunoblots of lysates of 293 T transfected cells and of pseudo-particles pelleted through 20 % sucrose-cushions are shown. The positions of the molecular weight markers are shown (kDa).
Figure 8 represents the infectivity of ΔHVR1 HCV pseudo-particles. Supernatants from producer cells were diluted and used to infect Huh-7 target cells before assessing transduction efficiency three days post-infection by FACS analysis.

### EXAMPLES :

### EXAMPLE 1 : Generation of HCV pseudo-particles (HCVpp)

HCV pseudo-particles (HCVpp) were generated by assembling the full-length, unmodified E1 glycoprotein and the C-terminally truncated form of E2 protein (deletion of the C-terminal Ala 746 residue, SEQ ID N°16) onto retroviral core proteins derived from murine leukemia virus (MLV). To investigate further whether functional HCVpp could also be produced with E1 and E2 expressed in *trans* or with only one of the two glycoproteins, expression vectors that encoded individually either E1 or E2 glycoproteins were designed.

### Construction of expression vectors encoding the viral components, i.e., E1, E2, or E1E2 glycoproteins and viral core proteins

Plasmids expressing wild type E1 E2 polyproteins were constructed by standard methods (Sambrook et *al*., 1989).

Briefly, the phCMV-7a expression vector encoding the E1 and E2 glycoproteins from a 1a type HCV was generated by inserting the blunted *Cl*al and Stul restriction fragment encoding the last 60 residues of HCV core (C) and all of E1 and E2 proteins from the pTM1p5E1E2(745) vector (Op De Beeck *et al.,* 2000) into the *Bam*HI digested and Klenow blunted vector phCMV-G (Nègre *et al.,* 2000).

The phCMV-E1 expression vector, expressing only HCV E1 glycoprotein, was derived from phCMV-7a by adding a stop codon to the C-terminus of E1 with primers 5'-actggacgacgcaaagctgc (SEQ ID N°2) and 5'-cgcggatcctacgcgtcgacgccggcaaa (SEQ ID -N°3). The resulting PCR fragment was digested with *Bam*HI and ligated into *Bam*HI-digested phCMV-7a.

phCMV-E2, expressing only HCV E2 glycoprotein, was obtained by fusing the N-terminus of E2 to the C-terminus of the HCV core using two PCR fragments generated with the two primer pairs 5'-tgcccgcttcagccgaaacccacgtcaccggggga (SEQ ID N°4) + 5'-gccagaagtcagatgctcaagg (SEQ ID N°5) and 5'-tactctgagtccaaaccg (SEQ ID N°6) + 5'-gtgacgtgggtttcggctgaagcgggcacagtcag (SEQ ID N°7). The two PCR fragments were then fused in a second round PCR. The resulting DNA fragment was digested with *Bam*HI and ligated into *Bam*HI cut phCMV-7a.

The sequence of the phCMV-ΔC E1 vector is shown in SEQ ID No 8, whereas the aminoacid sequence of E1 protein is shown in SEQ ID No 9. The sequence of the phCMV-ΔC E1E2 vector is shown in SEQ ID No 10, whereas the aminoacid sequence if the E1 E2 polyprotein is shown in SEQ ID No 11. The sequence of the phCMV-ΔC E2 vector is shown in SEQ ID No 12, whereas the aminoacid sequence of E2 protein is shown in SEQ ID No 13.

Expression vectors for E1 E2 glycoproteins of 1b genotype were constructed by similar strategies.

HCV E1 E2 were therefore expressed from a polyprotein containing the carboxy-terminus of the core (ΔC) protein, which served as signal peptide for E1, E2 or E1 and E2 glycoproteins.

### Generation of HCV pseudo-particles

Retroviruses were chosen as platforms for assembly of HCVpp because their cores can incorporate a variety of different cellular and viral glycoproteins and because they can easily package and integrate genetic markers into host cell DNA.

HCVpp were produced by transfecting 293T human embryo kidney cells (ATCC CRL-1573) with three expression vectors encoding a ΔCE1E2, ΔCE1 or ΔCE2 polyprotein, the MLV core proteins and a packaging-competent MLV-derived genome harbouring the GFP (green fluorescent protein) marker gene. This construct contains the GFP marker gene, whose expression is driven by the CMV (cytomegalovirus) immediate early promoter. Both CMV and GFP nucleic acid sequences where inserted in a retroviral vector derived from MLV in which the gag, pol and env viral gene where removed and in which the retroviral cis-acting elements that control vector genome packaging, reverse transcription and integration were retained.

Control pseudo-particles were generated with the VSV-G glycoprotein (Nègre *et al.,* 2000), the RD114 virus envelope glycoprotein (Sandrin *et al.,* 2002), and/or with assembly-defective MLV core proteins (MLV-G2A) (Swanstrom et *al*., 1997).

Briefly, expression constructs were transfected into 2.5x10⁶ 293T cells seeded the day before in 10 cm plates using a calcium-phosphate transfection protocol (Clontech, France) according to the manufacturer's recommendations. The medium (8 ml/plate) was replaced 16 hrs after transfection. Supernatants containing the pseudo-particles were harvested 24 hrs later, filtered through 0.45µm-pore-sized membranes and processed as described before (Nègre *et al.,* 2000).

### Immunoblot analysis of structural components of the pseudo-particles

Lysates of transfected cells and of pseudo-particles pelleted through 20% sucrose-cushions were immunobloted. Expression of E1 and E2 glycoproteins from HCV-1a genotype and of MLV core proteins was revealed in reducing and denaturing conditions with monoclonal antibodies against E1 (A4) (Dubuisson et *al*., 1994) and E2 (H52) (Flint *et al.,* 1999) or with an anti-capsid (MLV CA) antiserum (ViroMed Biosafety Laboratories, USA), as described previously (Sandrin *et al.,* 2002). VSV-G expressed in control pseudo-particles was detected with the monoclonal antibody P5D4 (Sigma-Aldrich, France).

Analysis of immunoblots of transfected cells showed that the structural components of the pseudo-particles were readily detected at the expected molecular weights; *i*.*e*. 30 kDa for E1, 60 kDa for E2, 60 kDa for VSV-G and 70 kDa for the RD114 glycoprotein. MLV core proteins were detected as a Gag protein precusor of 65 kDa that was partially processed by the MLV protease into mature core components.

The E1 and E2 glycoproteins were readily detected in the pellets of purified virions generated with the wild-type MLV core particles but not with the viral assembly-deficient MLV-G2A mutant. E2 present within the viral pellets migrated slightly slower than the cell-associated forms, due to modifications of the associated glycans by Golgi enzymes.

The presence of VSV-G in viral pellets generated with MLV-G2A assembly-defective core proteins is due to empty vesicles formed by VSV-G itself (Nègre *et al.,* 2000).

Comparison of the relative levels of VSV-G or HCV glycoproteins in producer cell lysates versus viral pellets suggests efficient incorporation of E1 and E2 into viral particles. Likewise, could E1 and E2 glycoproteins efficiently assemble on retroviral core proteins derived from HIV-1, raising the possibility of pathogenic interactions between the two parental viruses, *in vivo,* as co-infection of patients with HCV and HIV is prevalent (Dieterich, 2002).

Altogether, these results indicate that transient over-expression of E1 and E2 in 293T cells leads to specific and efficient incorporation of HCV glycoproteins into pseudo-particles generated with retroviral cores. Since HCV envelope glycoproteins have been shown to be retained in the ER (Op De Beeck et al. 2001), this implies that HCVpp form by budding into the ER lumen or, alternatively, that saturation/leakiness of the ER retention allows a fraction of E1 E2 to reach the cell surface where MLV budding normally occurs (Swanstrom and Wills, 1997).

Individual expression or co-expression in *trans* of E1 and E2, from distinct expression units, led to normal levels of synthesis, as compared to expression of both glycoproteins in *cis*, from a single E1E2 polyprotein. Moreover, HCVpp were found to incorporate similar levels of E2 glycoprotein, whether it was expressed alone, or co-expressed in *cis* or in *trans* with E1. Finally, incorporation of E1 expressed alone or co-expressed in *trans,* with E2, occurred but at reduced levels, consistent with the chaperone activity of E2 (Op De Beeck et *al*., 2001). Formation of HCVpp carrying E1 or E2 only will allow investigation of their respective roles in HCV cell entry and infectivity.

### EXAMPLE 2 : HCVpp cell line infectivity

Infectivity of HCVpp was assessed on a panel of target cell lines : Huh-7 human hepatocellular carcinoma (Nakabayashi et *al*., 1982), PLC/PRF/5 human hepatoma (CRL-8024), Hep3B human hepatocellular carcinoma (ATCC HB-8064), HepG2 human hepatocellular carcinoma (HB-8065), A431 human epidermoid carcinoma (CRL-1555), Caco-2 human colon adenocarcinoma (HTB-37), HCT 116 human colorectal carcinoma (CCL-247), HOS human osteosarcoma (CRL-1543), HT-1080 human fibrosarcoma (CCL-121), HT-29 human colorectal adenocarcinoma (HTB-38), LoVo human colorectal adenocarcinoma (CCL-229), MCF-7 human breast adenocarcinoma (HTB-22), 293T, TE671 human rhabdomyosarcoma (ATCC CRL-8805), U118 human glioblastoma (HTB-15), Jurkat human T cell leukemia (TIB-152), CEM human lymphoblastic leukemia (CCL-119), Molt-4 human lymphoblastic leukemia (CRL-1582), Raji Burkitt's lymphoma (CCL-86), CMMT Rhesus monkey mammalian carcinoma (CRL-6299), COS-7 African green monkey fibroblasts kidney (CRL-1651), VERO African green monkey kidney (CCL-81), PG-4 feline astrocyte (CRL-2032), BHK-21 golden hamster kidney (CCL-10), CHO Chinese hamster ovary (ATCC CCL-61), BRL 3A rat hepatocytes (CRL-1442), NIH3T3 mouse fibroblasts (CRL-1658) and QT6 quail fibrosarcoma (CRL-1708). Target cells were grown as recommended by the ATCC (American Type Culture Collection, Rockville, MD, USA)..

Target cells (seeded the day before 8x10⁴ cells/well in 12-well plates) were incubated for 3 hrs with dilutions of supernatants from producer cells containing the HCVpp, then washed and cultured until expression of the GFP marker gene harboured by the virions was assessed by FACS analysis 72 hrs later (Sandrin *et al*., 2002). Since the HCVpp were generated with replication-defective viral components, this procedure allowed evaluation of the specific infectivity of the pseudo-particles after a one-round infection process.

Infectious titres of up to 1.1×10⁵ TU/ml were detected for the HCVpp on Huh-7 human hepato-carcinoma cells (Figure 1). Upon concentration of the producer cell supernatants by ultra-centrifugation (Sandrin *et al.,* 2002), infectious titres of 2x10⁶ TU/ml, on average, could be readily obtained. The other target cell types used in the infection assays displayed weaker (PLC/PRF/5, Hep3B, HepG2, Caco-2, HT1080, HT-29, LoVo, MCF-7, U118, 293T, Vero) or undetectable (A431, HCT 116, HOS, TE671, Jurkat, Molt-4, CEM, Raji, CMMT, Cos-7, BHK-21, CHO, PG-4, BRL 3A, NIH3T3, QT6) levels of infection with the HCVpp. The infectivity of control pseudo-particles generated with VSV-G ranged from 7x10⁶ to 2x10⁷ TU/ml depending on the target cell type indicating that all these cells were readily infected with the control pseudo-particles. This suggested that all the molecules necessary for HCV entry were specifically expressed in the former cell types only.

Infectious HCVpp could be generated at comparable efficiencies with E1E2 glycoproteins derived from HCV genotypes 1a and 1 b (lanes h and i; Figure 2) and/or with retroviral core proteins derived from either HIV-1 or MLV (lanes d and h; Figure 2).

Incubation of HCVpp and target cells with AZT, a reverse-transcriptase inhibitor that prevents conversion of the retroviral RNA genome of the HCVpp as integration-competent proviral DNA, inhibited transduction (lane j; Figure 2).

Moreover, long-term expression of GFP could be demonstrated after serial passaging of the infected cells for more than one month.

These results indicated that infection of the target cells led to integration in host cell DNA and stable expression of the GFP marker gene transduced by the HCVpp. Additionally, no infection could be obtained with viral particles lacking both E1 and E2 glycoproteins, or lacking core proteins, or, alternatively, when using the MLV-G2A assembly-defective core proteins (lanes a-c; Figure 2).

The inventors have further demonstrated that hepatic cell infection could be achieved with HCVpp harbouring E1 and E2 glycoproteins of genotype 2a, 2b, 3a, 3b, 4a and 4b.

Altogether, these results support that HCVpp harbouring the E1 and E2 glycoproteins of any genotype and retroviral core proteins are infectious, leading to retroviral-mediated integration of their vector genome.

Finally, despite reduced levels of E1 incorporation, HCVpp generated with E1 and E2 glycoproteins expressed in *trans*, from distinct vectors, were nearly as infectious as those generated with the E1E2 polyprotein construct (lanes g and h; Figure 2). However, HCVpp assembled with either E1 or E2 glycoproteins only were 500-fold less infectious (lanes e-f; Figure 2), demonstrating that both glycoproteins need to be co-incorporated on the HCVpp to allow efficient virus entry and infection.

### EXAMPLE 3 : HCVpp primary hepatocyte infectivity

Hepatocytes represent the principal site of HCV replication *in vivo,* yet *ex vivo* studies have suggested that HCV may also infect lymphoid cells (Lindenbach and Rice, 2001). To address whether either cell types could be infected, human adult hepatocytes (Figure 3) and peripheral blood mononuclear cells (PBMCs) have been transduced. Pseudo-partictes were generated with core proteins derived from HIV-1 rather than from MLV, which do not permit transduction of non-proliferating target cells (Nègre *et al.,* 2000).

Cryopreserved primary hepatocytes, isolated from human adult biopsy samples checked for absence of HBV, HCV and HIV, were purchased from Biopredic International (Rennes, France) and cultured on collagen I coated plates according to recommendations of the supplier. Transduction of hepatocytes was determined by counting GFP-positive cells under UV microscope. Specificity of infection was demonstrated by absence of transduction when target cells were infected with pseudo-particles lacking glycoproteins (pp cores) or by the reduced levels of transduction when target cells were pre-incubated with JS-81 anti-CD81 antibodies (30 µg/ml) before infection. Infection of human PBMCs, isolated from healthy donors, was conducted as described previously (Sandrin *et al.,* 2002).

Relative to infection of Huh-7 cells, the HCVpp could readily infect the primary hepatocytes derived from different donors (Figure 3), yet transduction efficiencies varied with quality and cell culture viability of the individual biopsies. In contrast, poor or undetectable infection of PBMCs was found with the HCVpp although control pseudo-particles generated with VSV-G could readily infect these primary cells.

Altogether these data indicate that HCVpp closely mimic the tropism and early events of infection by wild-type HCV and preferentially infect hepatocytes and hepato-carcinoma cells.

### EXAMPLE 4 : HCVpp is a valid model of early steps of HCV infection

### Infectivity of HCVpp is mediated by E1 and E2 glycoproteins

It was further determined whether infectivity of the HCVpp is specifically mediated by E1 and E2 and their interaction with cell surface receptors. A panel of monoclonal antibodies previously shown to specifically react against HCV-1a glycoproteins (Dubuisson et *al.,* 1994; Flint et *al*., 1999; Patel et *al.,* 2000) was used.

HCVpp generated with HCV-1a E1E2 and with MLV core proteins were pre-incubated before infection of Huh-7 cells with saturating concentrations (20 µg/ml) of monoclonal antibodies against E1 (A4) or E2 (H31, H33, H35, H44, H48, H53, H54, H60 and H61) glycoproteins of genotype 1a, or with pooled antibodies (Hmix). Control experiments were performed using no antibodies or using pseudo-particles generated with VSV-G (VSV-Gpp).

Some of these monoclonal antibodies, like H35 and H48, for example, could neutralise the HCVpp of genotype 1 a and reduce their infectivity by up to 70% (Figure 4). That incomplete neutralisation might be due to the fact that these monoclonal antibodies, which were developed and selected for binding to intra-cellular E1 E2 complexes, have been shown to be sensitive to post-translational modifications of E2 (Flint M. et *al.,* 2000). Indeed, compared to its intra-cellular counterpart, E2 associated to HCVpp was found to have undergone sugar modifications, most likely as a result of its export through the cell secretory pathway.

In contrast, none of these genotype 1a-specific antibodies could neutralise the HCVpp of genotype 1 b or the control pseudo-particles generated with VSV-G (Figure 4). Neutralisation of the infectivity of these control pseudo-particles was achieved by using the VSV-G neutralising 41A.1 monoclonal antibody.

These data therefore demonstrate that infectivity of the pseudo-particles is specifically due to the incorporation of E1 and E2 glycoproteins, indicating that these HCVpp represent a valid model to investigate the early steps of HCV infection, *i*.*e*. receptor binding, membrane fusion and envelope uncoating.

### HCV infection is neutralised by serum from HCV-infected patients

The capacity of sera derived from chronically HCV-infected patients to neutralise infectivity of HCVpp was further assessed.

HCVpp of genotypes 1a or 1b were pre-incubated for 30 min at room temperature with sera from chronically HCV-infected patients diluted 1/50 before infection of Huh-7 target cells. Control experiments were performed using pseudo-particles generated with RD114 glycoproteins (RD114pp), rather than with VSV-G that exhibits sensitivity to human complement (Sandrin et *al.,* 2002). Efficient neutralisation of the control pseudo-particles was demonstrated using a hyper-immune goat serum raised against the RD114 SU glycoprotein (ViroMed Biosafety Laboratories, USA).

No or only weak neutralisation of control pseudo-particles could be detected using the sera of the HCV-infected patients. In contrast, most if not all of these sera could neutralise the infectivity of the HCVpp, in contrast to sera derived from healthy donors. Variable levels of neutralisation were detected depending on the donor and ranged from 20% to up to 90% of inhibition for HCVpp of both genotypes 1 a and 1b. Sera derived from patients infected with HCV of the 1 b genotype could neutralise the HCVpp generated with E1 E2 of genotype 1 a or 1 b with similar efficiencies (Figure 5).

### EXAMPLE 5 : Cell entry receptor usage by HCV pseudo-particles

Both the LDL receptor (LDLr) and CD81, a member of the tetraspanin family of receptors, have been proposed as putative HCV receptors (Pileri et *al*., 1998; Agnello et *al*., 1999). However, recent studies have questioned the role of these molecules as cell entry receptors despite their unequivocal capacity to bind HCV particles and/or glycoproteins (2023). Thus, the contribution of either cell surface molecules in the early stages of HCV cell entry was investigated by performing receptor-competition assays.

More recently, SR-B1 the human scavenger receptor class B type I expressed in steroidogenic tissues has been put forward as another putative HCV receptor (Scarselli, Ansuini et al. 2002). Thus, the contribution of SR-BI in the early stages of HCV cell entry was investigated by performing infection assays in non-permissive target cells engineered to express this HCV receptor candidate.

### LDLr

LDLr binds apoliprotein B within LDL and apoliproteins B and E (ApoB and ApoE) within VLDL complexes; both complexes have been found associated with HCV particles in plasma of infected patients (Andre et *al*., 2002).

LDL receptor competition assays were performed using purified LDLs (10 µg/ml, 100 µg/ml; Sigma-Aldrich, France) or with the monoclonal antibodies 5E11 (10 µg/ml, 50 µg/ml), 4G3 (anti-ApoB; 10 µg/ml, 50 µg/ml) or 1 B7 (anti-ApoE; 1 µg/ml ,10 µg/ml, 50 µg/ml, 100 µg/ml) (Ottawa Heart Institute Research corporation, Ottawa, Ontario, Canada), or mixed 5E11, 4G3 and 1B7 antibodies (with individual concentrations 10 µg/ml, 50 µg/ml), pre-incubated with the pseudo-particles prior to infection.

Purified LDLs were not found to out-compete infection of HCVpp on Huh-7 LDLr-positive cells, even when concentrations higher than 100 µg/ml were used. When monoclonal antibodies targeted to the receptor binding sites of ApoB and ApoE were pre-incubated with the HCVpp, only the anti-ApoE antibodies could inhibit, albeit weakly, the infectivity of the HCVpp. This is consistent with our observation that HCVpp could not or hardly infect LDLr-positive cell lines such as HepG2, Jurkat, CEM, Molt-4, Raji and TE671 (Figure 1). Thus, although these results do not exclude a role of LDLr in HCV entry, possibly *via* association of LDLs with HCV particles (Andre et *al*., 2002), they suggest that LDLr is not the major receptor of HCVpp entry.

### CD81

Recombinant GST-fusion polypeptides encompassing the large extra-cellular loop (LEL) of human CD81 which has been shown to bind HCV E2 (Flint *et al.,* 1999, (Pileri et *al.*, 1998) were then used to investigate the role of hCD81 in HCVpp cell entry.

hCD81-LEL GST-fusion polypeptides (4 µg/ml, 8 µg/ml, 16 µg/ml) were pre-incubated with the pseudo-particles before infection or JS-81 anti-CD81 antibodies (4 µg/ml, 10 µg/ml, 30 µg/ml; Pharmingen, France) were pre-incubated with the target cells prior to infection. Percentages of inhibition of the infectious titres obtained on Huh-7 cells relative to titres obtained in the absence of inhibitors were calculated. Control experiments were performed using pseudo-particles generated with VSV-G (VSV-Gpp). Comparative experiments were further conducted with NIH3T3, NIH3T3-hCD81 and Huh-7 cells infected with HCVpp and with control pseudo-particles generated with VSV-G.

The GST-fusion hCD81-LEL polypeptides pre-incubated with the HCVpp could specifically precipitate E1 E2 complexes, confirming their capacity to bind the E2 glycoprotein (Flint *et al*., 1999; Pileri et *al*., 1998), and were found to neutralise the infectivity of the HCVpp on Huh-7 cells, yet with a relatively poor efficiency. Inhibition ranged from 38% to 54% inhibition of infectivity and appeared to be dose-dependent.

Consistently, pre-incubation of Huh-7 cells with JS-81 monoclonal antibody, that blocks binding of recombinant E2 to hCD81 (Flint *et al.,* 1999), was found to inhibit infectivity of HCVpp in both Huh-7 target cells and human primary hepatocytes. At high antibody concentrations (30 µg/ml), over 90% inhibition of infection could be obtained for HCVpp based on genotypes 1a as well as 1b. However, several target cells non-permissive to infection by HCVpp, like TE671, Jurkat, CEM, Molt-4 and Raji cells (Figure 1) were found to express high densities of hCD81, indicating the lack of correlation between CD81 expression and infectivity.

Moreover, expression of hCD81 in non-permissive NIH3T3 mouse fibroblasts did not allow infection with the HCVpp (Figure 4C).

Altogether these results demonstrate that although hCD81 binds HCV E2 and might help cell surface attachment of HCV, it is not sufficient by itself to allow infection with HCVpp.

### SR-BI

A cDNA encoding SR-BI, also known as CLA-1 (CD36 and LIMPII Analogous-1) (Calvo and Vega, 1993; Webb et al., 1998) was introduced in an MLV retroviral vector and vector particles were generated and pseudotyped with the VSV-G glycoprotein. They were used to transduce CHO and 3T3 cells, that are non permissive to HCVpp infection, as shown in Figure 1. Expression of SR-BI in these transduced cells was verified by FACS analysis using antibodies, directed against the SR-BI ectodomain. The SR-BI-transduced cells as well as the parental cells were used as target cells for infection assays using the HCVpp. Huh7 cells were used as control permissive target cells.

In contrast to the Huh-7 cells, neither the SR-BI-transduced cells nor the parental CHO or 3T3 cells could be infected with HCVpp. These data indicates that although SR-BI binds E2 (Scarselli et al., 2002), it is not sufficient to allow entry of HCVpp in cells.

Therefore, as for hLDLR and hCD81, expression of SR-BI expression alone is not enough to render cell permissive to infection.

To address the possibility that HCV target cells need to co-express all molecules to allow HCVpp infection, CHO and 293 target cells, that respectively have no or low SR-B1 expression and that are both non permissive to infection with the HCVpp (Bartosch et al., 2003 a and b), were co-infected with two VSV-G-pseudotyped retroviral vectors carrying human CD81 and SR-BI, respectively. Co-expression of both cell suface molecules on either cell type was verified two days after transduction, by FACS analysis.

The CD81 and SR-BI co-expressing CHO or 293 cells were then used as target cells for infection assays using the HCVpp. Similar to parental CHO cells and to the same cells individually transduced by either CD81- or SR-BI-expression retroviral vectors, this resulted in absence of infectivity. In contrast to parental 293 cells as well as to CHO-CD81/SR-B1 cells, SR-B1-transfected 293 cells were found permissive to infection, indicating that co-expresssion of CD81 and SR-B1 is required for infection in human cells.

Since HepG2 human hepato-carcinoma cells are non-permissive to infection by the HCVpp, the inventors further thought that their non-permissiveness could be due to lack of co-expression of both CD81 and SR-BI. Indeed, HepG2 cells express SR-BI but lack CD81 expression.

Thus the HepG2 cells were transduced with a CD81-carrying MLV retroviral vector pseudotyped with VSV-G. CD81-expression was verified by FACS analysis two days after transduction and the CD81-transduced HepG2 cells were then used as target for infection assays using the HCVpp.

Compared to the parental HepG2 cells which were poorly infected with the HCVpp, ectopic expression of CD81 in these cells resulted in high infectivity of the HCVpp, similar to that observed in Huh-7 cells.

### EXAMPLE 6 : Deletion of hypervariable region I

HCV infection is not cleared by over 80% of patients and chronicity is associated with various forms of liver disease. Chronic infection is thought to be caused by the high mutation rate of HCV, which helps the virus to escape the host immune response. One of several mutation hotspots within the HCV genome has been localised to the N-terminus of the E2 glycoprotein. This so-called hypervariable region I is a major B cell epitope and a target of neutralising antibodies in vivo. Indeed HCVpp can be very efficiently neutralised by mouse monoclonal antibodies or by rabbit hyper-immune sera raised against HVR1 (Bartosch et al., 2003b). However, such antibodies are highly specific for the homologous sequence and do not neutralize slightly divergent HVR1 variants of the virus as well as ΔHVR1 HCVpp (Bartosch et al., 2003b). Thus, because of its variability, HVR1 is thought to play an important role in allowing HCV to escape the host's immune response. The antigenic variation of the HVR1 may also inhibit the development of a protective immune response against re-infection with heterologous HCV strains/genotypes. Finally does the antigenic dominance and extreme variability of the HVR1 hinder the development of vaccines against HCV. With the long-term aim to develop effective therapeutics which target more conserved parts of the HCV glycoproteins the inventors investigated whether the HVR1 is dispensable for infection.

For that purpose expression constructs encoding the polyprotein for wildtype E1E2 or a mutant version in which the HVR1 = the first 27 residues of E2 had been deleted from the polyprotein were generated. HCV pseudo-particles (HCVpp) were produced with either wild type or ΔHVR1 ΔCE1E2 polyprotein. Analysis of immunoblots of transfected cells showed that both wildtype and mutant E2 proteins were expressed to similar levels. Viral particles were harvested from the supernatant of transfected cells and purified by ultra-centrifugation through high-density sucrose cushions. ΔHVR1 and wild-type E2 were incorporated into particles to a similar level (Fig. 7). The infectivity of the wildtype and mutant HCVpp were tested as before on Huh-7 target cells. Infectious titres of up to 2.6x10⁵ TU/ml were detected for wildtype HCVpp (Fig. 8). ΔHVR1 HCVpp were about 5 to 10 fold less infectious, still a maximal titer of 5.1x10⁴ TU/ml was observed suggesting that the HVR1 aids the infection process, via interaction to SR-B1 receptor (Scarselli et al., 2002: Bartosch et al., 2003b).

ΔHVR1 HCVpp will be useful not only to induce antibodies against more conserved neutralising epitopes but also as a tool to detect neutralising epitopes that are target of the humoral response induced by HCV infection. The humoral response in sera from some HCV-infected patients and from experimentally inoculated chimpanzees has been characterised (Bartosch et al., 2003c). Neutralizing antibodies from chronic infections were relatively high-titered against the homologous and a heterologous genotype 1a pp and usually, but not always, cross-neutralized a pseudotyped virus of a different subgenotype. To determine if the pedigreed sera tested herein reacted with epitopes outside of the HVR1 region of the HCV envelope proteins, the panel of sera was tested at a dilution of 1:50 against the ΔHVR1 HCVpp. All of the sera that neutralized parental HCVpp also neutralized ΔHVR1 HCVpp. Thus, neutralizing antibodies from patients and chimpanzees persistently infected with HCV reacted with one or more additional epitopes elsewhere in the HCV envelope glycoproteins. The observation that chronically infected chimpanzees and humans do make neutralizing anti-HCV antibody and that this antibody is broadly cross-reactive, is consistent with the observation that immune globulin prepared from such chronically infected patients can protect against HCV infection. This has important implications for rethinking the feasibility of developing antibody-based passive and active immunophophylaxis strategies against HCV as well as for reassessing the relative importance of humoral and cellular immunity in controlling chronic HCV infections.

### Example 7 : HCVpp and cell-cell fusion assays

Cell-cell fusion assays were designed by co-culturing E1 E2-transfected 293T (donor) cells with target (effector) cells. The effector cells were hepato-carcinoma cells or, as control, 293T cells.

Transformed cells, transfected with the constructs expressing the envelope glycoproteins, are detached, counted and re-seeded at the same concentration (3x10⁵ cells/well) in six-well plates. Fresh target host cells (1x10⁶ cells per well) are then added onto the transfected cells and are cocultivated for 24 hours.

The coculture is submitted to a acidic pH drop by replacing the culture medium with a pH5-buffered DMEM for 5 min and incubating for 5 min Acidic medium was then replaced with a normal (neutral pH medium) and cultures were grown for 12 hours until cell-cell fusion was evaluated by scoring the syncytia.

Cultures are then stained by adding the May-Grunwald and Giemsa solutions (MERCK) according to the manufacturer recommendations. Cells containing two or more nuclei can be defined as syncytia. A fusion index is then defined as the percentage of (N-S)/T where N is the number of nuclei in the syncytia, S is the number of syncytia and T is the total number of nuclei counted.

The results of syncytia scoring are displayed in Table 1.

**Table 1. Results of syncytia assays with Huh-7 cells**

| Transfected glycoprotein | Syncytia at neutral pH¹ | Syncytia after pH-5 shock¹ |
|---|---|---|
| None | - | - |
| MLV-A | - | - |
| MLV-A-Rless | ++ | ++ |
| FPV-HA | - | ++ |
| VSV-G | + | +++ |
| HCV E1 | - | - |
| HCV E2 | - | - |
| HCV E1E2 | - | ++² |

| | | |
|---|---|---|
| ¹(-), fusion index of less than 1%, (+),fusion index between 1% and 5%; (++),fusion index between 5% and 20%; (+++),fusion index higher than 20%. ²no syncytia were detected when HCV E1E2-transfected cells were co-cultivated with 293 cells after a pH-5 shock | | |

Expression of control amphotropic murine leukaemia virus (MLV-A) pH-independent glycoproteins did not result in syncytia in these experimental conditions. However, extensive syncytia formation was detected when using a mutant form of MLV-A glycoprotein with shortened cytoplasmic tail (MLV-A-Rless).

Expression of pH-dependent fowl plague virus hemagglutinin (FPV-HA) resulted in syncytia formation only when the co-cultures were incubated for 5 min at pH-5.

Expression of the pH-dependent G glycoprotein of VSV (vesicular stomatitis virus) resulted in small syncytia formation at neutral pH and in extensive syncytia formation at acid pH.

Although expression of HCV E1 or HCV E2 alone did not results in syncytia under either experimental conditions, co-expression of both E1 and E2 in 293T cells co-cultivated with Huh-7 cells was sufficient to induce the formation of syncytia. This indicates that under such experimental conditions, HCV E1 E2 can fuse cells expressing the appropriate HCV receptors.

Moreover these results indicate that unlike most retroviral glycoproteins (*eg*., MLV-A), but like influenza virus hemagglutinin (*eg*., FPV-HA) or VSV-G, fusogenicity of HCV E1E2 is triggered by acid pH. These findings are confirmed by the sensitivity of the HCV pseudo-particles to inhibitors of endosomal acidifications (Table 2).

**Table 2. pH-sensitivity of infection by HCV pseudo-particles**

| Glycoprotein¹ | Bafilomycin-A concentration (nM)² | | | |
|---|---|---|---|---|
| | 0 | 50 | 100 | 200 |
| MLV-A | 100 | 91.2 | 70 | 45 |
| FPV-HA | 100 | 12.8 | 2.3 | 2.7 |
| VSV-G | 100 | 27 | 7.3 | 0.5 |
| HCV-E1E2 | 100 | 12.8 | 4.3 | 3 |

| | | | | |
|---|---|---|---|---|
| ¹GFP-carrying pseudo-particles generated with the indicated glycoprotein were used to infect Huh-7 cells in the presence of the indicated concentration of Bafilomycin A. The inhibitor was removed after 3 hr and infectivity was determined by FACS analysis 3 days later. ²results of infection for the indicated concentration of Bafilomycin-A are expressed as percentage of the infectivity determined in the absence of inhibitor. | | | | |

Thus, this novel cell-cell HCV fusion assay is highly valuable for the screening of molecules capable to inhibit HCV binding and cell entry by membrane fusion.

### Example 8: Assembly and increased infectivity of HCV pseudo-particles in the presence of HCV p7

The p7 protein may influence the conformational changes of the HCV envelope proteins that are perhaps important for cell binding and entry of the HCV virion. Accordingly, to gain more information on the p7 protein, expression constructs expressing a E1 E2p7 polyprotein were generated.

The phCMV-ΔCE1E2p7 (phCMV 7a p7-1) expression vector expressing HCV 1 a proteins delta core, E1, E2 and p7 was constructed using the Stu I, Bcl I digested fragment from plasmid pTM1p5E1E2p7 (Fournillier-Jacob et al, 1996; Cocquerel et al, 2000), comprising E1, E2 and p7 of HCV genotype 1a. This fragment was ligated into the previously described plasmid phCMV-7a after Bsu36 I digestion, blunting and subsequent digestion with Bcl I. The sequence of the phCMV-ΔCE1E2 vector is shown in SEQ ID No 14, whereas the aminoacid sequence of the ΔCE1E2 polyprotein is shown in SEQ ID No 15.

Infectivity of the HCV pseudo-particles generated with p7 (HCV-p7pp) was compared with that of HCVpp. Expression of p7 during assembly of the HCV pseudo-particles resulted in up to 10-fold increased titres. Similar increase of infectious titres mediated by p7 expression were obtained for HCVpp assembled with E1 E2 glycoproteins derived from HCV genotypes 1 a and 1b.

### Example 9: Deletion of the C-terminal amino acid residue of mature E2 glycoprotein

The fragment of the HCV polyprotein used to express the ΔCE1E2 polypeptide is usually numbered relative to the total HCV polyprotein. For the 1a strain H HCV genotype (accession number in GeneBank: AF009606), ΔC is therefore encompassed by amino-acids 132-191 (signal peptide: amino-acids 171-191), E1 by amino-acids 192-383, and E2 by amino-acids 384-746 (HVR1: amino-acids:384-410).

The inventors have co-expressed this full length polypeptide, from amino-acids 132 to 746 (with a short sequence, MNS, fused to the amino-terminus of ΔC and that is encoded by the translation initiation site of the ΔCE1E2 polypeptide gene) with the other components required to assemble infectious HCV pseudo-particles. HCV pseudo-particles can also be assembled with a shorter version of the ΔCE1E2 polypeptides, encompassing amino-acids 132-745 (and the MNS amino-terminal peptide).

Both types of HCV pseudo-particles, ie. those carrying the full length E1E2 glycoproteins (HCVpp(746)) and those carrying E1E2 glycoproteins deleted of the last amino-acid at position 746, an alanine, (HCVpp(745)) incorporate normal level of glycoproteins. However, the HCVpp(745) pseudo-particles are consistently 10-50 times more infectious than the HCVpp(746) pseudo-particles. This improvement in infectivity is therefore important in infection assays as well as in screening of neutralising compounds and antibodies.

### Example 10: Mice immunization with HCVpp elicit neutralizing antibodies

Balb/c mice, aged 4-5 weeks, in groups of 4 animals, received intraperitoneal injections of purified HCVpp harbouring E1E2 glycoproteins of genotype 1 a, 1 b, 2a or HCVpp Δ-HVR1 Δ-CE1E2, as described in example 6, or as control, with pseudo-particles devoid of viral surface glycoproteins or carrying a non-relevant glycoprotein, from RD114 feline endogenous virus (Bartosch et al., 2003a). About 1e6 purified HCVpp, corresponding roughly to 1e9 physical particles, were used per mice. Each mouse was boosted two times with identical inoculae. The sera of the animals were harvested and their neutralising activity was compared to that of control sera, harvested from the same animals before immunisation, in infection assays using the HCVpp.

Neither these control sera nor the sera from animals immunised with the control pseudo-particles could neutralise the infectivity of the different types of HCV pseudo-particles. In contrast, the sera of mice immunised with the HCV pseudo-particles could efficiently neutralise the infectivity of both the HCV pseudo-particle type matching that used for immunisation and the other types of HCV pseudo-particles. This resulted in over 90 % inhibition of infectivity when sera of mice immunised with the HCVpp were diluted to 1/80 before incubation with the HCVpp.

These data indicated that HCV pseudo-particles are useful to elicit the formation of neutralising antibodies as well as cross-neutralising antibodies. The ability to induce neutralizing anti-HCV antibodies should permit an assessment of the prospects for successful antibody-mediated passive and active immunoprophylaxis against hepatitis C.

In conclusion, the invention provides a tool that allows precise investigation of viral assembly of E1E2 glycoproteins (processing, maturation) and their role in cell entry of HCV. No structural modifications of the E 1 E2 glycoproteins were required for their correct assembly on retroviral and lentiviral cores and to generate high titer infectious HCVpp with functional E1 E2 glycoproteins. Deletion of the C-terminal residue of HCV E2 protein was further found to greatly enhance infectivity of the generated HCV pseudo-particles. The insertion of a marker gene into the HCVpp allowed precise and rapid determination of the infectivity of these pseudo-particles by flow-cytometry.

The development of these functional, infectious HCV pseudo-particles make it now possible to investigate early events of HCV infection, such as the identification of novel HCV receptor(s) or co-receptor(s), to carry out diagnostic assays for the detection of neutralising antibodies in seroconverted patients, and to develop efficient inhibitors to HCV infection.

### REFERENCES

Agnello V. et al., Proc Natl Acad Sci U S A 96, 12766-71. (1999).
Andre P. et al., J Virol 76, 6919-28. (2002).
Ausubel F.M. et al. (eds.), Current Protocols in Molecular Biology, John Wiley & Sons, Inc. (1994).
Bartosch, B., Dubuisson J. & Cosset, F.-L. 2003a. J. Exp. Med. 197, 633-642.
Bartosch et al., J. Biol. Chem. 2003b Aug 11 [Epub ahead of print].
Bartosch et al., 2003c. Submitted.
Baumert T. F., Ito S., Wong D. T., Liang T. J., J Virol 72, 3827-36. (1998).
Blight K. J., Kolykhalov A. A., Rice C. M., Science 290, 1972-4. (2000).
Buonocore L., Blight K. J., Rice C. M., Rose J. K., J Virol 76, 6865-72. (2002).
Calvo and Vega (1993) J. Biol. Chem. 268:18929-18935
Castet V, Moradpour D. Hepatology. 2003 Sep;38(3):771-4.
Cocquerel et al., J Virol, 74/8, 3623-3633 (2000)
Dieterich (2002) J Infect Dis 185, S128-37.
Dubuisson J. et al., J Virol 68, 6147-60. (1994).
Felgner et al. Science 337, 387-388. (1989).
Flint M. et al., J Virol 73, 6235-44. (1999).
Flint et al., J Virol 74, 702-709 (2000).
Fournillier-Jacob et al., J Gen Virol, 77, 1055-1064 (1996)
Guguen-Guillouzo and Guillouzo. Methods for preparation of adult and fetal hepatocytes. p 1-12. In A. Guillouzo and C. Guguen-Guillouzo (ed.), Isolated and cultured hepatocytes. Les Editions INSERM Paris. John Libbey and Co, Ltd., London, United Kingdom (1986).
Hsu, M., Zhang, J., Flint, M., Logvinoff, C., Cheng-Mayer, C., Rice, C. M. & McKeating, J. A. (2003) Proc. Natl. Acad. Sci. USA 100, 7271-7276.
Lavanchy D. et al., J. Viral Hepat 6, 35-47 (1999).
Lindenbach B. D., Rice C. M., Flaviviridae: The Viruses and Their Replication. Knipe D. M., Howley P. M., Eds., Fields Virology, 4th ed. (Lippincott Williams & Wilkins, Philadelphia, Pa, 2001).
Lohmann V. et al., Science 285, 110-3. (1999).
Matsuura Y. et al., Virology 286, 263-75. (2001).
Nakabayashi H. et al., Cancer Res 42, 3858-63. (1982).
Nègre D. et al., Gene Ther 7, 1613-1623 (2000).
Op De Beeck A. et al., J Biol Chem 275, 31428-37. (2000).
Op De Beeck A., L. Cocquerel, J. Dubuisson, J Gen Virol 82, 2589-95. (2001).
Owsianka A. et al., J Gen Virol 82, 1877-83. (2001).
Paetzel et al. (2002) ).Chem Rev. Dec;102(12):4549-80
Patel A. H. et al., J Gen Virol 81, 2873-83. (2000).
Pietschmann T. et al., J Virol 76, 4008-21. (2002).
Pilei P. et al., Science 282, 938-41. (1998).
Robertson et al. (1998) Archives of Virology 143, 2493-2503
Rose N. F. et al., Cell 106, 539-49. (2001).
Sambrook, J., Fritsch, E. F., and Maniatis, T. Molecular Cloning: A Laboratory Manual , 2nd Ed. , Cold Spring Harbor Laboratory, Cold Spring Harbor, NY (1989).
Sandrin V. et al., Blood 100, 823-832 (2002).
Scarselli, E. et al., EMBO J. 21: 5017-25 (2002).
Swanstrom R., Wills J. W., in Retroviruses J. M. Coffin, S. H. Hughes, H. E. Varmus, Eds. (Cold Spring Harbor Laboratory Press, New York, USA, 1997) pp. 263-334.
von Heijne G. (1990) J Membr Biol. 115(3):195-201
Wellnitz S. et al., J Virol 76, 1181-93. (2002).
Webb et al., J. Biol. Chem., 273:15241-15248. (1998)
Wu et al., J. Biol. Chem. 263:14621-14624. (1988)
Wu et al., J. Biol. Chem. 267:963-967. (1992)
Yagnik et al. (2000) Proteins 40, 355-366

## Claims

1. A method for producing infectious hepacivirus-like particles ex *vivo* comprising the steps of:
- providing a first nucleic acid sequence comprising a packaging competent retroviral-derived genome;
- providing a second nucleic acid sequence comprising a cDNA encoding core proteins from said retrovirus;
- providing a third nucleic acid sequence comprising a cDNA encoding a polyprotein comprising successively a signal peptide from a type I membrane protein, and a hepacivirus or GB virus E1 protein and/or a hepacivirus or GB virus E2 protein, wherein the C-terminal transmembrane domain of the E1 and E2 proteins is unmodified with respect to native E1 and E2 proteins ;
- transfecting host cells with said nucleic acid sequences and maintaining the transfected cells in culture for sufficient time to allow expression of the cDNAs to produce structural proteins from hepacivirus or GB virus and retrovirus; and allowing the structural proteins to form virus-like particles.

2. The method according to claim 1, wherein said third nucleic acid sequence comprises a cDNA encoding a polyprotein comprising successively a hepacivirus or GB virus core protein, and a hepacivirus or GB virus E1 protein and/or a hepacivirus or GB virus E2 protein.

3. The method according to claim 1 or 2, wherein said packaging competent retroviral-derived genome and core proteins are from a retrovirus selected from the group consisting of MLV, ALV, RSV, MPMV, HIV-1, HIV-2, SIV, EIAV, CAEV, or HFV.

4. The method according to claim 2 or 3, wherein said polyprotein comprises a hepacivirus or GB virus core protein and a hepacivirus or GB virus E1 protein.

5. The method according to any of claims 2 to 4, wherein said polyprotein comprises a hepacivirus or GB virus core protein and a hepacivirus or GB virus E2 protein.

6. The method according to any of claims 1 to 5, wherein said third nucleic acid sequence comprises a cDNA encoding a polyprotein that further comprises a hepacivirus or GB virus p7 protein.

7. The method according to any of claims 1 to 6, wherein said polyprotein comprises native hepacivirus or GB virus E1 and/or E2 protein, and optionally native hepacivirus or GB virus p7 protein.

8. The method according to any of claims 2 to 7, wherein said polyprotein comprises a native hepacivirus or GB virus core protein, a native hepacivirus or GB virus E1 protein and native hepacivirus or GB virus E2 protein, and optionally a native p7 protein.

9. The method according to any of claims 2 to 8, wherein core protein, E1 protein and E2 protein, and optionally p7 protein, are derived from a same hepacivirus or GB virus.

10. The method according to claim 9, wherein said hepacivirus or GB virus is a hepatitis C virus (HCV).

11. The method according to claim 10, wherein said HCV core protein comprises the last 21 amino acids of the carboxy-terminus of HCV core.

12. The method according to claim 9 or 10, wherein said E2 protein is a mutated E2 protein selected from the group consisting of a E2 protein deleted from its C-terminal amino acid residue, and a native E2 protein wherein the hypervariable region 1 (HRV1) has been deleted.

13. The method according to any of preceding claims, wherein said nucleic acid sequence comprising a packaging competent retroviral-derived genome further comprises a transgene.

14. An infectious hepacivirus-like particle obtainable by a method according to any of preceding claims, comprising the core proteins from a retrovirus, and a E1 hepacivirus or GB virus glycoprotein and/or a E2 hepacivirus or GB virus glycoprotein.

15. The infectious particle according to claim 14, comprising E1 and E2 hepacivirus or GB virus glycoproteins.

16. The infectious particle according to claim 14, comprising E1 hepacivirus or GB virus glycoprotein.

17. The infectious particle according to claim 14, comprising E2 hepacivirus or GB virus glycoprotein.

18. The infectious particle according to any of claim 14 to 17, further comprising a hepacivirus or GB virus p7 protein.

19. The infectious particle according to any of claim 14 to 18, comprising native E1 and/or E2 hepacivirus or GB virus glycoprotein, and optionally native p7 protein.

20. The infectious particle according to any of claim 14 to 19, wherein core E1 and E2 protein, and optionally p7 proteins, are derived from a same hepacivirus or GB virus.

21. The infectious particle according to claim 20, wherein said hepacivirus or GB virus is HCV.

22. The infectious particle according to claim 21, wherein said E2 protein is a mutated E2 protein selected from the group consisting of a native E2 protein deleted from its C-terminal amino acid residue, and a native E2 protein wherein the hypervariable region 1 (HRV1) has been deleted.

23. The infectious particle according to any of claims 14 to 23, wherein said retrovirus is selected from the group consisting of MLV, ALV, RSV, MPMV, HIV-1, HIV-2, SIV, EIAV, CAEV, or HFV.

24. The infectious particle according to any of claims 14 to 23, wherein said nucleic acid sequence comprising a packaging competent retroviral-derived genome further comprises a transgene.

25. Use of three nucleic acid sequences for the preparation of a medicament useful as a vaccine against hepatitis, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a signal peptide from a type I membrane protein, and a hepacivirus or GB virus E1 protein and/or a hepacivirus or GB virus E2 protein, wherein the C-terminal transmembrane domain of the E1 and E2 proteins is unmodified with respect to native E1 and E2 proteins ;
and, when transferred into cells of a subject, the nucleic acids sequences allow the production of structural proteins from hepacivirus or GB virus and retrovirus, wherein the structural proteins form virus-like particles that are immunogenic.

26. The use according to claim 25 wherein said third nucleic acid sequence comprises a cDNA encoding a polyprotein comprising successively a hepacivirus or GB virus core protein, and a hepacivirus or GB virus E1 protein and/or a hepacivirus or GB virus E2 protein.

27. The use according to claim 25 or 26, wherein said third nucleic acid sequence comprises a cDNA encoding a polyprotein that further comprises a hepacivirus or GB virus p7 protein.

28. The use according to any of claims 25 to 27, wherein said hepacivirus or GB virus is HCV.

29. A method for *ex vivo* identification of a receptor for hepacivirus or GB virus E1 and/or E2 glycoprotein comprising detection of the binding of an infectious particle according to any of claims 14 to 23, to a cell receptor.

30. A method for *ex vivo* identifying a cell receptor for hepacivirus or GB virus comprising the step consisting of:
- transfecting a cell which is not permissive for hepacivirus or GB virus infection with a nucleic acid sequence encoding a protein likely to be a receptor for hepacivirus or GB virus;
- contacting said transformed cell with a hepacivirus-like particle according to any of claims 14 to 23;
- determining whether said transformed cell has become permissive or not for hepacivirus or GB virus infection; and
- identifying as a cell receptor for hepacivirus or GB virus said protein expressed by the transformed cell that has become permissive.

31. A method for *ex vivo* identifying a cell receptor for a hepacivirus or GB virus comprising the step consisting of:
- providing an expression cDNA library obtained from a cell permissive for hepacivirus or GB virus infection;
- transfecting cells that are not permissive for hepacivirus or GB virus infection with said expression cDNA library;
- contacting said transformed cells with hepacivirus or GB virus -like particles according to any of claims 14 to 23;
- identifying and isolating those transformed cells that have become permissive for hepacivirus or GB virus infection;
- isolating the expression vector transfected in cells that have become permissive; and
- identifying as a receptor for hepacivirus or GB virus the proteins encoded by the cDNA sequence of said isolated expression vectors.

32. A method of *ex vivo* screening or identification of molecules capable of interfering with hepacivirus or GB virus entry in cells comprising comparison of the level of cell infection by an infectious particle according to any of claims 14 to 23 in the presence or the absence of a candidate molecule.

33. A method of *in vitro* diagnosis of a hepacivirus or GB virus infection in a patient, comprising detecting immune complexes formed by interaction of anti-hepacivirus or GB virus antibodies likely to be present in a biological sample of the patient with hepacivirus-like particle according to any of claim 14 to 23.

34. A method of *in vitro* diagnosis of a hepacivirus or GB virus infection in a patient, comprising detecting an inhibitory effect of anti-hepacivirus or GB virus antibodies likely to be present in a biological sample of the patient, on the infection of a permissive cell by hepacivirus-like particles according to any of claims 14 to 23.

35. A diagnostic kit useful for the method of claim 34, comprising a hepacivirus-like particle according to any of claims 14 to 23 and appropriate means of detection of said immune complexes.

36. Vaccine composition comprising a hepacivirus-like particle according to any of claim 14 to 24 and a pharmaceutically acceptable carrier.

37. A method for *in vitro* transferring a transgene of interest in a hepatic cell comprising infecting a cell with a hepacivirus-like particle as described in any of claims 14 to 24, wherein the hepacivirus-like particle carries a transgene of interest.

38. Use of a hepacivirus-like particle according to any of claims 14 to 24, that carries a transgene of interest, for the preparation of a medicament for the prevention or treatment of a disease in a patient, wherein the hepacivirus-like particle allows the transfer of the transgene of interest into a cell of the patient, and encodes a product that has a prophylactic or therapeutic effect against the disease.

39. A transformed host cell that contains :
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a signal peptide from a type I membrane protein, and a hepacivirus or GB virus E1 protein and/or a hepacivirus or GB virus E2 protein, wherein the C-terminal transmembrane domain of the E1 and E2 proteins is unmodified with respect to native E1 and E2 proteins.

40. The transformed host cell according to claim 39 wherein said third nucleic acid sequence comprising a cDNA encoding a polyprotein comprising successively a hepacivirus or GB virus core protein, and a hepacivirus or GB virus E1 protein and/or a hepacivirus or GB virus E2 protein.

41. The transformed host cell according to claim 39 or 40, wherein said third nucleic acid sequence comprises a cDNA encoding a polyprotein that further comprises a HCV p7 protein.

42. The transformed host cell according to any of claims 39 to 41, wherein said hepacivirus or GB virus is HCV.

43. A method of *ex vivo* screening of molecules capable of interfering with hepacivirus or GB virus entry in cells comprising comparing the level of fusion of a transformed host cell according to any of claims 39 to 42 to a target host cell, in the presence or the absence of a candidate molecule.

44. The method according to claim 43, comprising the steps consisting of:
- co-culturing said transformed host cell with a target host cell, in the absence or presence of a candidate molecule, under conditions that allow syncytia formation, i.e. cell-cell fusion, and hepacivirus-like particle entry in target host cell in the absence of any candidate molecule;
- assessing syncytia formation in the absence or in the presence of said candidate molecule;
- comparing syncytia formation measured in presence of said candidate molecule with syncytia formation measured in absence of any candidate molecule;
- identifying as a molecule capable of interfering with hepacivirus or GB virus entry the candidate molecule for which syncytia formation, as measured in the presence of said molecule, is decreased as compared to syncytia formation measured in the absence of any candidate molecule.

45. The method, according to any of claims 29 to 34, 37, and 42 to 44, wherein said hepacivirus or GB virus is HCV.

## Patentansprüche

1. Ein Verfahren zur Herstellung von infektiösen Hepacivirus-ähnlichen Partikeln *ex vivo*, dass die Schritte umfasst:
- Bereitstellen einer ersten Nukleinsäuresequenz, die ein verpackungskompetentes retroviral-abgeleitetes Genom umfasst;
- Bereitstellen einer zweiten Nukleinsäuresequenz, die eine cDNA umfasst, die Kernproteine des Retrovirus kodiert;
- Bereitstellen einer dritten Nukleinsäuresequenz, die eine cDNA umfasst, die ein Polyprotein kodiert, das aufeinanderfolgend ein Signalpeptid eines Membranproteins vom Typ I und ein Hepacivirus oder GB-Virus E1-Protein und/oder ein Hepacivirus oder GB-Virus E2-Protein umfasst, wobei die C-terminale Transmembrandomain der E1- und E2-Proteine in Bezug auf natürliche E1- und E2-Proteine unmodifiziert ist;
- Transfizieren von Wirtszellen mit den Nukleinsäuresequenzen und Aufrechterhalten der transfizierten Zellen in Kultur für eine ausreichende Zeit, um Exprimierung der cDNAs zu ermöglichen, um Strukturproteine des Hepacivirus oder des GB-Virus und des Retrovirus herzustellen; und es den Strukturproteinen zu ermöglichen, Virus-ähnliche Partikel zu bilden.

2. Das Verfahren gemäß Anspruch 1, wobei die dritte Nukleinsäuresequenz eine cDNA umfasst, die ein Polyprotein kodiert, das aufeinanderfolgend ein Hepacivirus oder GB-Virus Kernprotein und ein Hepacivirus oder GB-Virus E1-Protein und/oder ein Hepacivirus oder GB-Virus E2-Protein umfasst.

3. Das Verfahren gemäß Anspruch 1 oder 2, wobei das verpackungskompetente retroviral-abgeleitete Genom und die Kernproteine von einem Retrovirus sind, der aus der Gruppe, die aus MLV, ALV, RSV, MPMV, HIV-1, HIV-2, SIV, EIAV, CAEV oder HFV besteht, ausgewählt wird.

4. Das Verfahren gemäß Anspruch 2 oder 3, wobei das Polyprotein ein Hepacivirus oder GB-Virus Kernprotein und ein Hepacivirus oder GB-Virus E1-Protein umfasst.

5. Das Verfahren gemäß einem der Ansprüche 2 bis 4, wobei das Polyprotein ein Hepacivirus oder GB-Virus Kernprotein und ein Hepacivirus oder GB-Virus E2-Protein umfasst.

6. Das Verfahren gemäß einem der Ansprüche 1 bis 5, wobei die dritte Nukleinsäuresequenz eine cDNA umfasst, die ein Polyprotein kodiert, das weiter ein Hepacivirus oder GB-Virus p7-Protein umfasst.

7. Das Verfahren gemäß einem der Ansprüche 1 bis 6, wobei das Polyprotein ein natürliches Hepacivirus oder GB-Virus E1- und/oder E2-Protein und optional ein natürliches Hepacivirus oder GB-Virus p7-Protein umfasst.

8. Das Verfahren gemäß einem der Ansprüche 2 bis 7, wobei das Polyprotein ein natürliches Hepacivirus oder GB-Virus Kernprotein, ein natürliches Hepacivirus oder GB-Virus E1-Protein und ein natürliches Hepacivirus oder GB-Virus E2-Protein und optional ein natürliches p7-Protein umfasst.

9. Das Verfahren gemäß einem der Ansprüche 2 bis 8, wobei sich das Kernprotein, das E1-Protein und das E2-Protein und optional das p7-Protein von demselben Hepacivirus oder GB-Virus ableiten.

10. Das Verfahren gemäß Anspruch 9, wobei das Hepacivirus oder das GB-Virus ein Hepatitis C Virus (HCV) ist.

11. Das Verfahren gemäß Anspruch 10, wobei das HCV Kernprotein die letzten 21 Aminosäuren des Carboxyterminalen HCV Kerns umfasst.

12. Das Verfahren gemäß Anspruch 9 oder 10, wobei das E2-Protein ein mutiertes E2-Protein ist, das aus der Gruppe, die aus einem E2-Protein, das von dessen C-terminalen Aminosäurerest entfernt worden ist, und ein natürliches E2-Protein bei dem der hypervariable Bereich 1 (HRV1) entfernt worden ist, besteht, ausgewählt wird.

13. Das Verfahren gemäß einem der vorangegangenen Ansprüche, wobei die Nukleinsäuresequenz, die ein verpackungskompetentes retroviral-abgeleitetes Genom umfasst, weiter ein Transgen umfasst.

14. Ein infektiöser Hepacivirus-ähnlicher Partikel, der durch ein Verfahren gemäß einem der vorangegangenen Ansprüche erhältlich ist, der die Kernproteine eines Retrovirus, und eines E1-Hepacivirus oder GB-Virus Glycoproteins und/oder eines E2-Hepacivirus oder GB-Virus Glycoproteins umfasst.

15. Das infektiöse Partikel gemäß Anspruch 14, der E1- und E2-Hepacivirus oder GB-Virus Glycoproteine umfasst.

16. Das infektiöse Partikel gemäß Anspruch 14, der ein E1-Hepacivirus oder GB-Virus Glycoprotein umfasst.

17. Das infektiöse Partikel gemäß Anspruch 14, der ein E2-Hepacivirus oder GB-Virus Glycoprotein umfasst.

18. Das infektiöse Partikel gemäß einem der Ansprüche 14 bis 17, der weiter ein Hepacivirus oder GB-Virus p7-Protein umfasst.

19. Das infektiöse Partikel gemäß einem der Ansprüche 14 bis 18, der ein natürliche E1- und/oder E2-Hepacivirus oder GB-Virus Glycoprotein und optional ein natürliche p7-Protein umfasst.

20. Das infektiöse Partikel gemäß einem der Ansprüche 14 bis 19, wobei sich das E1- und das E2-Kernprotein und optional die p7-Proteine von demselben Hepacivirus oder GB-Virus ableiten.

21. Das infektiöse Partikel gemäß Anspruch 20, wobei das Hepacivirus oder das GB-Virus das HCV ist.

22. Das infektiöse Partikel gemäß Anspruch 21, wobei das E2-Protein ein mutiertes E2-Protein ist, das aus der Gruppe, die aus einem natürlichen E2-Protein, das von dessen C-terminalen Aminosäurerest getrennt worden ist, und einem natürlichen E2-Protein bei dem der hypervariable Bereich 1 (HRV1) entfernt worden ist, besteht, ausgewählt wird.

23. Das infektiöse Partikel gemäß einem der Ansprüche 14 bis 22, wobei das Retrovirus aus der Gruppe, die aus MLV, ALV, RSV, MPMV, HIV-1, HIV-2, SIV, EIAV, CAEV oder HFV besteht, ausgewählt wird.

24. Das infektiöse Partikel gemäß einem der Ansprüche 14 bis 23, wobei die Nukleinsäuresequenz, die ein verpackungskompetentes retroviral-abgeleitetes Genom umfasst, weiter ein Transgen umfasst.

25. Verwendung von drei Nukleinsäuresequenzen für die Herstellung eines Medikaments, das als ein Impfstoff gegen Hepatitis verwendbar ist, wobei die Nukleinsäuresequenzen sind:
- eine erste Nukleinsäuresequenz, die ein verpackungskompetentes retroviral-abgeleitetes Genome umfasst;
- eine zweite Nukleinsäuresequenz, die eine cDNA umfasst, die Kernproteine des Retrovirus kodiert;
- eine dritte Nukleinsäuresequenz, die eine cDNA umfasst, die ein Polyprotein kodiert, das aufeinanderfolgend ein Signalpeptid eines Membranproteins vom Typ I und ein Hepacivirus oder GB-Virus E1-Protein und/oder ein Hepacivirus oder GB-Virus E2-Protein umfasst, wobei die C-terminale Transmembrandomain der E1- und E2-Proteine in Bezug auf natürliche E1- und E2-Proteine unmodifiziert ist;
und wenn in Zellen eines Subjekts transferiert, ermöglichen die Nukleinsäuresequenzen die Herstellung von Strukturproteinen von Hepacivirus oder GB-Virus und Retrovirus, wobei die Strukturproteine virusähnliche Partikel bilden, die immunogen sind.

26. Die Verwendung gemäß Anspruch 25, wobei die dritte Nukleinsäuresequenz eine cDNA umfasst, die ein Polyprotein kodiert, das aufeinanderfolgend ein Hepacivirus oder GB-Virus Kernprotein und ein Hepacivirus oder GB-Virus E1-Protein und/oder ein Hepacivirus oder GB-Virus E2-Protein umfasst.

27. Die Verwendung gemäß Anspruch 25 oder 26, wobei die dritte Nukleinsäuresequenz eine cDNA umfasst, die ein Polyprotein kodiert, das weiter ein Hepacivirus oder GB-Virus p7-Protein umfasst.

28. Die Verwendung gemäß einem der Ansprüche 25 bis 27, wobei das Hepacivirus oder das GB-Virus ein HCV ist.

29. Ein Verfahren für die *ex vivo* Identifizierung eines Rezeptors für ein Hepacivirus oder GB-Virus E1- und/oder E2-Glycoprotein, das das Detektieren des Bindens eines infektiösen Partikels gemäß einem der Ansprüche 14 bis 23 an einen Zellenrezeptor umfasst.

30. Ein Verfahren für die *ex vivo* Identifizierung eines Zellrezeptors für ein Hepacivirus oder ein GB-Virus, das die Schritte umfasst, bestehend aus:
- Transfizieren einer Zelle, die für eine Hepacivirus oder GB-Virus Infektion nicht permissiv ist, mit einer Nukleinsäuresequenz, die ein Protein kodiert, das wahrscheinlich ein Rezeptor für einen Hepacivirus oder einen GB-Virus ist;
- In-Kontakt-bringen der transformierten Zelle mit einem Hepacivirus-ähnlichen Partikel gemäß einem der Ansprüche 14 bis 23;
- Bestimmen ob die transformierte Zelle für eine Hepacivirus oder GB-Virus Infektion permissiv geworden ist oder nicht; und
- Identifizieren des Proteins, das durch die transformierte Zelle, die permissiv geworden ist, exprimiert wird, als ein Zellrezeptor für ein Hepacivirus oder ein GB-Virus.

31. Ein Verfahren für die *ex vivo* Identifizierung eines Zellrezeptors für einen Hepacivirus oder einen GB-Virus, das die Schritte umfasst, bestehend aus:
- Bereitstellen einer cDNA Exprimierungsbibliothek, die von einer Zelle erhalten wird, die für eine Hepacivirus oder GB-Virus Infektion permissiv ist;
- Transfizieren von Zellen, die für eine Hepacivirus oder GB-Virus Infektion nicht permissiv sind, mit der cDNA Exprimierungsbibliothek;
- In-Kontakt-bringen der transformierten Zellen mit Hepacivirus oder GB-Virus-ähnlichen Partikeln gemäß einem der Ansprüche 14 bis 23;
- Identifizieren und Isolieren dieser transformierten Zellen, die für eine Hepacivirus oder GB-Virus Infektion permissiv geworden sind;
- Isolieren des Exprimierungsvektors, der in Zellen transfiziert worden ist, die permissiv geworden sind; und
- Identifizieren der Proteine, die durch die cDNA Sequenz der isolierten Exprimierungsvektoren kodiert sind, als ein Rezeptor für ein Hepacivirus oder ein GB-Virus.

32. Ein Verfahren zum *ex vivo* Screenen oder zum Identifizieren von Molekülen, die einen Hepacivirus oder GB-Virus Eintrag in Zellen beeinträchtigen können, das den Vergleich des Niveaus an Zellinfektion durch einen infektiösen Partikel gemäß einem der Ansprüche 14 bis 23 in Gegenwart oder Abwesenheit eines Kandidatenmoleküls umfasst.

33. Ein Verfahren der *in vitro* Diagnose einer Hepacivirus oder GB-Virus Infektion bei einem Patienten, das das Detektieren von Immunkomplexen, die durch Wechselwirkung von anti-Hepacivirus oder GB-Virus Antikörpern, die wahrscheinlich in einer biologischen Probe des Patienten vorhanden sind, mit Hepacivirus-ähnlichen Partikeln gemäß einem der Ansprüche 14 bis 23 umfasst.

34. Ein Verfahren der *in vitro* Diagnose einer Hepacivirus oder GB-Virus Infektion bei einem Patienten, das das Detektieren einer inhibitorischen Wirkung von anti-Hepacivirus oder GB-Virus Antikörpern, die wahrscheinlich in einer biologischen Probe des Patienten vorhanden sind, auf die Infektion einer permissiven Zellen durch einen Hepacivirus-ähnlichen Partikeln gemäß einem der Ansprüche 14 bis 23 umfasst.

35. Ein Diagnosekit, das für das Verfahren nach Anspruch 34 verwendbar ist, das einen Hepacivirus-ähnlichen Partikel gemäß einem der Ansprüche 14 bis 23 und geeignete Mittel zur Detektion der Immunkomplexe umfasst.

36. Impfstoffzusammensetzung, die ein Hepacivirus-ähnliches Partikel gemäß einem der Ansprüche 14 bis 24 und einen pharmazeutisch geeigneten Träger umfasst.

37. Ein Verfahren für das *in vivo* Transferieren eines Transgens von Interesse in eine hepatische Zelle, das das Infizieren einer Zelle mit einem Hepacivirus-ähnlichen Partikel, wie in einem der Ansprüche 14 bis 24 beschrieben, umfasst, wobei der Hepacivirus-ähnliche Partikel ein Transgen von Interesse trägt.

38. Verwendung eines Hepacivirus-ähnlichen Partikels gemäß einem der Ansprüche 14 bis 24, der ein Transgen von Interesse trägt, für die Herstellung eines Medikaments für die Prävention oder die Behandlung einer Erkrankung in einem Patienten, wobei der Hepacivirus-ähnliche Partikel den Transfer des Transgens von Interesse in eine Zelle des Patienten ermöglicht und ein Produkt kodiert, dass eine prophylaktische oder therapeutische Wirkung gegen die Erkrankung hat.

39. Eine transformierte Wirtszelle, die enthält:
- eine erste Nukleinsäuresequenz, die ein verpackungskompetentes retroviral-abgeleitetes Genome umfasst;
- eine zweite Nukleinsäuresequenz, die eine cDNA umfasst, die Kernproteine des Retrovirus kodiert;
- eine dritte Nukleinsäuresequenz, die eine cDNA umfasst, die ein Polyprotein kodiert, das aufeinanderfolgend ein Signalpeptid eines Membranproteins vom Typ I und ein Hepacivirus oder GB-Virus E1-Protein und/oder ein Hepacivirus oder GB-Virus E2-Protein umfasst, wobei die C-terminale Transmembrandomain der E1- und E2-Proteine in Bezug auf natürliche E1- und E2-Proteine unmodifiziert ist.

40. Die transformierte Wirtszelle gemäß Anspruch 39, wobei die dritte Nukleinsäuresequenz eine cDNA umfasst, die ein Polyprotein kodiert, das aufeinanderfolgend ein Hepacivirus oder GB-Virus Kernprotein und ein Hepacivirus oder GB-Virus E1-Protein und/oder ein Hepacivirus oder GB-Virus E2-Protein umfasst.

41. Die transformierte Wirtszelle gemäß Anspruch 39 oder 40, wobei die dritte Nukleinsäuresequenz eine cDNA umfasst, die ein Polyprotein kodiert, das weiter ein HCV p7-Protein umfasst.

42. Die transformierte Wirtszelle gemäß einem der Ansprüche 39 bis 41, wobei das Hepacivirus oder das GB-Virus ein HCV ist.

43. Ein Verfahren zum *ex vivo* Screenen von Molekülen, die einen Hepacivirus oder GB-Virus Eintrag in Zellen beeinträchtigen können, das das Vergleichen des Niveaus an Fusion einer transformierten Wirtszelle gemäß einem der Ansprüche 39 bis 42 mit einer Zielwirtszelle in Gegenwart oder Abwesenheit eines Kandidatenmoleküls umfasst.

44. Das Verfahren gemäß Anspruch 43, das die Schritte umfasst:
- Co-Kultivieren der transformierten Wirtszelle mit einer Zielwirtszelle in Abwesenheit oder Gegenwart eines Kandidatenmoleküls unter Bedingungen, die Synzytiumbildung, d.h. Zell-Zell-Fusion, ermöglichen und einen Eintrag eines Hepacivirus-ähnlichen Partikels in eine Zielwirtszelle in Abwesenheit eines Kandidatenmoleküls;
- Beurteilen der Synzytiumbildung in Abwesenheit oder in Gegenwart des Kandidatenmoleküls;
- Vergleichen der Synzytiumbildung, die in Gegenwart des Kandidatenmoleküls gemessen wurde, mit der Synzytiumbildung, die in Abwesenheit eines Kandidatenmoleküls gemessen wurde;
- Identifizieren des Kandidatenmoleküls für das die Synzytiumbildung, wie in Gegenwart des Moleküls gemessen, verringert ist im Vergleich zu der Synzytiumbildung, die in Abwesenheit eines Kandidatenmoleküls gemessen wurde, als ein Molekül, das einen Hepacivirus oder einen GB-Virus Eintrag beeinträchtigen kann.

45. Das Verfahren gemäß einem der Ansprüche 29 bis 34, 37 und 42 bis 44, wobei das Hepacivirus oder das GB-Virus ein HCV ist.

## Revendications

1. Procédé de production de pseudo-particules d'hépacivirus infectieuses *ex vivo* comprenant les étapes consistant à :
- fournir une première séquence d'acide nucléique comprenant un génome dérivé d'un rétrovirus compétent pour l'empaquetage ;
- fournir une deuxième séquence d'acide nucléique comprenant un ADNc codant pour des protéines core provenant dudit rétrovirus ;
- fournir une troisième séquence d'acide nucléique comprenant un ADNc codant pour une polyprotéine comprenant successivement un peptide signal issu d'une protéine membranaire de type 1 et une protéine E1 d'hépacivirus ou de virus GB et/ou une protéine E2 d'hépacivirus ou de virus GB, où le domaine transmembranaire C-terminal des protéines E1 et E2 est non modifié par rapport aux protéines E1 et E2 natives ;
- transfecter des cellules hôtes avec lesdites séquences d'acide nucléique et maintenir les cellules transfectées en culture pendant un temps suffisant pour permettre l'expression des ADNc pour produire des protéines structurales provenant d'hépacivirus ou de virus GB et de rétrovirus ; et laisser les protéines structurales former des pseudo-particules virales.

2. Procédé selon la revendication 1, dans lequel ladite troisième séquence d'acide nucléique comprend un ADNc codant pour une polyprotéine comprenant successivement une protéine core d'hépacivirus ou de virus GB, et une protéine E1 d'hépacivirus ou de virus GB et/ou une protéine E2 d'hépacivirus ou de virus GB.

3. Procédé selon la revendication 1 ou 2, dans lequel ledit génome dérivé d'un rétrovirus compétent pour l'empaquetage et les protéines core proviennent d'un rétrovirus choisi dans le groupe constitué de MLV, ALV, RSV, MPMV, VIH-1, VIH-2, SIV, EIAV, CAEV ou HFV.

4. Procédé selon la revendication 2 ou 3, dans lequel ladite polyprotéine comprend une protéine core d'hépacivirus ou de virus GB et une protéine E1 d'hépacivirus ou de virus GB.

5. Procédé selon l'une quelconque des revendications 2 à 4, dans lequel ladite polyprotéine comprend une protéine core d'hépacivirus ou de virus GB et une protéine E2 d'hépacivirus ou de virus GB.

6. Procédé selon l'une quelconque des revendications 1 à 5, dans lequel ladite troisième séquence d'acide nucléique comprend un ADNc codant pour une polyprotéine qui comprend en outre une protéine p7 d'hépacivirus ou de virus GB.

7. Procédé selon l'une quelconque des revendications 1 à 6, dans lequel ladite polyprotéine comprend la protéine E1 et/ou E2 native d'hépacivirus ou de virus GB, et éventuellement la protéine p7 native d'hépacivirus ou de virus GB.

8. Procédé selon l'une quelconque des revendications 2 à 7, dans lequel ladite polyprotéine comprend une protéine core native d'hépacivirus ou de virus GB, une protéine E1 native d'hépacivirus ou de virus GB et une protéine E2 native d'hépacivirus ou de virus GB, et éventuellement une protéine p7 native.

9. Procédé selon l'une quelconque des revendications 2 à 8, dans lequel la protéine core, la protéine E1 et la protéine E2, et éventuellement la protéine p7, sont dérivées d'un même hépacivirus ou virus GB.

10. Procédé selon la revendication 9, dans lequel ledit hépacivirus ou virus GB est un virus de l'hépatite C (VHC).

11. Procédé selon la revendication 10, dans lequel ladite protéine core du VHC comprend les 21 derniers acides aminés de l'extrémité carboxy du core du VHC.

12. Procédé selon la revendication 9 ou 10, dans lequel ladite protéine E2 est une protéine E2 mutée choisie dans le groupe constitué d'une protéine E2 délétée de son résidu d'acide aminé C-terminal et d'une protéine E2 native dans laquelle la région hypervariable 1 (HRV1) a été délétée.

13. Procédé selon l'une quelconque des revendications précédentes, dans lequel ladite séquence d'acide nucléique comprenant un génome dérivé d'un rétrovirus compétent pour l'empaquetage comprend en outre un transgène.

14. Pseudo-particule d'hépacivirus infectieuse susceptible d'être obtenue par un procédé selon l'une quelconque des revendications précédentes, comprenant les protéines core provenant d'un rétrovirus, et une glycoprotéine E1 d'hépacivirus ou de virus GB et/ou une glycoprotéine E2 d'hépacivirus ou de virus GB.

15. Particule infectieuse selon la revendication 14, comprenant les glycoprotéines E1 et E2 d'hépacivirus ou de virus GB.

16. Particule infectieuse selon la revendication 14, comprenant la glycoprotéine E1 d'hépacivirus ou de virus GB.

17. Particule infectieuse selon la revendication 14, comprenant la glycoprotéine E2 d'hépacivirus ou de virus GB.

18. Particule infectieuse selon l'une quelconque des revendications 14 à 17, comprenant en outre une protéine p7 d'hépacivirus ou de virus GB.

19. Particule infectieuse selon l'une quelconque des revendications 14 à 18, comprenant la glycoprotéine E1 et/ou E2 native d'hépacivirus ou de virus GB, et éventuellement la protéine p7 native.

20. Particule infectieuse selon l'une quelconque des revendications 14 à 19, dans laquelle les protéines core, E1 et E2 et éventuellement les protéines p7, sont dérivées d'un même hépacivirus ou virus GB.

21. Particule infectieuse selon la revendication 20, dans laquelle ledit hépacivirus ou virus GB est le VHC.

22. Particule infectieuse selon la revendication 21, dans laquelle ladite protéine E2 est une protéine E2 mutée choisie dans le groupe constitué d'une protéine E2 native délétée de son résidu d'acide aminé C-terminal, et d'une protéine E2 native dans laquelle la région hypervariable 1 (HRV1) a été délétée.

23. Particule infectieuse selon l'une quelconque des revendications 14 à 23, dans laquelle ledit rétrovirus est choisi dans le groupe constitué de MLV, ALV, RSV, MPMV, VIH-1, VIH-2, SIV, EIAV, CAEV ou HFV.

24. Particule infectieuse selon l'une quelconque des revendications 14 à 23, dans laquelle ladite séquence d'acide nucléique comprenant un génome dérivé d'un rétrovirus compétent pour l'empaquetage comprend en outre un transgène.

25. Utilisation de trois séquences d'acide nucléique pour la préparation d'un médicament utile en tant que vaccin contre l'hépatite, où les séquences d'acide nucléique sont :
- une première séquence d'acide nucléique comprenant un génome dérivé d'un rétrovirus compétent pour l'empaquetage ;
- une deuxième séquence d'acide nucléique comprenant un ADNc codant pour des protéines core provenant dudit rétrovirus ;
- une troisième séquence d'acide nucléique comprenant un ADNc codant pour une polyprotéine comprenant successivement un peptide signal issu d'une protéine membranaire de type 1 et une protéine E1 d'hépacivirus ou de virus GB et/ou une protéine E2 d'hépacivirus ou de virus GB, où le domaine transmembranaire C-terminal des protéines E1 et E2 est non modifié par rapport aux protéines E1 et E2 natives ;
et, lorsqu'elles sont transférées dans les cellules d'un sujet, les séquences d'acide nucléique permettent la production de protéines structurales provenant d'hépacivirus ou de virus GB et de rétrovirus, où les protéines structurales forment des pseudo-particules virales qui sont immunogènes.

26. Utilisation selon la revendication 25, dans laquelle ladite troisième séquence d'acide nucléique comprend un ADNc codant pour une polyprotéine comprenant successivement une protéine core d'hépacivirus ou de virus GB et une protéine E1 d'hépacivirus ou de virus GB et/ou une protéine E2 d'hépacivirus ou de virus GB.

27. Utilisation selon la revendication 25 ou 26, dans laquelle ladite troisième séquence d'acide nucléique comprend un ADNc codant pour une polyprotéine qui comprend en outre une protéine p7 d'hépacivirus ou de virus GB.

28. Utilisation selon l'une quelconque des revendications 25 à 27, dans laquelle ledit hépacivirus ou virus GB est le VHC.

29. Procédé d'identification ex *vivo* d'un récepteur pour la glycoprotéine E1 et/ou E2 d'hépacivirus ou de virus GB comprenant la détection de la liaison d'une particule infectieuse selon l'une quelconque des revendications 14 à 23, à un récepteur cellulaire.

30. Procédé d'identification *ex vivo* d'un récepteur cellulaire pour hépacivirus ou virus GB comprenant les étapes consistant à :
- transfecter une cellule qui est non permissive pour une infection par hépacivirus ou virus GB avec une séquence d'acide nucléique codant pour une protéine susceptible d'être un récepteur pour hépacivirus ou virus GB ;
- mettre en contact ladite cellule transformée avec une pseudo-particule d'hépacivirus selon l'une quelconque des revendications 14 à 23 ;
- déterminer si ladite cellule transformée est devenue permissive ou non pour une infection par hépacivirus ou virus GB ; et
- identifier comme récepteur cellulaire pour hépacivirus ou virus GB, ladite protéine exprimée par la cellule transformée qui est devenue permissive.

31. Procédé d'identification *ex vivo* d'un récepteur cellulaire pour un hépacivirus ou un virus GB comprenant les étapes consistant à :
- fournir une banque d'expression d'ADNc obtenue à partir d'une cellule permissive pour une infection par hépacivirus ou virus GB ;
- transfecter des cellules qui ne sont pas permissives pour une infection par hépacivirus ou virus GB avec ladite banque d'expression d'ADNc ;
- mettre en contact lesdites cellules transformées avec des pseudo-particules d'hépacivirus ou de virus GB selon l'une quelconque des revendications 14 à 23;
- identifier et isoler ces cellules transformées qui sont devenues permissives pour une infection par hépacivirus ou virus GB ;
- isoler le vecteur d'expression transfecté dans les cellules qui sont devenues permissives ; et
- identifier comme récepteur pour hépacivirus ou virus GB, les protéines codées par la séquence d'ADNc desdits vecteurs d'expression isolés.

32. Procédé de criblage ou d'identification *ex vivo* de molécules capables d'interférer avec l'entrée d'hépacivirus ou de virus GB dans des cellules comprenant la comparaison du niveau d'infection cellulaire par une particule infectieuse selon l'une quelconque des revendications 14 à 23 en présence ou en l'absence d'une molécule candidate.

33. Procédé de diagnostic *in vitro* d'une infection par hépacivirus ou virus GB chez un patient, comprenant la détection des complexes immuns formés par l'interaction d'anticorps anti-hépacivirus ou virus GB susceptibles d'être présents dans un échantillon biologique du patient avec une pseudo-particule d'hépacivirus selon l'une quelconque des revendications 14 à 23.

34. Procédé de diagnostic *in vitro* d'une infection par hépacivirus ou virus GB chez un patient, comprenant la détection d'un effet inhibiteur des anticorps anti-hépacivirus ou virus GB susceptibles d'être présents dans un échantillon biologique du patient, sur l'infection d'une cellule permissive par des pseudo-particules d'hépacivirus selon l'une quelconque des revendications 14 à 23.

35. Kit de diagnostic utile pour le procédé selon la revendication 34, comprenant une pseudo-particule d'hépacivirus selon l'une quelconque des revendications 14 à 23 et un moyen de détection approprié desdits complexes immuns.

36. Composition vaccinale comprenant une pseudo-particule d'hépacivirus selon l'une quelconque des revendications 14 à 24 et un véhicule pharmaceutiquement acceptable.

37. Procédé de transfert *in vitro* d'un transgène d'intérêt dans une cellule hépatique comprenant l'infection d'une cellule avec une pseudo-particule d'hépacivirus telle que décrite dans l'une quelconque des revendications 14 à 24, dans lequel la pseudo-particule d'hépacivirus porte un transgène d'intérêt.

38. Utilisation d'une pseudo-particule d'hépacivirus selon l'une quelconque des revendications 14 à 24, qui porte un transgène d'intérêt, pour la préparation d'un médicament pour la prévention ou le traitement d'une maladie chez un patient, dans laquelle la pseudo-particule d'hépacivirus permet le transfert du transgène d'intérêt dans une cellule du patient, et code pour un produit qui a un effet prophylactique ou thérapeutique contre la maladie.

39. Cellule hôte transformée qui contient :
- une première séquence d'acide nucléique comprenant un génome dérivé d'un rétrovirus compétent pour l'empaquetage ;
- une deuxième séquence d'acide nucléique comprenant un ADNc codant pour des protéines core provenant dudit rétrovirus ; et
- une troisième séquence d'acide nucléique comprenant un ADNc codant pour une polyprotéine comprenant successivement un peptide signal issu d'une protéine membranaire de type I et une protéine E1 d'hépacivirus ou de virus GB et/ou une protéine E2 d'hépacivirus ou de virus GB, où le domaine transmembranaire C-terminal des protéines E1 et E2 est non modifié par rapport aux protéines E1 et E2 natives.

40. Cellule hôte transformée selon la revendication 39, dans laquelle la troisième séquence d'acide nucléique comprend un ADNc codant pour une polyprotéine comprenant successivement une protéine core d'hépacivirus ou de virus GB et une protéine E1 d'hépacivirus ou de virus GB et/ou une protéine E2 d'hépacivirus ou de virus GB.

41. Cellule hôte transformée selon la revendication 39 ou 40, dans laquelle la troisième séquence d'acide nucléique comprend un ADNc codant pour une polyprotéine qui comprend en outre une protéine p7 du VHC.

42. Cellule hôte transformée selon l'une quelconque des revendications 39 à 41, dans laquelle ledit hépacivirus ou le virus GB est le VHC.

43. Procédé de criblage *ex vivo* de molécules capables d'interférer avec l'entrée d'hépacivirus ou de virus GB dans des cellules comprenant la comparaison du niveau de fusion d'une cellule hôte transformée selon l'une quelconque des revendications 39 à 42 à une cellule hôte cible, en présence ou en l'absence d'une molécule candidate.

44. Procédé selon la revendication 43, comprenant les étapes consistant à :
- co-cultiver ladite cellule hôte transformée avec une cellule hôte cible, en l'absence ou en présence d'une molécule candidate, dans des conditions qui permettent la formation de syncytia, c'est-à-dire la fusion cellule-cellule, et l'entrée de pseudo-particules d'hépacivirus dans la cellule hôte cible en l'absence de toute molécule candidate ;
- estimer la formation de syncytia en l'absence ou en présence de ladite molécule candidate ;
- comparer la formation de syncytia mesurée en présence de ladite molécule candidate avec la formation de syncytia mesurée en l'absence de toute molécule candidate ;
- identifier comme molécule capable d'interférer avec l'entrée d'hépacivirus ou de virus GB, la molécule candidate pour laquelle la formation de syncytia, mesurée en présence de ladite molécule, est diminuée par comparaison à la formation de syncytia mesurée en l'absence de toute molécule candidate.

45. Procédé, selon l'une quelconque des revendications 29 à 34, 37, et 42 à 44, dans lequel ledit hépacivirus ou virus GB est le VHC.
